# EUROPEAN PATENT APPLICATION

(11) **EP 4 534 099 A1**
(43) Date of publication of application: **09.04.2025**
(21) Application number: 23811885.5
(22) Date of filing: 25.05.2023
(51) Int. Cl.: A61K 45/00, A61P 35/00, A61P 37/04, A61P 43/00, C12N 5/0735, C12N 5/078, C12N 5/0783, C12N 5/0789, C12N 5/10, C12N 15/09, C12N 15/12, C12N 15/24, A61K 35/15, A61K 35/545

(54) **PRODUCTION METHOD FOR PROLIFERATIVE MYELOID CELLS THAT CONSTITUTIVELY PRODUCE IL-12P70**

(30) Priority: 27.05.2022 JP 2022087065
(71) Applicant: Beijing Tianyifang Bio. Dev. Co., Ltd., Beijing, 100176 (CN); National Cancer Center, Tokyo 104-0045 (JP)
(72) Inventor: UEMURA, Yasushi, Kashiwa-shi, Chiba 277-8577 (JP); FUKUDA, Kyoko, Kashiwa-shi, Chiba 277-8577 (JP)
(74) Representative: HGF
(86) International application number: PCT/JP2023/019528
(87) International publication number: WO 2023/229013

(57) **Abstract**

The invention provides a method for producing a proliferative myeloid cell that constitutively produces IL-12p70, comprising the steps of: (1) introducing a nucleic acid encoding c-MYC into a myeloid cell; (2) culturing the cell obtained by the step (1) in the presence of GM-CSF and M-SCF; and (3) introducing a nucleic acid encoding an IL-12p35 subunit and a nucleic acid encoding an IL-12p40 subunit into the cell obtained by the step (2).

## Description

### TECHNICAL FIELD

The present invention relates to a method for producing a proliferative myeloid cell that constitutively produces IL-12p70, a proliferative myeloid cell obtained thereby, and a pharmaceutical using the cell.

### BACKGROUND ART

Interleukin-12p70 (IL-12p70) is an inflammatory cytokine that is produced by myeloid cells, such as macrophages or dendritic cells (DC), in response to infection or cancer, and they activate T cells and NK cells to promote production of interferon-γ (IFN-γ). When the production of IFN-γ is promoted, antigen presentation is increased, cell-mediated immune response is induced, and tumor-promoting processes such as angiogenesis are inhibited. Therefore, IL-12p70 has been anticipated as a cancer therapeutic agent, but has been prevented from being applied to cancer treatment because of dose-liming toxicity related to systemic administration of IL-12p70 (Non-Patent Document 1).

A strategy for actively eliciting cancer-reactive T-cell responses by inducing IL-12p70 production by DC is one approach that is anticipated to exhibit superior clinical effects in cancer treatment. Currently, so-called DC vaccine therapy, in which antitumor immune responses are activated by loading a cancer antigen to autologous DC and returning the resultant to the body of the patient, generally uses monocyte-derived DC. However, the monocytes are present in a very small amount in peripheral blood and cannot be proliferated by culture, and hence, there is a difficulty in preparation of DC in an effective amount for the treatment. Moreover, the IL-12p70 production by monocyte-derived DC is different among patients, and hence, a sufficient antitumor effect has not been attained.

In order to solve these problems, the present inventors have improved a method for producing proliferative myeloid cells from iPS cells (Patent Documents 1 and 2, and Non-Patent Document 2). However, the proliferative myeloid cells reported so far have been inferior in antitumor effects compared to monocyte-derived DC.

### REFERENCE DOCUMENT LIST

### PATENT DOCUMENT

Patent Document 1: WO2012/043651
Patent Document 2: WO2021/230304

### NON-PATENT DOCUMENT

Non-Patent Document 1: Clin. Cancer Res. 3: 409-417, 1997
Non-Patent Document 2: J. Immunol. Regen. Med. 12: 100042, 2021

### SUMMARY OF INVENTION

### PROBLEM TO BE SOLVED BY THE INVENTION

The present invention has been devised for the purpose of providing proliferative myeloid cells capable of stably producing IL-12p70, and exhibiting excellent antitumor effects, and a method for producing the same.

### MEANS FOR SOLVING THE PROBLEMS

The present inventors have earnestly researched, and as a result, have successfully created a proliferative myeloid cell that produces IL-12p70 constitutively and in large amount regardless of induction by CD40-CD40 ligand interaction or toll-like receptor stimulation.

Specifically, in one embodiment, the present invention provides a method for producing a proliferative myeloid cell that constitutively produces IL-12p70, comprising the steps of: (1) introducing a nucleic acid encoding c-MYC into a myeloid cell; (2) culturing a cell obtained by the step (1) in the presence of GM-CSF and M-SCF; and (3) introducing a nucleic acid encoding an IL-12p35 subunit and a nucleic acid encoding an IL-12p40 subunit into a cell obtained by the step (2).

In the method, a nucleic acid encoding BMI1, and a nucleic acid encoding MDM2, LYL1 or BCL2 isoform alpha, are preferably further introduced in the step (1).

The myeloid cell is preferably differentiated from a pluripotent stem cell.

In another embodiment, the present invention provides a proliferative myeloid cell that constitutively produces IL-12p70, produced by the above-described method.

In another embodiment, the present invention provides a proliferative myeloid cell that constitutively produces IL-12p70, comprising an exogenous nucleic acid encoding c-MYC, an exogenous nucleic acid encoding an IL-12p35 subunit, and an exogenous nucleic acid encoding an IL-12p40 subunit.

The cell preferably proliferates in GM-CSF and M-CSF-dependent manners.

The cell preferably produces IL-12p70 in an amount of at least 300 pg/1×10⁴ cells/24 hours in the absence of CD40-CD40 ligand interaction or toll-like receptor stimulation.

The cell preferably produces at least 5-fold more IL-12p70 than a monocyte-derived dendritic cell in the presence of toll-like receptor stimulation.

The cell preferably expresses at least one costimulatory molecule selected from the group consisting of CD86, CD80, and CD83.

The cell may not express a major histocompatibility complex class I and/or class II molecule.

The cell preferably is deficient in genes encoding major histocompatibility complex class I and/or class II genes and/or a gene encoding a factor involved in the expression thereof.

The cell preferably further comprises an exogenous nucleic acid encoding a chimeric antigen receptor.

The cell is preferably further deficient in a SIRPα gene.

The cell is preferably deficient in endogenous genes encoding major histocompatibility complex class I and/or class II and/or a gene encoding a factor involved in the expression thereof, and further comprises an exogenous nucleic acid encoding a desired major histocompatibility complex class I and/or class II.

In another embodiment, the present invention provides a composition for activating a T cell and/or a NK cell, comprising the above-descried cell.

The composition preferably induces interferon-γ production by the T cell and/or the NK cell.

In another embodiment, the present invention provides a pharmaceutical composition for preventing or treating cancer in a subject, comprising the above-described cell.

In another embodiment, the present invention provides a method for preventing or treating cancer in a subject, comprising the step of administering the above-described cell to the subject.

The method preferably further comprises the step of administering an immune checkpoint inhibitor to the subject.

### EFFECTS OF INVENTION

The proliferative myeloid cell of the present invention, unlike monocyte-derived DC, can constitutively and abundantly produce IL-12p70 without requiring any specific stimulation or induction, and exhibits synergistic antitumor effects when used in combination with an immune checkpoint inhibitor. Thus, the proliferative myeloid cell of the present invention can exert a stronger therapeutic effect on cancer than monocyte-derived DC. Also, the proliferative myeloid cell of the present invention can activate T cells and NK cells even without antigen presentation, and hence, HLA may be removed. Therefore, the proliferative myeloid cell of the present invention is useful for developing a universal cell preparation for cancer treatment.

### BRIEF DESCRIPTION OF DRAWINGS

FIG. 1 is a schematic diagram illustrating a production schedule for mouse IL-12p70-producing proliferative myeloid cells (using feeder cells).
FIG. 2 is a schematic diagram illustrating the structure of an expression vector of mouse IL-12p70.
FIG. 3 is a cytogram illustrating the results obtained by analyzing, by flow cytometry, the expression of cell surface markers (CD11b, CD11c, F4/80, DEC205, Gr-1, and CD33) in pMC, IL12-pMC, and BM-DC.
FIG. 4 is a histogram illustrating the results obtained by analyzing, by flow cytometry, the expression of cell surface markers (MHC class I, MHC class II, CD40, CD80, and CD86) in pMC, IL12-pMC, and BM-DC.
FIG. 5 is a graph illustrating the results of MTT assay for evaluating proliferation of pMC and IL12-pMC in the presence of GM-CSF and/or M-CSF.
FIG. 6 is a graph illustrating the amounts of IL-12p70 produced by pMC, by IL12-pMC, and by BM-DC, each with or without OK432 stimulation.
FIG. 7 is a graph illustrating the amounts of IFN-γ produced from OVA peptide-specific CD8⁺ T cells induced by pMC, by IL12-pMC, and by BM-DC, each with or without OVA peptide loading.
FIG. 8 is a graph illustrating the amounts of IFN-γ produced from NK cells induced by pMC, by IL12-pMC, by BM-DC, or by OK432-stimulated BM-DC (OK432-DC) (all without OVA peptide loading).
FIG. 9 is a graph illustrating proliferation of OVA peptide-specific CD8⁺ T cells induced by pMC, by GM-pMC, by IL12-pMC, and by BM-DC, each with or without OVA peptide loading.
FIG. 10 is a graph illustrating the amounts of IFN-γ produced from OVA peptide-specific CD8⁺ T cells induced by pMC, by GM-pMC, by IL12-pMC, and by BM-DC, each with or without OVA peptide loading.
FIG. 11 is a histogram illustrating the results obtained by analyzing, by flow cytometry, target cells in the spleen from mice administered therapeutic cells (OVA peptide-loaded pMC, IL12-pMC, or BM-DC) (with OVA peptide loading (high eFluor670) or without loading (low eFluor670)).
FIG. 12 is a boxplot illustrating antigen-specific cytotoxicity (% specific lysis) *in vivo* induced by pMC, by IL12-pMC, and by BM-DC.
FIG. 13 is a graph illustrating temporal change of a tumor volume of OVA-expressing cancer cells subcutaneously inoculated in a mouse administered OVA peptide-loaded IL12-pMC or BM-DC, and in an untreated mouse.
FIG. 14 is a Kaplan-Meier plot illustrating a survival rate of each mouse in FIG. 13.
FIG. 15 illustrates photographs of the results of ELISpot assay for examining frequency of IFN-γ-producing cells in CD8⁺ T cells collected from a mouse administered with IL12-pMC or BM-DC loaded with OVA peptide or SIY peptide, and from a naive mouse.
FIG. 16 is a graph illustrating the number of spots in FIG. 15.
FIG. 17 is a graph illustrating temporal change of a tumor volume of OVA-expressing cancer cells subcutaneously inoculated in a mouse administered with an isotype control antibody, an anti-CD8α antibody, or an anti-NK1.1 antibody in addition to OVA peptide-loaded IL12-pMC.
FIG. 18 is a Kaplan-Meier plot illustrating a survival rate of each mouse in FIG. 17.
FIG. 19 is a graph illustrating temporal changes of tumor volumes of OVA-expressing cancer cells subcutaneously inoculated in mice administered with ICIs and/or OVA peptide-loaded IL12-pMC, and in untreated mice.
FIG. 20 is a Kaplan-Meier plot illustrating a survival rate of each mouse group in FIG. 19.
FIG. 21 is a graph illustrating temporal changes of tumor volumes of AH1-expressing cancer cells subcutaneously inoculated in mice administered ICIs and/or AH1 peptide-loaded IL12-pMC, and in untreated mice.
FIG. 22 is a Kaplan-Meier plot illustrating a survival rate of each mouse group in FIG. 21.
FIG. 23 is a graph illustrating the number of spots detected by ELISpot assay for examining a frequency of IFN-γ-producing cells in CD8⁺ T cells collected from mice administered ICIs and AH1 peptide- or β-gal peptide-loaded IL12-pMC, and from naive mice.
FIG. 24 is a cytogram illustrating the results obtained by analyzing, by flow cytometry, the expression of CD8 and NK1.1 in CD45⁺ cells in cancer tissues of ICIs/OVA peptide-loaded IL12-pMC-administered mice, ICIs-administered mice, OVA peptide-loaded IL12-pMC-administered mice, and untreated mice.
FIG. 25 is a boxplot illustrating ratios of CD8⁺ T cells and NK cell to CD45⁺ cells in the cancer tissues of each mouse group in FIG. 24.
FIG. 26 is a cytogram illustrating the results obtained by analyzing, by flow cytometry, the expression of perforin and granzyme B in CD8⁺ T cells in cancer tissues from ICIs/OVA peptide-loaded IL12-pMC-administered mice, ICIs-administered mice, OVA peptide-loaded IL12-pMC-administered mice, and untreated mice.
FIG. 27 is a boxplot illustrating the percentage of perforin-positive and granzyme B-positive cells to CD8⁺ T cells in the cancer tissues of each mouse group in FIG. 26.
FIG. 28 is a cytogram illustrating the results obtained by analyzing, by flow cytometry, the expression of perforin and granzyme B in NK1.1⁺ cells in cancer tissues of ICIs/OVA peptide-loaded IL12-pMC-administered mice, ICIs-administered mice, OVA peptide-loaded IL12-pMC-administered mice, and untreated mice.
FIG. 29 is a boxplot illustrating the percentage of perforin-positive and granzyme B-positive cells to NK1.1⁺ cells in the cancer tissues of each mouse group in FIG. 28.
FIG. 30 is a graph illustrating temporal change of IL-12p70 concentration in blood of mice administered OVA peptide-loaded IL12-pMC.
FIG. 31 is a graph illustrating temporal change of IFN-γ concentration in blood of mice administered OVA peptide-loaded IL12-pMC.
FIG. 32 is a graph illustrating temporal change in tumor volume of OVA-expressing cancer cells subcutaneously inoculated in ICIs/OVA peptide-loaded IL12-pMC-administered mice, ICIs/OVA peptide-loaded IL12-pMC/anti-IFNγR antibody-administered mice, and untreated mice.
FIG. 33 is a graph illustrating a tumor volume on day 21 from subcutaneous inoculation of OVA-expressing cancer cells in ICIs/OVA peptide-loaded IL12-pMC-administered mice, and ICIs/OVA peptide-loaded IL12-pMC/anti-IFNγR antibody-administered mice.
FIG. 34 is a Kaplan-Meier plot illustrating a survival rate of each mouse group in FIG. 32.
FIG. 35 is a schematic diagram illustrating a production schedule (not using feeder cells) for human IL-12p70-producing proliferative myeloid cells.
FIG. 36 is a schematic diagram illustrating the structure of an expression vector of human IL-12p70.
FIG. 37 is a histogram illustrating the results obtained by analyzing, by flow cytometry, the expression of CD19 in human pMC and human IL12-pMC.
FIG. 38 is a histogram illustrating the results obtained by analyzing, by flow cytometry, the expression of cell surface markers (HLA class I, HLA class II, CD40, CD80, CD83 and CD86) in human pMC and human IL12-pMC.
FIG. 39 is a graph illustrating the results of MTT assay for evaluating proliferation of human pMC and human IL12-pMC in the presence of GM-CSF and/or M-CSF.
FIG. 40 is a graph illustrating the amount of IL-12p70 produced by human DC, human pMC, and human IL12-pMC, each with or without poly (I:C) stimulation.
FIG. 41 is a graph illustrating the amount of IL-10 produced by human DC, human pMC, and human IL12-pMC, each with or without poly (I:C) stimulation.
FIG. 42 is a graph illustrating the amount of IFN-γ produced from hTERT peptide-specific CD8⁺ T cells induced by human pMC, human GMM-pMC, or human IL12-pMC, each with or without hTERT peptide loading.
FIG. 43 is a graph illustrating the amount of IFN-γ produced from alloreactive T cells mixed with human pMC, human IL12-pMC, human DC, or poly (I:C)-stimulated human DC, in various ratios.
FIG. 44 is a graph illustrating the amount of IFN-γ produced from NK cells induced by human pMC, human IL12-pMC, human DC, or poly (I:C)-stimulated human DC.
FIG. 45 is a graph illustrating cell proliferation of human IL12-pMC or human pMC, exposed or not were irradiated in the presence or absence of GM-CSF and M-CSF.
FIG. 46 is a diagram illustrating the results of evaluation of tumorigenicity of nonirradiated human IL12-pMC in the body of severely immunodeficient mice, based on luciferase bioluminescence.
FIG. 47 is a diagram illustrating the results of evaluation of tumorigenicity of irradiated human IL12-pMC in the body of severely immunodeficient mice, based on luciferase bioluminescence.
FIG. 48 is a graph illustrating temporal change of a tumor volume of MO4 cells subcutaneously inoculated in the left leg and in the right leg of mice administered in the right leg with IL12-pMC (without peptide loading), and of untreated mice.
FIG. 49 is a graph illustrating temporal change of the median of the tumor diameter in each mouse group of FIG. 48.
FIG. 50 is a Kaplan-Meier plot illustrating a survival rate of each mouse group in FIG. 48.
FIG. 51 is a graph illustrating temporal change of a tumor volume of MO4 cells subcutaneously inoculated in the left leg and the right leg of mice administered IL12-pMC (without peptide loading) and/or anti-PD-L1 antibody, and of untreated mice.
FIG. 52 is a graph illustrating temporal change of the median of the tumor diameter in each mouse group of FIG. 51.
FIG. 53 is a Kaplan-Meier plot illustrating a survival rate of each mouse group in FIG. 51.
FIG. 54 is a cytogram illustrating the results obtained by analyzing, by flow cytometry, the expression of H-2Kb/H-2Db and I-Ab in pMC and pMC-Univ, each with or without IFN-γ stimulation.
FIG. 55 is a schematic diagram illustrating the structure of an expression vector of membrane-bound mouse IL-12.
FIG. 56 is a cytogram illustrating the results obtained by analyzing, by flow cytometry, the expression of IL-12 on the cell surface of pMC, pMC-Univ, mbIL12-pMC, and mbIL12-pMC-Univ.
FIG. 57 is a schematic diagram illustrating the structure of an expression vector of GC33-based chimeric antigen receptor (mouse).
FIG. 58 is a cytogram illustrating the results obtained by analyzing, by flow cytometry, the expression of CD19, SIRPα, and IL-12 on the cell surface of, and GPC3 binding to, pMC-Univ, pMC-Univ-SIRPα-KO, pMC-Univ-GC33-Z, and pMC-Univ-SIRPα-KO-GC33-Z.
FIG. 59 is a cytogram illustrating the results obtained by analyzing, by flow cytometry, the expression of CD19, SIRPα, and IL-12 on the cell surface of, and GPC3 binding to, mbIL12-pMC-Univ, mbIL12-pMC-Univ-SIRPα-KO, mbIL12-pMC-Univ-GC33-Z, and mbIL12-pMC-Univ-SIRPα-KO-GC33-Z.
FIG. 60 is a cytogram illustrating the results obtained by analyzing, by flow cytometry, the expression of GPC3 on the cell surface of MC38-Luc-GPC3.
FIG. 61 is a graph comparing cytotoxicity on a cancer cell model of pMC-Univ, pMC-Univ-SIRPα-KO, pMC-Univ-GC33-Z, and pMC-Univ-SIRPα-KO-GC33-Z.
FIG. 62 is a graph comparing cytotoxicity on a cancer cell model of mbIL12-pMC-Univ, mbIL12-pMC-Univ-SIRPα-KO, mbIL12-pMC-Univ-GC33-Z, and mbIL12-pMC-Univ-SIRPα-KO-GC33-Z.
FIG. 63 is a graph comparing the ability of pMC-Univ, pMC-Univ-SIRPα-KO-GC33-Z, mbIL12-pMC-Univ, and mbIL12-pMC-Univ-SIRPα-KO-GC33-Z to induce IFN-γ production by NK cells or T cells.
FIG. 64 is a cytogram illustrating the results of confirmation, by flow cytometry, of lack of H-2Kb/H-2Db in MC38-Luc-GPC3-H-2Kb/H-2Db-KO.
FIG. 65 is a diagram illustrating the results of *in vivo* imaging of an MC38-Luc-GPC3-H-2Kb/H-2Db-KO cell-inoculated mice untreated or administered therapeutic cells (pMC-Univ, pMC-Univ-SIRPα-KO-GC33-Z, mbIL12-pMC-Univ, or mbIL12-pMC-Univ-SIRPα-KO-GC33-Z) (on day 14).
FIG. 66 is a diagram illustrating a total flux of luciferase luminescence of each mouse in FIG. 65.
FIG. 67 is a Kaplan-Meier plot illustrating a survival rate of each mouse in FIG. 65.
FIG. 68 is a diagram illustrating the results of *in vivo* imaging of MC38-Luc-GPC3-H-2Kb/H-2Db-KO cell-inoculated mice administered mbIL12-pMC-Univ, or mbIL12-pMC-Univ-SIRPα-KO-GC33-Z in various concentrations (on day 18).
FIG. 69 is a diagram illustrating a total flux of luciferase luminescence of each mouse of FIG. 68.
FIG. 70 is a graph illustrating the results of FIG. 69 with respect to each mouse group.
FIG. 71 is a histogram illustrating the results obtained by analyzing, by flow cytometry, the expression of cell surface markers (HLA-ABC, HLA-A2, HLA-A24, HLA-DR, and CD19) in human pMC, pMC-HLA-KO, pMC-HLA-KO-A2, pMC-HLA-KO-A24, IL12-pMC-HLA-KO-A2, and IL12-pMC-HLA-KO-A24.
FIG. 72 is a graph comparing the proliferative responses of pMC donor-T cells to human pMC, pMC-HLA-KO, pMC-HLA-KO-A2, and pMC-HLA-KO-A24.
FIG. 73 is a graph comparing the proliferative responses of donor A-T cells to human pMC, pMC-HLA-KO, pMC-HLA-KO-A2, and pMC-HLA-KO-A24.
FIG. 74 is a graph comparing the proliferative responses of donor B-T cells to human pMC, pMC-HLA-KO, pMC-HLA-KO-A2, and pMC-HLA-KO-A24.
FIG. 75 is a schematic diagram illustrating the structure of an expression vector of membrane-bound human IL-12.
FIG. 76 is a histogram illustrating the results obtained by analyzing, by flow cytometry, the expression of IL-12 on the cell surface of human pMC and human mbIL12-pMC.
FIG. 77 is a graph comparing the IL12 secretion ability of human pMC, human IL12-pMC, and human mbIL12-pMC.
FIG. 78 is a histogram illustrating the results obtained by analyzing, by flow cytometry, the expression of cell surface markers (CD115, CD116, IL12Rβ2, CD16, CD32, CD64, FcεRIα, SIRPα, and CD3) in human pMC, human IL-12-pMC, human mbIL12-pMC, and human DC.
FIG. 79 is a schematic diagram illustrating the structure of an expression vector of GC33-based chimeric antigen receptor (human).
FIG. 80 is a cytogram illustrating the results obtained by analyzing, by flow cytometry, the expression of CD19 and IL-12 and the binding of GPC3 on the cell surface of human pMC, human pMC-GC33-Z, human mbIL12-pMC, and human mbIL12-pMC-GC33-Z.
FIG. 81 is a graph comparing the ability of human pMC, human IL12-pMC, human mbIL12-pMC, human pMC-GC33-Z, and human mbIL12-pMC-GC33-Z to induce IFN-γ production in NK cells.
FIG. 82 is a graph comparing the ability of human pMC, human IL12-pMC, human mbIL12-pMC, human pMC-GC33-Z, and human mbIL12-pMC-GC33-Z to induce IFN-γ production in T cells.

### MODE FOR CARRYING OUT THE INVENTION

The present invention will now be specifically described, and it is to be noted that the present invention is not limited to embodiments described herein.

According to a first embodiment, the present invention provides a method for producing a proliferative myeloid cell that constitutively produces IL-12p70, comprising the steps of: (1) introducing a nucleic acid encoding c-MYC into a myeloid cell; (2) culturing a cell obtained by the step (1) in the presence of GM-CSF and M-SCF; and (3) introducing a nucleic acid encoding an IL-12p35 subunit and a nucleic acid encoding an IL-12p40 subunit into a cell obtained by the step (2).

The "myeloid cell" is a generic term referring to, among leukocytes, monocytes, macrophages, dendritic cells, and progenitor cells thereof. Myeloid cell-specific markers are known, and any combination thereof can define a myeloid cell. The myeloid cell used as a starting cell in the method of the present embodiment can be defined as, for example, CD11b⁺CD33⁺, and may preferably further express CCR1, CCR2, CCR5, CXCR2, CXCR4, CD172a, CD205, or CD206.

The myeloid cell according to the present embodiment may be derived from any vertebrate, and is preferably derived from a mammal such as a mouse, a rat, a rabbit, sheep, a goat, a pig, a cow, a monkey, or a human, and is particularly preferably derived from a human.

The myeloid cell according to the present embodiment may be isolated from peripheral blood, or may be differentiated from a stem cell, and is preferably differentiated from a stem cell. The "stem cell" refers to a cell having self-renewal ability and differentiation ability, and is classified, in accordance with the differentiation ability, into a totipotent stem cell, a pluripotent stem cell, a unipotent stem cell, and the like. The myeloid cell according to the present embodiment may be differentiated from any one of the stem cells, and is preferably differentiated from a pluripotent stem cell. Examples of the "pluripotent stem cell" include an embryonic stem (ES) cell, an induced pluripotent stem (iPS) cell, an embryonic germ (EG) cell, and a pluripotent germ (mGS) cell. The myeloid cell according to the present embodiment may be differentiated from any of the pluripotent stem cells, and is preferably differentiated from an ES cell or an iPS cell, and is more preferably differentiated from an iPS cell.

The methods for preparing a pluripotent stem cell have been sufficiently established (for example, as for an iPS cell, Cell, 2007; 131(5): 861-872, and Stem Cell Reports, 2016; 6: 213-227), and a pluripotent stem cell can be prepared in accordance with a method known in this field. Alternatively, an iPS cell line or an ES cell line having been established may be obtained from, for example, Kyoto University CiRA Foundation (CiRA_F), RIKEN BioResource Center (RIKEN BRC), American Type Culture Collection (ATCC), or the like.

The methods for inducing a pluripotent stem cell into a myeloid cell have been already established (for example, WO2021/230304), and a myeloid cell can be prepared in accordance with a method known in this field. For example, pluripotent stem cells are induced into mesodermal cells by culturing the pluripotent stem cells on feeder cells such as an OP9 cell, which have the effect of causing the pluripotent stem cells to differentiate into blood cells. Alternatively, without using feeder cells, pluripotent stem cells are cultured in suspension in a medium containing VEGF, bFGF, BMP4, etc., to form an embryoid body containing many mesodermal cells. Then, by culturing the mesodermal cells in the presence of GM-CSF, it is possible to induce the mesodermal cells into myeloid cells.

In a method of the present embodiment, a nucleic acid encoding c-MYC is introduced into a myeloid cell. Optionally, a nucleic acid encoding BMI1, a nucleic acid encoding MDM2, a nucleic acid encoding LYL1, or a nucleic acid encoding BCL2 isoform alpha may be further introduced. A "c-MYC" (also referred to as "MYC" or "human MYC" for the human form), "BMI1" and "MDM2" are all well known as factors that control cell proliferation. "LYL1" is known as a leukemia-related transcription factor. "BCL2 isoform alpha" is known as an apoptosis controlling factor. All of c-MYC, BMI1, MDM2, LYL1, and BCL2 isoform alpha used in the present embodiment may be derived from any vertebrate, and are preferably derived from a mammal such as a mouse, a rat, a rabbit, sheep, a goat, a pig, a cow, a monkey, or a human, and are particularly preferably derived from a human.

Genes respectively encoding c-MYC, BMI1, MDM2, LYL1, and BCL2 isoform alpha have been already cloned, and nucleic acid sequence information thereof is available from a designated database. For example, NM_002467 can be used for a human MYC gene, NM_010849.4 can be used for a mouse c-MYC gene, NM_005180 can be used for a human BMI1 gene, NM_002392 can be used for a human MDM2 gene, NM_005583.5 can be used for a human LYL1 gene, and NM_000633 can be used for a human BCL2 isoform alpha gene (all NCBI Ref Seq IDs).

The c-MYC, BMI1, MDM2, LYL1, and BCL2 isoform alpha used in the present embodiment may encompass variants and homologues thereof having equivalent activities. In other words, c-MYC, BMI1, MDM2, LYL1, and BCL2 isoform alpha according to the present embodiment can include proteins consisting of amino acid sequences having 80% or more, preferably 90% or more, and more preferably about 95% or more sequence identity to the amino acid sequences registered in the database, as long as the cell proliferation control activity is maintained. The amino acid sequence identity can be calculated with sequence analysis software, or a program commonly used in this field (such as FASTA, or BLAST). Also, c-MYC, BMI1, MDM2, LYL1, and BCL2 isoform alpha according to the present embodiment can include a protein consisting of an amino acid sequence obtained by substituting, deleting, inserting, and/or adding one to several amino acids in the amino acid sequence registered in the database, as long as the cell proliferation control activity is retained. Here, the term "one to several" may be, for example, 1 to 30, preferably 1 to 10, and particularly preferably 1 to 5.

The nucleic acid encoding each of c-MYC, BMI1, MDM2, LYL1, and BCL2 isoform alpha can be introduced into a myeloid cell by a method known in this field, and for example, the nucleic acid may be cloned to an expression vector to be introduced into a myeloid cell. Examples of usable expression vectors include, but are not limited to, viral vectors such as a retrovirus, lentivirus, adenovirus, and Sendai virus, and plasmid vectors such as pCMV. Also, the nucleic acids encoding c-MYC, BMI1, MDM2, LYL1, and BCL2 isoform alpha may be introduced respectively with individual vectors, or may be introduced with a single polycistronic vector.

In the method of the present embodiment, the expression vector can be introduced into a cell by a method known in this field in accordance with the type thereof. For example, a non-viral vector can be introduced by lipofection, electroporation, microinjection or the like. A viral vector can be introduced by infecting a cell at an appropriate titer or multiplicity of infection (MOI).

Next, the myeloid cell into which the nucleic acid encoding c-MYC has been introduced is cultured in the presence of GM-CSF and M-CSF, thereby obtaining proliferative myeloid cells. Here, the term "proliferative" refers to cells being able to be cultured for a long period of time (for example, at least 3 months).

"GM-CSF" (granulocyte macrophage colony stimulating factor) and "M-CSF" (macrophage colony stimulating factor) are both known as a factor for promoting differentiation and proliferation of granulocyte/monocyte progenitor cells. GM-CSF and M-CSF used in the present embodiment may be derived from any vertebrate, and are preferably derived from a mammal such as a mouse, a rat, a rabbit, sheep, a goat, a pig, a cow, a monkey, or a human, and are particularly preferably derived from a human.

GM-SCF and M-SCF are commercially available, and such products can be used in the present embodiment. Preferable examples of the commercially available products include Recombinant Human M-CSF (R&D Systems, 216-MC), Recombinant mouse M-CSF (R&D Systems, 416-ML), Recombinant Human GM-CSF (R&D Systems, 215-GM), and Recombinant mouse GM-SCF (R & D Systems, 415-ML).

The concentrations of GM-CSF and M-CSF in the culture can be appropriately determined. The concentration of GM-CSF may be, for example, 10 ng/mL to 200 ng/mL, can be preferably 30 ng/mL to 150 ng/mL, more preferably 30 ng/mL to 100 ng/mL, and is further preferably 30 ng/mL to 50 ng/mL, and most preferably 50 ng/mL. The concentration of M-CSF may be, for example, 10 ng/mL to 200 ng/mL, can be preferably 30 ng/mL to 150 ng/mL, more preferably 50 ng/mL to 100 ng/mL, and is further preferably 50 ng/mL to 80 ng/mL, and most preferably 50 ng/mL.

The medium for culturing the myeloid cell may be, for example, αMEM, DMEM, IMDM, or the like, and one medium selected therefrom can be used alone, or two or more types thereof can be used as a mixture. The myeloid cells may be seeded at a concentration in a range of, for example, 1×10⁵/mL to 5×10⁵/mL. The period of the culture in the presence of GM-CSF and M-CSF may be, for example, 14 to 180 days, and may be preferably 20 to 60 days. Regarding the culture conditions, for example, in the case of mammalian-derived myeloid cells, the cells are preferably cultured under conditions of 37°C and 5% CO₂.

Next, a nucleic acid encoding an IL-12p35 subunit, and a nucleic acid encoding an IL-12p40 subunit are introduced into the proliferative myeloid cell, and thereby IL-12p70 (also simply referred to as "IL-12"), which is a heterodimer containing the IL-12p35 subunit and the IL-12p40 subunit, is produced. "IL-12p70" is a cytokine activating T cells and NK cells, and induces IFN-γ production by the T cells and the NK cells.

The IL-12p35 subunit and the IL-12p40 subunit used in the present embodiment may be derived from any vertebrate, and are preferably derived from a mammal such as a mouse, a rat, a rabbit, sheep, a goat, a pig, a cow, a monkey, or a human, and are particularly preferably derived from a human.

A gene encoding the IL-12p35 subunit and a gene encoding the IL-12p40 subunit have been already cloned, and nucleic acid sequence information thereof is available from a designated database. For example, NM_000882.4 can be used for a human IL-12p35 subunit gene, NM_001159424 can be used for a mouse IL-12p35 subunit gene, NM_002187.3 can be used for a human IL-12p40 subunit gene, and NM_001303244 can be used for a mouse IL-12p40 subunit gene (all NCBI Ref Seq IDs).

The IL-12p35 subunit and the IL-12p40 subunit used in the present embodiment may encompass variants and homologues thereof having equivalent activities. In other words, the IL-12p35 subunit and the IL-12p40 subunit used in the present embodiment can include proteins consisting of amino acid sequences having 80% or more, preferably 90% or more, and more preferably about 95% or more sequence identity to the amino acid sequence registered in the database, as long as IL-12p70 retaining cytokine activity can be constituted. Also, the IL-12p35 subunit and the IL-12p40 subunit used in the present embodiment can include a protein consisting of an amino acid sequence obtained by substituting, deleting, inserting, and/or adding one to several amino acids in the amino acid sequence registered in the database, as long as IL-12p70 retaining cytokine activity can be constituted. The term "one to several" means the same as defined above.

The nucleic acids encoding the IL-12p35 subunit and the IL-12p40 subunit may be introduced into a cell with an appropriate expression vector by a method known in this field, in the same manner as the nucleic acids encoding c-MYC, BMI1, MDM2, LYL1, and BCL2 isoform alpha.

Optionally, the nucleic acids encoding the IL-12p35 subunit and the IL-12p40 subunit according to the present embodiment may be fused to a single open reading frame via a nucleic acid encoding a peptide linker. In other words, the IL-12p35 subunit and the IL-12p40 subunit according to the present embodiment may be linked via a peptide linker. The length of the peptide linker is not especially limited, and may be, for example, 5 to 20 amino acids in length. The sequence of the peptide linker is not also especially limited, and it may be a linker sequence generally used in this field, and for example, a GS linker, a GG linker, a GGS linker, or the like can be used.

Optionally, a nucleic acid encoding a transmembrane domain may be added to the nucleic acids encoding the IL-12p35 subunit and the IL-12p40 subunit according to the present embodiment. In other words, the IL-12p70 produced in the present embodiment may be a cell membrane-bound IL-12 that is fused to a transmembrane domain. Examples of the transmembrane domain usable in the present embodiment include a transmembrane domain of CD80, a transmembrane domain of CD83, a transmembrane domain of CD86, a transmembrane domain of CD4, and a transmembrane domain of CD8α.

According to the method of the present embodiment, proliferative myeloid cells that constitutively produce IL-12p70 can be obtained.

In other words, according to a second embodiment, the present invention provides a proliferative myeloid cell, produced by the above-described method, that constitutively produces IL-12p70.

In other words, according to a third embodiment, the present invention provides a proliferative myeloid cell that constitutively produces IL-12p70, and contains an exogenous nucleic acid encoding c-MYC, an exogenous nucleic acid encoding the IL-12p35 subunit, and an exogenous nucleic acid encoding the IL-12p40 subunit. The terms "c-MYC", "IL-12p35 subunit", "IL-12p40 subunit", "IL-12p70", "proliferative", and "myeloid cell" used in the present embodiment are the same as those defined in the first embodiment. The proliferative myeloid cell of the present embodiment may be produced, for example, by the method of the first embodiment, and accordingly, the proliferative myeloid cell of the present embodiment may proliferate in GM-CSF and M-CSF-dependent manners.

Here, the phrase "constitutively produce IL-12p70" means that the production amount of IL-12p70 is constitutive regardless of extracellular stimulation. Usually, in order for macrophages and dendritic cells to produce IL-12p70, induction, e.g., by CD40-CD40 ligand interaction or toll-like receptor stimulation, is required. In contrast, the proliferative myeloid cell according to the second and third embodiments can stably produce IL-12p70 without such induction. Also, the phrase "produce IL-12p70" refers to IL-12p70 being extracellularly secreted, or IL-12p70 being expressed on the cell surface when it is of the cell membrane-bound type.

"CD40" (known also as TNFRSF5) is a member of the TNF receptor superfamily expressed in an antigen presenting cell such as a B cell, a dendritic cell, or a macrophage. "CD40 ligand (CD40L)" (known also as TNFSF5, gp39, or CD154) is a member of the TNF superfamily expressed mainly in an activated CD4-positive T cell, and induces IL-12 production by an antigen presenting cell through interaction with CD40.

"Toll-like receptor (TLR)" is a receptor playing a key role in innate immune responses, and 12 types (TLR1 to TLR9, and TLR11 to TLR13) have been identified in a mouse, and 10 types (TLR1 to TLR10) have been identified in a human. Activation of NF-κB is caused by activation of any one of the TLRs, and thus, production of inflammatory cytokines including IL-12 can be induced. Various TLR ligands have been identified, and examples include, but are not limited to, Pam3CSK4 (TLR1/TLR2 agonist), MALP-2 (TLR2/TLR6 agonist), polyinosinic-polycytidylic acid (poly(I:C)) (TLR3 agonist), lipopolysaccharide (LPS) (TLR4 agonist), flagellin (TLR5 agonist), imiquimod (TLR7 agonist), resiquimod (R-848) (TLR7/TLR8 agonist), and CpG-DNA (TLR9 agonist).

The proliferative myeloid cell of the second and third embodiments can produce, in the absence of CD40-CD40 ligand interaction or TLR stimulation, IL-12p70 in an amount of at least 300 pg/1×10⁴ cells/24 hours, at least 750 pg/1×10⁴ cells/24 hours, or at least 1000 pg/1×10⁴ cells/24 hours, and can produce IL-12p70 in an amount of, for example, 300 to 2000 pg/1×10⁴ cells/24 hours, preferably 750 to 2000 pg/1×10⁴ cells/24 hours, more preferably 750 to 1800 pg/1×10⁴ cells/24 hours, and particularly preferably 1000 to 1500 pg/1×10⁴ cells/24 hours.

The above IL-12p70 production can be at least 3-fold, and can be preferably at least 4-fold the IL-12p70 production by monocyte-derived dendritic cells with TLR stimulation.

Here, the term "monocyte-derived dendritic cell" refers to a dendritic cell induced from a monocyte isolated from peripheral blood. Monocyte-derived dendritic cells are used in dendritic cell-based cancer vaccines (so-called DC vaccines) in cancer immunotherapy, and the methods for their preparation are well established. Specifically, monocytes isolated from peripheral blood may be induced into dendritic cells by culturing in the presence of GM-CSF and IL-4. A monocyte-derived dendritic cell can be defined based on expression of a marker, and may be defined as, for example, CD14-negative, CD40-positive, CD80-positive, CD83-positive, CD86-positve, or HLA-ABC-positive and/or HLA-DR-positive.

The monocyte-derived dendritic cell as a control for comparing the IL-12p70 production may be derived from any vertebrate, and is preferably derived from the same animal as that from which the proliferative myeloid cell to be compared is derived. For example, a mouse proliferative myeloid cell is preferably compared with a mouse monocyte-derived dendritic cell, and a human proliferative myeloid cell is preferably compared with a human monocyte-derived dendritic cell.

TLR stimulation for inducing the IL-12p70 production by the monocyte-derived dendritic cell may be a single one of, or a combination of two or more TLR ligands defined above, and may be preferably poly (I:C) stimulation.

The proliferative myeloid cell of the second and third embodiments can produce IL-12p70 in the absence of CD40-CD40 ligand interaction, and hence, there is no need to express CD40. In addition, the proliferative myeloid cell of the second and third embodiments need not express a major histocompatibility complex (MHC) class I and/or class II molecule. This is because the proliferative myeloid cell of the second and third embodiments constitutively produces IL-12p70, and therefore, unlike normal macrophages and dendritic cells, can activate T cells and NK cells without presenting antigens. A cell that does not express an MHC class I and/or class II molecule is referred to as a "universal cell" or "universal type cell".

A proliferative myeloid cell that does not express an MHC class I and/or class II molecule can be prepared by deleting an MHC class I and/or class II gene, and/or deleting a gene encoding a factor involved in the expression of an MHC class I and/or class II molecule. Examples of the factor involved in the expression of an MHC class I include β2-microglobulin and TAP (TAP1, and TAP2). An example of the factor involved in the expression of an MHC class II includes MHC class II transactivator (CIITA). Techniques for preparing a cell that does not express an MHC class I and/or class II molecule by deleting such a gene have been sufficiently established. For example, for a human proliferative myeloid cell, the cell that does not express an HLA class I and class II molecule can be prepared by disrupting, through genome editing, HLA-A gene, HLA-B gene, HLA-C gene, B2M gene and/or CIITA gene (for example, WO2019/160077, and WO2020/260563).

In the proliferative myeloid cell of the second and third embodiments, in addition to the defect in an endogenous MHC class I and/or class II gene and/or genes encoding factors involved in the expression thereof, an exogenous nucleic acid encoding a desired MHC class I and/or class II may be introduced. The desired MHC class I and/or class II may be any one as long as it can present a cancer antigen, and may be preferably selected to match with an MHC class I and/or class II in a majority of subjects to which the proliferative myeloid cell is to be administered.

Regarding humans, HLA frequency deviation according to race or region has been analyzed (for example, for Japan, https://hla.orjp/about/hla/), and a desired HLA gene can be selected based on HLA types highly frequently found regardless of race or region. Examples of such HLA types include HLA-A1, A2, A3, A11, A23, A24, A26, A29, A30, A31, A32, A33, A68, B7, B8, B13, B14, B15, B18, B27, B35, B38, B39, B40, B44, B46, B48, B51, B54, B55, B57, B58, B61, B62, B71, B75, C1, C2, C3, C4, C5, C6, C7, C8, C12, C14, C15, and C16. It may be selected preferably from the group consisting of HLA-A1, A2, A3, A11, A24, A26, A30, A33, A68, B7, B8, B13, B35, B40, B44, B46, B51, B58, C1, C3, C4, C6, C7, C12, and C14, and selected particularly preferably from the group consisting of HLA-A1, A2, A11, and A24.

The proliferative myeloid cell of the second and third embodiments may further contain an exogenous nucleic acid encoding a chimeric antigen receptor. The "chimeric antigen receptor" (hereinafter also referred to as "CAR") is an artificial receptor molecule containing an antigen binding domain, a transmembrane domain, and an intracellular signaling domain. The antigen binding domain may contain antigen-specific monoclonal antibody-derived VH and VL, which can be linked to each other via a linker (that is, a scFv). The transmembrane domain may be a transmembrane domain of CD4, CD8α, CD80, CD83, or CD86. The intracellular signaling domain may be an intracellular signaling domain of CD3ζ, CD16, CD19, CD32, CD40, CD64, CD80, CD83, CD86, FCERIG, FCGRI, NKp30, NKp44, NKp46, NKG2D, ITGAM, SLAMF7, Dectin-1, MARCO, DAP10, MEGF10, TLR1, TLR2, TLR3, TLR4, TLR5, TLR6, TLR7, TLR8, and TLR9, and preferably has an immunoreceptor tyrosine-based activation motif (ITAM).

Examples of an antigen targeted by CAR include CD10, CD19, CD123, CD20, CD22, CD25, CD30, CD33, CD34, CSPG4, CD37, CD45, CD115, CD117, CD133, CD135, CD140a, CD140b, CD309, CLEC14, NKG2D, SLAMF7, TRBC1, CD44V6, CD45, BCMA, CLL-1, NKG2D, SLAMF7, TACI, GD2, CLDN6/Claudin18.2, G250, CAMPATH-1, PSMA, FAP, T4, B7-H3, CA-IX, CEA, EGFR, EGFRvIII, IGFR, GPC3, HER-2, A33, HER-3, MUC-1, MMP-2, PSMA, PSCA, PSA, TAG72, EpCAM, IL3R, and MCSP. The proliferative myeloid cell of the second and third embodiments may express one of, or a plurality of, CARs recognizing antigens selected from those described above, in accordance with the type of cancer to be targeted.

The proliferative myeloid cell of the second and third embodiments may preferably be further deficient in the SIRPα gene. Here, the phrase "deficient in SIRPα gene" encompasses not only the gene being modified so that SIRPα cannot be expressed, but also the gene being modified so that a loss-of-function SIRPα is expressed. Modifications that cause loss of function of SIRPα include, for example, deletion of the intracellular signaling domain of SIRPα. The SIRPα gene can be deleted by methods well known in this field, e.g., by genome editing.

The proliferative myeloid cell of the second and third embodiments preferably expresses at least one costimulatory molecule selected from CD86 (also known as B7-2), CD80 (also known as B7-1), and CD83 (also known as HB15). CD86, CD80, and CD83 are all costimulatory molecules for activating T cells and NK cells, and when these are expressed, T cells and NK cells can be activated more effectively.

In addition, it is preferable that the proliferative myeloid cell of the second and third embodiments not produce IL-10 by TLR stimulation. IL-10 is an anti-inflammatory cytokine, and the immune reaction can be suppressed because macrophages and dendritic cells usually produce IL-10 by TLR stimulation. In contrast, when the proliferative myeloid cell of the second and third embodiments does not produce IL-10, the immune reaction is not suppressed, and cell-mediated immune responses can be more efficiently induced.

The proliferative myeloid cell of the second and third embodiments can produce IL-12p70 constitutively in large amount without requiring special stimulation or induction, and therefore, is useful for treatment of cancer and viral infectious diseases, etc. Also, the proliferative myeloid cell of the second and third embodiments need not express an MHC (HLA) molecule, and hence is useful for developing a universal cell preparation for treatment of cancer and viral infectious diseases, etc.

For example, as in Examples below, a human proliferative myeloid cell of a certain embodiment can produce 3-fold more IL-12p70 than poly(I:C)-stimulated human monocyte-derived dendritic cells, and does not produce IL-10 even with poly (I:C) stimulation. Alternatively, a human proliferative myeloid cell of a certain embodiment expresses IL-12p70 on the cell surface. Such a cell can very efficiently induce a cell-mediated immune response.

According to a fourth embodiment, the present invention provides a composition for activating a T cell and/or a NK cell, containing the proliferative myeloid cell.

"T cell" refers to a cell expressing a T cell receptor (TCR)/CD3 complex on the surface, and is roughly divided into a helper T (Th) cell (CD4⁺ T cell) and a cytotoxic T (Tc) cell (CB8⁺ T cell), and is further classified into various subsets based on expression of various immunophenotype markers. For example, Th cells are classified into Th1, Th2, Th9, Th17, Th22, follicular helper T (Tfh), etc., and Tc cells are classified into Tc1, Tc2, Tc9, Tc17, etc., but are not limited to these. The T cell according to the present embodiment may be of any subset, and is preferably a Tc cell (CD8⁺ T cell).

"NK cell" (natural killer cell) is a type of cytotoxic lymphocyte belonging to the innate lymphoid cell (ILC) family. NK cells do not express the TCR/CD3 complex and the B cell receptor (BCR) and can be defined, for example, as CD3⁻ CD56⁺.

In the present embodiment, "activating a T cell and/or a NK cell" refers to inducing or promoting the proliferation, differentiation, maturation, cytokine production, etc., of a T cell and/or a NK cell. The composition according to the present embodiment can induce the production of inflammatory cytokines, such as interferon γ (IFN-γ), TNF-α, CCL5, or XCL1, by T cells and/or NK cells; induce proliferation of T cells and/or NK cells; induce the expression of cytotoxic molecules (perforin or granzyme B) in T cells and/or NK cells; or promote the expression of CD69. The composition of the present embodiment can preferably induce IFN-γ production by T cells and/or NK cells.

The composition of the present embodiment contains the proliferative myeloid cell as an active ingredient. The composition of the present embodiment may consist of only the active ingredient, and may contain, as any component, a carrier, a buffer, or another component (such as synthetic peptide) that is pharmaceutically acceptable and is known. For example, the pharmaceutical composition of the present embodiment may be prepared as a liquid formulation using a carrier such as phosphate buffered saline that can maintain the viability of the cells that are the active ingredient. In this case, the proliferative myeloid cell may be suspended in the carrier at a concentration of, for example, 1×10⁶ to 1×10⁸ cells/mL.

Optionally, the proliferative myeloid cell according to the present embodiment may be irradiated. Irradiation can arrest proliferation of the proliferative myeloid cells, and can improve the *in vivo* safety of the composition of the present embodiment. The proliferative myeloid cell according to the present embodiment may be irradiated with, for example, 5 to 85 Gy, preferably 10 to 65 Gy, and more preferably 10 to 40 Gy X-rays or γ-rays.

In order to activate T cells or NK cells *in vitro* by the composition of the present embodiment, the composition of the present embodiment may be mixed with the T cells or the NK cells and be incubated for a certain period of time. The incubation time may be, for example, 12 to 72 hours. The incubation temperature can be appropriately set in accordance with the type of the cell to be used, and may be, for example, 37°C when using a mammalian cell.

In order to activate T cells or NK cells *in vivo* by the composition of the present embodiment, the composition of the present embodiment may be administered to a subject. The composition of the present embodiment can be administered by an appropriate method such as injection, infusion, or inoculation. Preferably, the composition of the present embodiment may be infused intravenously, subcutaneously, intradermally, into a lymph node, intraperitoneally, or into a cancer tissue. The dose of the composition of the present embodiment may be varied depending on the age, the weight, the health condition and the like of the subject, and may be, for example, 1×10⁵ to 1×10⁷ cells/kg (body weight). The dose may be administered once, or administered in divided doses a plurality of times.

The composition of the fourth embodiment contains the proliferative myeloid cell that constitutively produces IL-12p70 as the active ingredient, and hence, it is more easily prepared than ordinary DC vaccines, and in addition, has a higher ability to activate T cells and NK cells than an ordinary DC vaccine. Therefore, the composition is useful as a cell preparation for treatment of cancer and viral infectious diseases, etc.

For example, as in Examples below, human proliferative myeloid cells of a certain embodiment have a three-fold or higher ability to induce IFN-γ production in antigen-specific CD8⁺ T cells than other previously reported human proliferative myeloid cells, and a fifty-fold or higher ability to induce IFN-γ production in NK cells than poly(I:C)-stimulated human monocyte-derived dendritic cells. Such cells are very useful for a cell preparation for treatment of cancer and viral infectious diseases, etc.

According to a fifth embodiment, the present invention provides a pharmaceutical composition containing the proliferative myeloid cell for preventing or treating cancer in a subject.

In the present embodiment, "preventing" includes, not only preventing the onset of cancer in a subject who may develop cancer, but also reducing the risk of developing cancer, or delaying the progression or reducing the severity of symptoms if the subject develops cancer by treating the subject before the onset of cancer.

In the present embodiment, "treating" includes, not only completely curing cancer, but also remission or alleviation of symptoms of cancer, delaying or stopping the progression of cancer, and improving the life prognosis of cancer patients.

The "subject" of the present embodiment may be any vertebrate, is preferably a mammal such as a mouse, a rat, a rabbit, sheep, a goat, a pig, a cow, a monkey, or a human, and is particularly preferably a human. The subject can be of any age and may be an infant, child, adolescent, adult, and elderly subjects.

The proliferative myeloid cell according to the present embodiment may be preferably allogenic or autologous to the subject. Also, the proliferative myeloid cell according to the present embodiment may be optionally irradiated. The proliferative myeloid cell according to the present embodiment may be irradiated with, for example, 5 to 85 Gy, preferably 10 to 65 Gy, and more preferably 10 to 40 Gy X-rays or γ-rays.

The proliferative myeloid cell according to the present embodiment may be loaded with a cancer antigen when it expresses MHC class I and/or class II. Methods for loading a myeloid cell with a cancer antigen have been sufficiently established, and a cancer antigen can be loaded by methods known in this field. For example, the myeloid cell may be cultured in a medium containing a full-length or partial fragment of a cancer antigen peptide or a tumor lysate. Alternatively, a nucleic acid encoding a full-length or partial fragment of a cancer antigen peptide may be introduced into the cell using a viral vector or non-viral vector.

The cancer antigen usable in the present embodiment is not especially limited, and may be, for example, cancer-testis antigens listed in CTDatabase (http://www.cta.lncc.br) such as MPHOSPH1, CDCA1, DEPDC1, LY6K, IMP3, TTK, NY-ESO-1, and KK-LC-1; differentiation antigens such as gp100, Melan-A, and tyrosinase; overexpressed antigens such as HER2, CEA, and glypican 3; driver gene mutated antigens such as K-RAS and EGFR; mutated antigens such as survivin-2B and bcr/abl; viral antigens such as E6 and E7 derived from HPV and EBNA-2, 3, 4, and 6 derived from EBV; and neoantigens (mutant antigens newly expressed in individual cancer patients).

The pharmaceutical composition of the present embodiment may be prepared in the same manner as the composition of the fourth embodiment, and may contain the proliferative myeloid cell as the active ingredient, and may further contain any component.

The pharmaceutical composition of the present embodiment may contain, as any component, an anticancer agent, and an immune checkpoint inhibitor in addition to those defined in the fourth embodiment. The "immune checkpoint inhibitor" refers to a substance that binds to immune checkpoint molecules or ligands thereof and inhibits immunosuppressive signaling. The immune checkpoint inhibitor usable in the present embodiment is not especially limited, and may be, for example, an anti-PD-1 antibody, an anti-PD-L1 antibody, an anti-CTLA-4 antibody, an anti-B7 antibody, an anti-CD27 antibody, an anti-CD70 antibody, an anti-CD73 antibody, an anti-CD47 antibody, an anti-KIR antibody, an IDO inhibitor, an anti-CD137 antibody, an anti-TIM3 antibody, etc. The "anticancer agent" usable in the present invention may be any known anticancer agent, and may be, for example, methotrexate, actinomycin D, 5-fluorouracil, paclitaxel, pemetrexed, cisplatin, etc.

The pharmaceutical composition of the present invention can be administered by an appropriate method such as injection, infusion, or inoculation. Preferably, the pharmaceutical composition of the present embodiment may be infused intravenously, subcutaneously, intradermally, into a lymph node, or into a cancer tissue. The dose of the pharmaceutical composition of the present embodiment may be varied depending on the age, the weight, the severity of the cancer, and may be, for example, 1×10⁵ to 1×10⁷ cells/kg (body weight). The dose may be administered once, or be administered in divided doses a plurality of times.

According to a sixth embodiment, the present invention provides a method for preventing or treating cancer in a subject, including a step of administering the proliferative myeloid cell to the subject. The terms "preventing", "treating", "subject", and "proliferative myeloid cell" used in the present embodiment are the same as those defined in the fifth embodiment.

In the method of the present embodiment, the proliferative myeloid cell is formulated and administered as defined in the fourth and fifth embodiments. The dose of the proliferative myeloid cell is the same as that defined in the fourth and fifth embodiments.

The method of the present embodiment may further include a step of administering an immune checkpoint inhibitor. The "immune checkpoint inhibitor" used in the present embodiment is the same as that defined in the fifth embodiment.

The method of the present embodiment may further include a step of administering an anticancer agent. The "anticancer agent" used in the present embodiment is the same as that defined in the fifth embodiment.

The step of administering the proliferative myeloid cell, and the step of administering an anticancer agent or an immune checkpoint inhibitor can be performed consecutively or simultaneously. For example, an anticancer agent or an immune checkpoint inhibitor may be administered after administering the proliferative myeloid cell; the proliferative myeloid cell may be administered after administering an anticancer agent or an immune checkpoint inhibitor; or the proliferative myeloid cell and an anticancer agent or an immune checkpoint inhibitor may be simultaneously administered.

The pharmaceutical composition of the fifth embodiment, and the method of the sixth embodiment exhibit higher antitumor activity than general cancer treatment using a monocyte-derived DC, and in particular, exhibit synergistic antitumor activity when used together with an immune checkpoint inhibitor. Therefore, the pharmaceutical composition of the fifth embodiment and the method of the sixth embodiment are useful for treatment of intractable cancer that has been difficult to treat by a conventional method.

### EXAMPLES

The present invention will now be further described with reference to examples. It is noted that these examples do not limit the present invention at all.

### 1. Production of mouse IL-12-producing proliferative myeloid cells

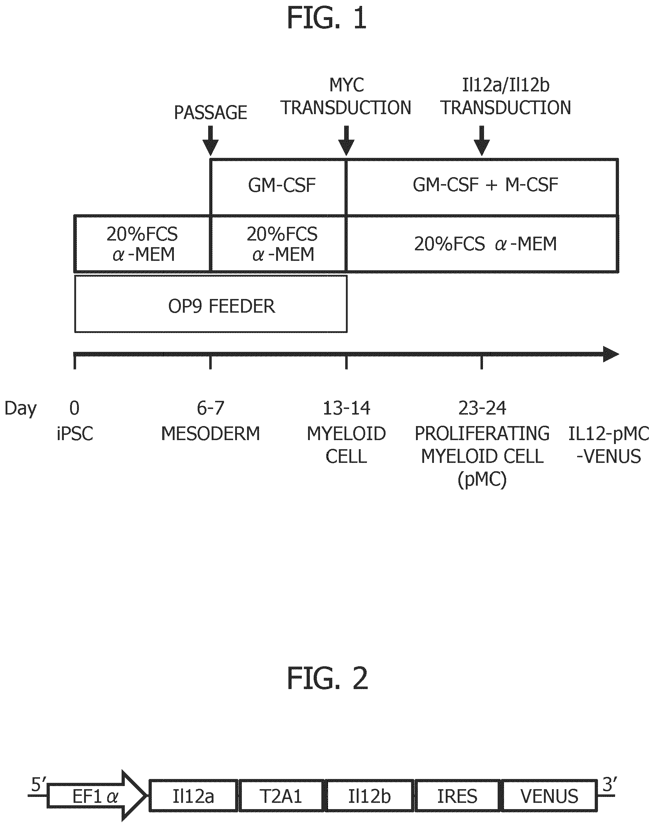

Mouse IL-12p70-producing proliferative myeloid cells were produced through the following procedures. The production schedule is illustrated in FIG. 1.

### (1-1) Production of mouse proliferative myeloid cells

C57BL/6N mouse iPS cells were produced by the method described by Araki R. et al. (Nature, 2013 Feb 7; 494 (7435): 100-4. doi: 10.1038/nature11807). Mouse proliferative myeloid cells were produced by the method described by Zhang R. et al. (Cancer Immunol Res. 2015 Jun; 3 (6): 668-77. doi: 10.1158/2326-6066. CIR-14-0117). Specifically, the iPS cell was cultured for 6 to 7 days on OP9 cells (purchased from RIKEN BRC CELL BANK) in 20% FCS-containing αMEM (Thermo Fisher Scientific) to induce differentiation into mesodermal cells. The mesodermal cells were collected and transferred onto fresh OP9 cells, and were cultured for 6 to 7 days in the presence of 20 ng/mL mouse GM-CSF recombinant (R&D Systems, 415-ML) to induce differentiation into myeloid-type cells. The myeloid-type cells (hereinafter also referred to as "MC") were collected and infected with a lentiviral vector containing the human c-MYC gene, and were cultured for 7 to 10 days in the presence of 30 ng/mL mouse GM-CSF recombinant and 50 ng/mL human M-CSF recombinant (Miltenyi Biotec). Myeloid-type cells that proliferate in GM-CSF/M-CSF-dependent manners appeared and were named "proliferative myeloid cells (pMC)". All cell cultures in this study were performed in an incubator at 37°C and 5% CO₂.

### (1-2) Production of a lentiviral vector expressing mouse IL-12p70

A fusion cDNA, which is mouse I112a and I112b cDNAs (Ref Seq IDs: NM_001159424 and NM_001303244; SEQ ID NOs: 1 and 2) connected by a nucleic acid sequence (sequence number 3) encoding the self-cleaving peptide T2A derived from *Thosea asigna virus* (TaV), was synthesized by entrusting GenScript. The fusion cDNA was inserted into the multicloning site of the CSII-EF-MCS-IRES2-Venus plasmid (RIKEN BRC DNA BANK) to obtain CSII-EF-I112a-T2A1-I112b-MCS-IRES2-Venus (FIG. 2).

CSII-EF-I112a-T2A1-I112b-MCS-IRES2-Venus, pCAG-HIVgp (RIKEN BRC DNA BANK), and pCMV-VSV-G-RSV-Rev (RIKEN BRC DNA BANK) were introduced into 293 T cells (purchased from RIKEN BRC CELL BANK) by lipofection. Three days after lipofection, the culture supernatant was collected and filtered through a filter with a pore size of 0.45 µm, and then the viral particles were concentrated using a Lenti-X concentrator (Clontech). The obtained viral particles were suspended in DMEM, dispensed into cryovials, and stored at -150°C.

### (1-3) Production of mouse IL-12p70-producing proliferative myeloid cells

The pMC produced in (1-1) above were seeded on 48-well plates, and were infected with the lentiviral vector produced in (1-2) above. For growth culture after the infection, αMEM containing 20% FCS, 30 ng/mL mouse GM-CSF recombinant, and 50 ng/mL human M-CSF recombinant (hereinafter referred to as the "pMC medium") was used. Eight days after the infection, Venus-positive cells were isolated by flow cytometry. The isolated Venus-positive cells continued to proliferate for 3 months or more. The cells were named "IL-12p70 producing proliferative myeloid cells (IL12-pMC)".

### 2. Surface marker analysis of mouse IL-12p70-producing proliferative myeloid cells

Expression of surface markers in the pMC and IL12-pMC produced in 1 above was analyzed by immunostaining and flow cytometry. In the immunostaining, the antibodies shown in Table 1 below were used. As a control, bone marrow-derived dendritic cells (BM-DC) prepared by the following procedures were used. Bone marrow cells (1.5×10⁶) from C57BL/6N mice were cultured in 90-mm petri dish for microorganisms. As the medium, RPMI 1640 (Sigma Aldrich) containing 10% FCS, 50 µg/mL mouse GM-CSF recombinant, and 50 µM 2-mercaptoethanol was used. Half of the medium was changed on days 3 and 6 after the start of the culture. On day 8, 50 µg/mL mouse IL-4 (BioLegend) was added thereto, and floating cells on the day 9 were used as the BM-DC.

### Table 1. Antibodies used for marker staining

**[Table 1]**

| Antigen | Species | Clone | Source |
|---|---|---|---|
| CD11b | Mouse | M1/70 | BioLegend |
| CD11c | Mouse | N418 | BioLegend |
| F4/80 | Mouse | BM8 | BioLegend |
| DEC205 | Mouse | NLDC-145 | BioLegend |
| Gr-1 | Mouse | RB6-8C5 | BioLegend |
| CD33 | Mouse | 9A11-CD33 | ThermoFisher Scientific |
| MHC Class-I (H-2Kb/Db) | Mouse | 28-8-6 | BioLegend |
| MHC Class-II (I-A/I-E) | Mouse | M5/114.15.2 | BioLegend |
| CD40 | Mouse | 1C10 | ThermoFisher Scientific |
| CD80 | Mouse | 16-10A1 | BioLegend |
| CD86 | Mouse | GL-1 | BioLegend |

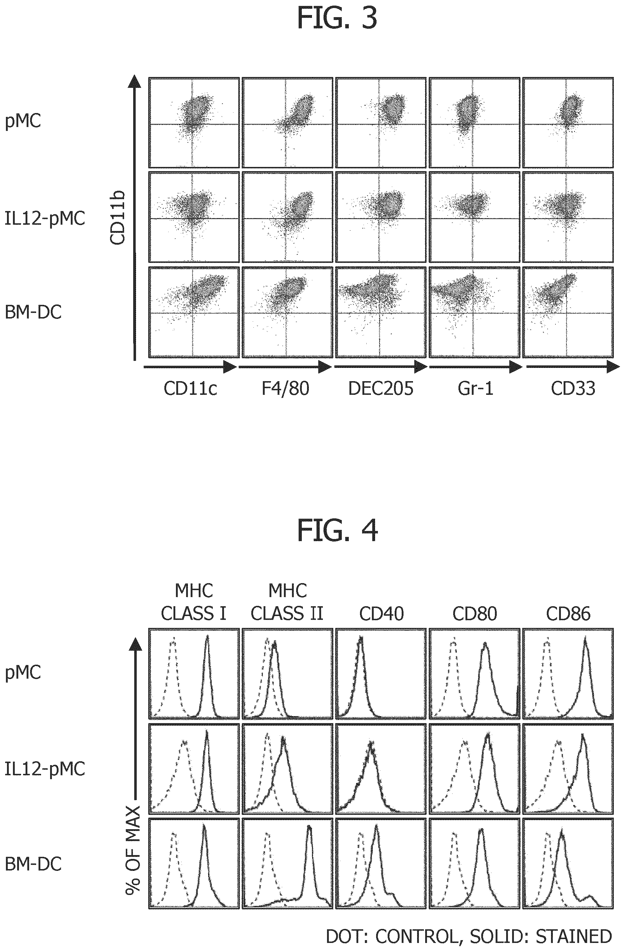

The results are shown in FIGS. 3 and 4. In FIG. 4, the dotted lines show the results obtained by staining with a rat IgG2b antibody (RTK4530) (negative control). Similar to the BM-DC, the pMC and the IL12-pMC expressed myeloid differentiation markers, macrophage markers, and dendritic cell markers, such as CD11b, CD11c, F4/80, DEC205, Gr-1, and CD33 (FIG. 3). In addition, similarly to the BM-DC, the pMC and the IL12-pMC expressed the antigen presentation molecules MHC class I and class II, as well as the costimulatory molecules CD80 and CD86 (FIG. 4). This suggests that the pMC and the IL12-pMC have T cell-stimulating activity. On the other hand, the pMC and the IL12-pMC did not express CD40, unlike the BM-DC (FIG. 4).

### 3. Characteristic analysis of mouse IL-12p70-producing proliferative myeloid cell

### (3-1) Proliferation ability

The pMC and the IL12-pMC produced in 1 above were seeded on 96-well plates (2×10³ cells/well) and cultured in 20% FCS-containing αMEM supplemented with mouse GM-CSF recombinant (30 ng/mL), human M-CSF recombinant (50 ng/mL), or a combination thereof. Proliferation in each culture condition was evaluated over time by MTT assay. Every 24 hours from the start of the culture, 0.5 mg/mL 3-(4,5-dimethyl-2-thiazolyl)- 2,5-diphenyl-2H-tetrazolium bromide (MTT) reagent (Merck Millipore) was added, and 4 hours later, the metabolized formazan was measured based on absorbance at a wavelength of 595 nm.

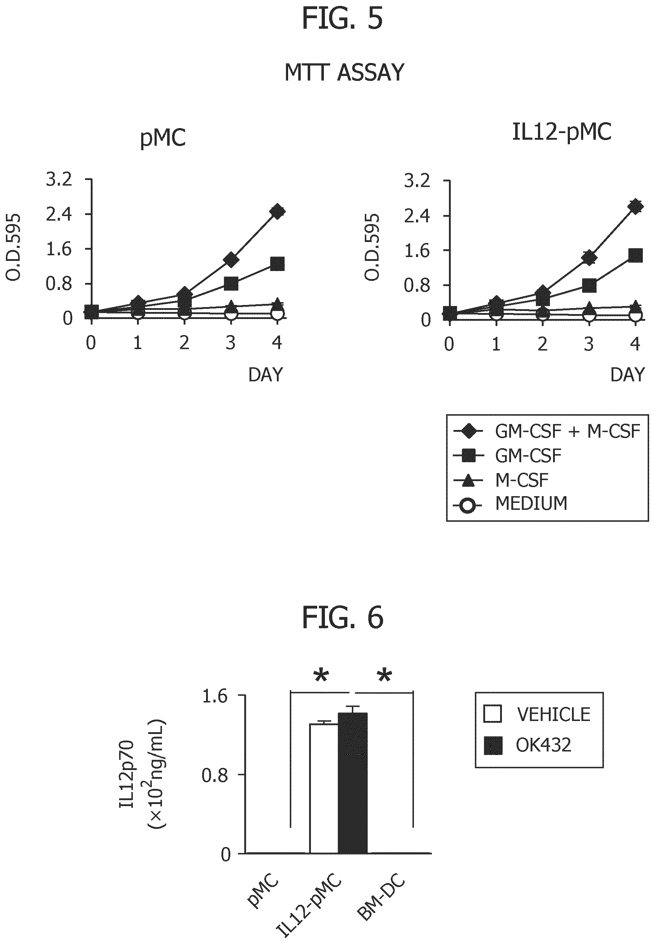

The results are shown in FIG. 5. The proliferation of the pMC and the IL12-pMC was GM-CSF dependent, and the proliferation was further enhanced by adding GM-CSF and M-CSF in combination to the medium. Furthermore, this proliferation continued for 3 months or more. These results indicate that pMC and IL12-pMC are unique cells whose proliferation can be controlled by GM-CSF and M-CSF, and that they can be mass-produced by using GM-CSF and M-CSF.

### (3-2) Production of IL-12p70

The pMC and the IL12-pMC produced in 1 above and the BM-DC produced in 2 above were seeded on 96-well plates (1.0×10⁴ cells/100 µL) by using 10% FCS-containing RPMI 1640. The cells were cultured for 24 hours after adding 5 µg/mL OK432 (picibanil, Chugai Pharmaceutical Co., Ltd.). Culture supernatants were collected, and IL-12p70 was quantified by ELISA MAX^{™} Deluxe Set Mouse IL-12 (p70) (BioLegend). The IL-12p70 production without adding OK432 (vehicle) was also measured in the same manner (control).

The results are shown in FIG. 6 and Table 2. In the figure, an asterisk (*) indicates a p-value obtained by one-way ANOVA with Tukey post-hoc test (*p < 0.05). Surprisingly, the IL12-pMC produced IL-12p70 regardless of the addition of OK432, and the amount produced was more than 100-fold higher than that of BM-DC with OK432. The pMC did not produce IL-12p70 regardless of the addition of OK432.

### Table 2. IL-12p70 production

**[Table 2]**

| | pMC | IL12-pMC | BM-DC |
|---|---|---|---|
| Vehicle | 0.0078 ng/ml | 129.99607 ng/ml | 0.0078 ng/ml |
| OK432 | 0.0078 ng/ml | 141.3043 ng/ml | 0.036 ng/ml |

### (3-3) Ability to induce IFN-γ production of antigen-specific CD8⁺ T cells

The pMC and the IL12-pMC produced in 1 above and the BM-DC produced in 2 above were loaded with 10 µM chicken ovalbumin 257-264 peptide (Eurofins Genomics K.K.) (hereinafter also referred to as the "OVA peptide"). After 16 hours, the resultant cells were seeded on 96-well plates (5.0×10⁴ cells/well), and were irradiated (85 Gy). OVA peptide-specific CD8⁺ T cells (hereinafter, also referred to as the "OT-I T cells") were isolated from splenocytes of OT-I mice (OVA peptide-specific T cell receptor transgenic mice, C57BL/6-Tg (TcraTcrb) 1100 Mjb/J, The Jackson Laboratory Japan, Inc.) by using CD8α⁺ T Cell Isolation Kit, mouse (Miltenyi Biotec). The OT-I T cells (1.0×10⁴ cells/well) were added to each of the wells of the pMC, the IL12-pMC, and the BM-DC, and were co-cultured for 3 days in RPMI 1640 containing 10% FCS and 2-mercaptoethanol (50 µM). Culture supernatants were collected, and IFN-γ was quantified using ELISA MAX^{™} Standard Set Mouse IFN-γ (BioLegend). The IFN-γ production in the absence of OVA peptide loading (vehicle) in each cells was also determined in the same way (control).

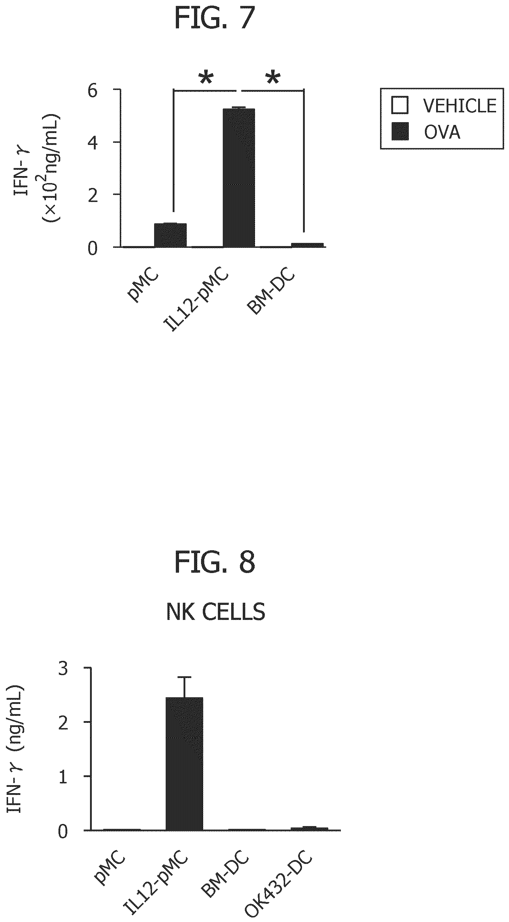

The results are shown in FIG. 7 and Table 3. In the figure, an asterisk (*) indicates a p-value obtained by one-way ANOVA with Tukey post-hoc test (*p < 0.05). The OT-I T cells co-cultured with OVA-peptide-unloaded pMC, IL12-pMC and BM-DC did not produce IFN-γ. On the other hand, the OT-I T cells co-cultured with OVA-peptide-loaded pMC, IL12-pMC and BM-DC produced IFN-γ. In particular, the OT-I T cells co-cultured with OVA-peptide-loaded IL12-pMC produced the highest amount of IFN-γ. Surprisingly, the amount of the IFN-γ produced by the OT-I T cells co-cultured with OVA-peptide-loaded IL12-pMC was more than 5-fold higher than that produced by the OT-I T cells co-cultured with OVA-peptide-loaded pMC or BM-DC.

### Table 3. Amount of IFN-γ produced by antigen-specific CD8⁺ T cells

**[Table 3]**

| | pMC | IL12-pMC | BM-DC |
|---|---|---|---|
| Vehicle | 0 ng/ml | 1.08 ng/ml | 0 ng/ml |
| OVA | 87.57 ng/ml | 524.1 ng/ml | 12.62 ng/ml |

### (3-4) Ability to induce IFN-γ production of NK cells

NK cells were isolated from splenocytes of C57BL/6N mice by using NK Cell Isolation Kit, mouse (Miltenyi Biotec). The pMC and the IL12-pMC produced in 1 above, the BM-DC produced in 2 above, and the BM-DC stimulated with the OK432 (OK432-DC) through the procedures in (3-2) above (all without OVA peptide loading) were seeded on 96-well plates (5.0×10⁴ cells/well) and were irradiated (85 Gy). The NK cells (1.0×10⁴ cells/well) were added to each of the wells of the pMC, the IL12-pMC, and the BM-DC, and were co-cultured for 3 days in RPMI 1640 containing 10% FCS and 2-mercaptoethanol (50 µM). Culture supernatants were collected, and IFN-γ was quantified using ELISA MAX^{™} Standard Set Mouse IFN-γ.

The results are shown in FIG. 8 and Table 4. Surprisingly, the amount of IFN-γ produced by the NK cells co-cultured with the IL12-pMC was more than 60-fold higher than that produced by the NK cells co-cultured with the other cells.

Table 4. Amount of IFN-γ produced by NK cells

**[Table 4]**

| pMC | IL12-pMC | BM-DC | OK432-DC |
|---|---|---|---|
| 0.0156 ng/ml | 2.4359 ng/ml | 0.0156 ng/ml | 0.03589 ng/ml |

These results demonstrate that IL12-pMC can be mass-produced, have a higher IL-12p70 production ability than BM-DC, and are superior in inducing IFN-γ production in CD8⁺ T cells and NK cells.

### 4. Comparison between IL12-pMC and GM-pMC

International Publication No. WO2021/230304, and a report by Mashima H., et al. (Oncoimmunology, 2020 Sep 6; 9 (1): 1814620. doi: 10.1080/2162402X.2020.1814620) disclose a mouse GM-CSF-producing proliferative myeloid cell (which is referred to as "ipAPC-GM" in the former, and as "GM-pMC" in the latter; and is referred to as "GM-pMC" in the Examples). The IL12-pMC and the GM-pMC were compared with each other regarding the ability to induce proliferation and IFN-γ production of T cells.

### (4-1) Proliferation-inducing activity on antigen-specific CD8⁺ T cells

GM-pMC was prepared in accordance with procedures described in the report by Mashima H. et al. (Oncoimmunology, 2020 Sep 6; 9 (1): 1814620. doi: 10.1080/2162402X.2020.1814620). The pMC and the IL12-pMC produced in 1 above, the BM-DC produced in 2 above, and the GM-pMC, were loaded with 10 µM OVA peptide. After 16 hours, the resultant cells were seeded on 96-well plates (5.0×10³ cells/well), and were irradiated (10 Gy). OT-I T cells (1.0×10⁴ cells/well) were added to each of the wells of the pMC, the IL12-pMC and the BM-DC, and were co-cultured in RPMI 1640 containing 10% FCS and 2-mercaptoethanol (50 µM). After 48 hours, 1 µCi 3H-thymidine (Perkin Elmer Inc.) was added thereto, and after 16 hours, the amount of radiation taken up by the OT-I T cells was measured with a liquid scintillation counter. The proliferation-inducing activity of each of the cells without OVA peptide loading (Medium) was also measured in the same manner (control).

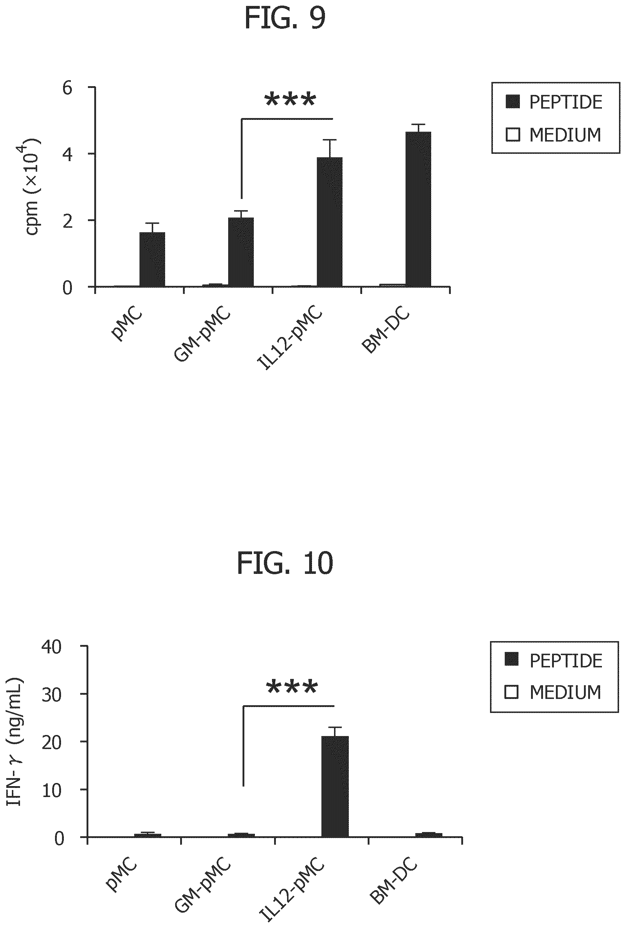

The results are shown in FIG. 9 and Table 5. In the figure, an asterisk (*) indicates a p-value obtained by one-way ANOVA with Tukey post-hoc test (***p < 0.001). None of the OVA-peptide-unloaded pMC, GM-pMC, IL12-pMC and BM-DC induced the proliferation of the OT-I T cells (white bar: Medium). All of the OVA-peptide-loaded pMC, GM-pMC, IL12-pMC and BM-DC induced the proliferation of the OT-I T cells (black bar: Peptide). Surprisingly, the proliferation-inducing activity of the IL12-pMC on the antigen-specific CD8⁺ T cells was about twice as high as that of the GM-pMC (Table 5).

### Table 5. Proliferation of antigen-specific CD8⁺ T cells

**[Table 5]**

| | pMC | GM-pMC | IL12-pMC | BM-DC |
|---|---|---|---|---|
| Peptide | 16312.3 cpm | 20745.3 cpm | 38896.3 cpm | 46486.7 cpm |

### (4-2) Ability to induce IFN-γ production of antigen-specific CD8⁺ T cells

The pMC, the GM-pMC, the IL12-pMC and the BM-DC were loaded with the OVA peptide and were co-cultured with the OT-I T cell, using the same procedures as (4-1) above. After 48 hours, culture supernatants were collected, and IFN-γ was quantified using ELISA MAX^{™} Standard Set Mouse IFN-γ. The IFN-γ production in the absence of OVA peptide loading (medium) in each cells was also determined in the same way (control).

The results are shown in FIG. 10 and Table 6. In the figure, an asterisk (*) indicates a p-value obtained by one-way ANOVA with Tukey post-hoc test (***p < 0.001). None of the OVA-peptide-unloaded pMC, GM-pMC, IL12-pMC and BM-DC induced the IFN-γ production of the OT-I T cells (white bar: Medium). All of the OVA-peptide-loaded pMC, GM-pMC, IL12-pMC and BM-DC induced the IFN-γ production of the OT-I T cells (black bar: Peptide). Surprisingly, the ability of the IL12-pMC to induce the IFN-γ production of the antigen-specific CD8⁺ T cells was about 30-fold higher than that of the GM-pMC and about 25-fold higher than that of the pMC and the BM-DC (Table 6).

### Table 6. Amount of IFN-γ produced by antigen-specific CD8⁺ T cells

**[Table 6]**

| | pMC | GM-pMC | IL12-pMC | BM-DC |
|---|---|---|---|---|
| Peptide | 0.655 ng/ml | 0.691 ng/ml | 21.113 ng/ml | 0.829 ng/ml |

These results indicate that IL12-pMC are significantly superior to GM-pMC in proliferation-inducing activity and in ability to induce IFN-γ production on antigen-specific CD8⁺ T cells.

### 5. Induction of antigen-specific cytotoxicity in vivo

### (5-1) Therapeutic cells

The pMC and IL12-pMC prepared in 1 above, and the BM-DC prepared in 2 above, were loaded with 10 µM OVA peptide and were used as therapeutic cells.

### (5-2) Target cells

Splenocytes were collected from mice C57BL/6-Tg (CAG-EGFP) C14-Y01-FM131Osb (The Jackson Laboratory Japan, Inc.) (hereinafter referred to as the "EGFP mouse"), which are MHC-matched to C57BL/6N mice and ubiquitously express EGFP, and were divided into two groups. To one group, 10 µM OVA peptide was added, and the resultant was cultured for 1 hour in a 5% CO₂ incubator at 37°C, and then washed. The other group was cultured without OVA peptide for 1 hour in a 5% CO₂ incubator at 37°C, and then washed. As the medium, RPMI 1640 containing 10% FCS and 2-mercaptoethanol (50 µM) was used. The splenocytes loaded with OVA peptide were labeled with a high concentration (0.5 µM) of eFluor 670 (Thermo Fisher Scientific) and the splenocytes not loaded with OVA peptide were labeled with a low concentration (0.005 µM) of eFluor 670. The labeling was performed in accordance with the protocol provided by the manufacturer. A 1:1 ratio mixture of these cells was used as target cells.

### (5-3) Induction of cytotoxicity and evaluation

The therapeutic cells (1×10⁵) were intraperitoneally administered to C57BL/6N mice (Charles River Laboratories Japan, Inc.) on day 0 and day 7 (treated mice). On day 14, the target cells (5×10⁶) were intravenously administered. Similarly, the target cells were also administered to mice not administered the therapeutic cells (untreated mice). After 20 hours, the mice were euthanized, the spleens were harvested, and the percentage of high eFluor 670 and low eFluor 670 cells in the target cells (EGFP-positive) present in the spleen were analyzed by flow cytometry.

Antigen-specific cytotoxicity (% specific lysis) was calculated in accordance with the following calculation formula: 100 - [100 × (% of high eFluor 670 in treated mice/% of low eFluor 670 in treated mice)/(% of high eFluor 670 in untreated mice/% of low eFluor 670 in untreated mice]

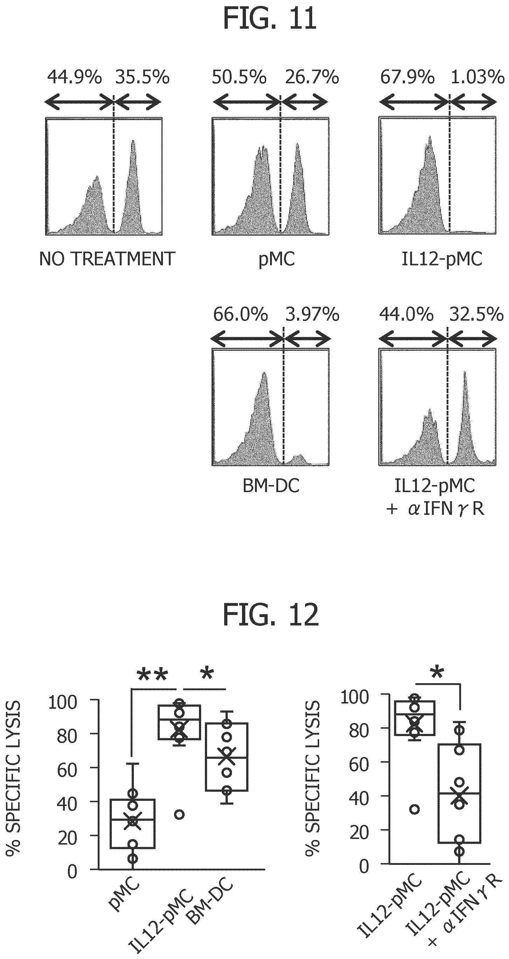

FIG. 11 illustrates representative histograms obtained through the flow cytometry analysis. In the mice administered the OVA peptide-loaded IL12-pMC, OVA peptide-loaded target cells (high eFluor 670) were eliminated with high efficiency (FIG. 11 upper right, 1.03%). The target cells were also eliminated in the mice administered the OVA peptide-loaded BM-DC, but IL12-pMC treatment was superior.

The antigen-specific cytotoxicity (% specific lysis) is shown in FIG. 12. In the figure, an asterisk (*) indicates a p-value obtained by one-way ANOVA with Tukey post-hoc test (*p < 0.05, **p < 0.01). Antigen-specific cytotoxicity was induced in the IL12-pMC-treated mice more than in the pMC- and BM-DC-treated mice (FIG. 12 left).

Here, when an anti-IFN-γ receptor antibody (αIFNγR Ab, clone name: GR20, Bio X cell) was administered to the IL12-pMC-treated mice, the ability to eliminate target cells was reduced (FIG. 11 lower right, 32.5%), and the antigen-specific cytotoxicity was attenuated (FIG. 12 right). These results suggested that IFN-γ plays an important role in the induction of *in vivo* antigen-specific cytotoxicity by IL12-pMC.

### 6. Preventive effect of IL12-pMC on cancer

### (6-1) Administration schedule

IL12-pMC or BM-DC (1×10⁵) loaded with the OVA peptide through the procedures in (5-1) above were intraperitoneally administered to C57BL/6N mice (Charles River Laboratories Japan, Inc.) on day 0 and day 7. Seven days after the last administration, 1×10⁵ mouse melanoma B16 cells expressing full-length of OVA protein (B16-OVA MO4, Merck Millipore) (hereinafter referred to as the "MO4 cells") were inoculated subcutaneously in the right flank. Tumor growth and survival were evaluated over time for each mouse (n=12). The endpoint was defined as the point when the long diameter of the tumor exceeded 15 mm.

### (6-2) Tumor volume

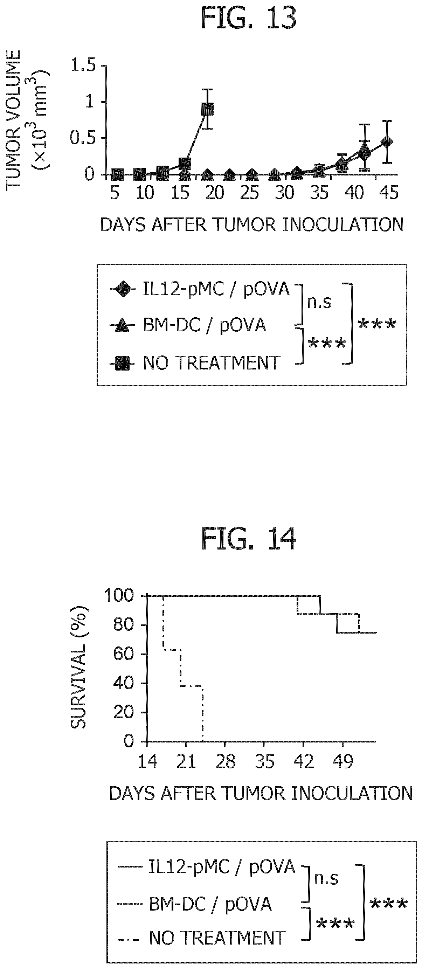

The change in tumor volume over time is shown in FIG. 13. In the figure, an asterisk (*) indicates a p-value obtained by two-tailed Student's t-test (***p < 0.001), and "n. s." means "no significant difference". The OVA peptide-loaded IL12-pMC and BM-DC suppressed cancer to the same extent.

### (6-3) Survival rate of mice

Survival rates of the mice are shown in FIG. 14. In the figure, an asterisk (*) indicates a p-value obtained by log-rank test (***p < 0.001), and "n. s." means "no significant difference". The survival time of the mouse administered the OVA peptide-loaded IL12-pMC or BM-DC was extended as compared with that of the untreated mouse.

### (6-4) Evaluation of IFN-γ production ability ex vivo

CD8⁺ T cells were collected from the spleens of mice in which cancer engraftment was completely prevented by administration of OVA peptide-loaded IL12-pMC or BM-DC (surviving 56 days after MO4 cell transplantation), and from naive mice (mice that had never been administered therapeutic or cancer cells) using a CD8α⁺ T Cell Isolation Kit, mouse (Miltenyi Biotec). RMA-S cells (Int J Cancer Suppl. 1991; 6: 38-44. doi: 10.1002/ijc.2910470711) were used as target cells (antigen-presenting cells). RMA-S cells cannot normally express MHC class I molecules on the cell surface, but when peptides are bound to MHC class I molecules and the MHC class I molecules are stabilized, the MHC class I molecule is expressed on the cell surface. RMA-S cells were loaded with the OVA peptide or SIY peptide (Eurofins Genomics K.K.) and were irradiated (45 Gy). The CD8⁺ T cells and the target cells were co-cultured for 20 hours on anti-IFN-γ antibody-immobilized membrane plates in RPMI 1640 containing 10% FCS and 2-mercaptoethanol (50 µM).

ELISpot assay was performed with BD^{™} ELISPOT Mouse IFN-γ ELISPOT Set (BD Biosciences). The number of spots was measured using ELIPHOTO Counter (Minerva Tech).

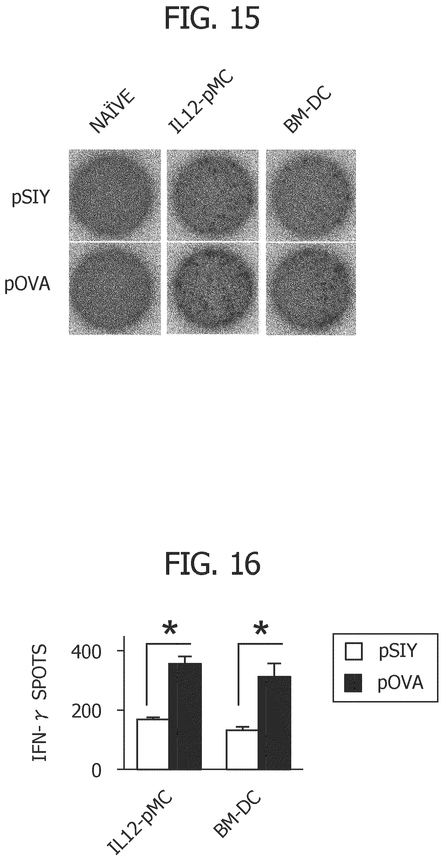

Photographs of the spots are shown in FIG. 15. In the ELISpot assay, the sites where IFN-γ production occurred were detected as spots, and a larger number of spots indicates a higher frequency of CD8⁺ T cells (OVA-specific T cells) that produced IFN-γ. No spots were observed in the co-culture of RMA-S cells and CD8⁺ T cells from naive mice, whereas many spots were observed in the co-culture of RMA-S cells and CD8⁺ T cells from mice administered OVA peptide-loaded IL12-pMC or BM-DC.

The number of spots is shown in FIG. 16. In the figure, "pOVA" means the case of using OVA peptide-loaded RMA-S cells (black bar), and "pSIY" means the case of using SIY peptide-loaded RMA-S cells (negative control) (white bar). An asterisk (*) indicates a p-value obtained by two-tailed Student's t-test (*p < 0.05). The number of IFN-γ-producing cells was increased when the CD8⁺ T cells from mice administered OVA peptide-loaded IL12-pMC were co-cultured with OVA peptide-loaded RMA-S cells compared to when co-cultured with SIY peptide-loaded RMA-S cells, similar to the CD8⁺ T cells of mice administered OVA peptide-loaded BM-DC. These results indicate that OVA-specific T cells are present at a higher frequency in the CD8⁺ T cells from mice treated with OVA peptide-loaded IL12-pMC.

These results demonstrate that IL12-pMC can induce antigen-specific T cells to the same extent as BM-DC, and can be used as a preventive cancer vaccine. It also demonstrates that, by using a desired antigen, T cells against the antigen can be induced and that preventive treatment can be performed against diseases caused by cells expressing the antigen.

### 7. Mechanism of antitumor effect of IL12-pMC

### (7-1) Administration schedule

MO4 cells (1×10⁵) were subcutaneously inoculated into the flank of C57BL/6N mice (day 0). OVA peptide-loaded IL12-pMC (1×10⁵) were intraperitoneally administered on days 4, 11, and 18. An anti-CD8α antibody (Bio X cell) or anti-NK1.1 antibody (Bio X cell) (200 µg each) was administered intraperitoneally to eliminate CD8⁺ T cells or NK cells. As a negative control, an isotype control antibody (Bio X cell) was intraperitoneally administered. Tumor growth and survival were evaluated over time for each mouse (n=12). The endpoint was defined as the point when the long diameter of the tumor exceeded 15 mm.

### (7-2) Tumor volume

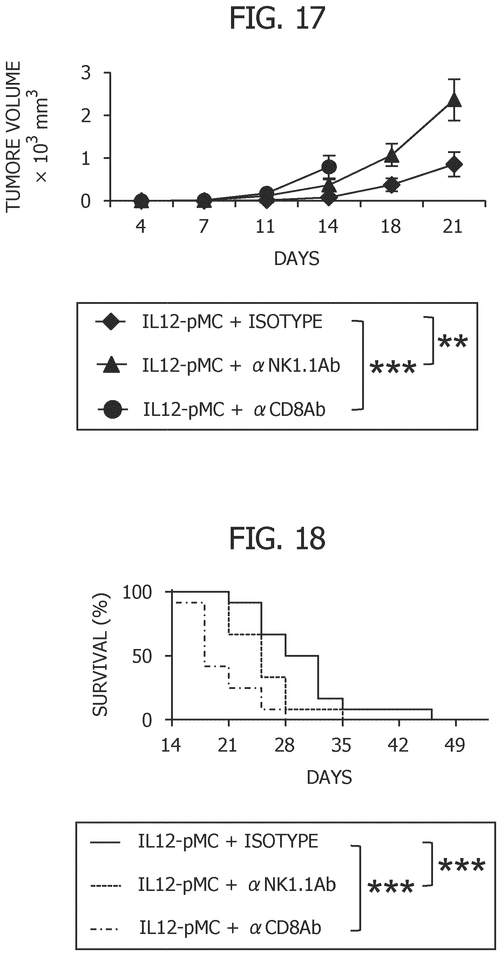

The changes in tumor volume over time are shown in FIG. 17. In the figure, an asterisk (*) indicates a p-value obtained by two-tailed Student's t-test (**p < 0.01, ***p < 0.001). The antitumor effect was reduced when the anti-CD8α antibody or the anti NK1.1 antibody was administered. In particular, when the anti-CD8α antibody was administered, the antitumor effect was much more attenuated compared to when the anti-NK1.1 antibody was administered.

### (7-3) Survival rate of mice

Survival rates of the mice are shown in FIG. 18. In the figure, an asterisk (*) indicates a p-value obtained by log-rank test (***p < 0.001). The survival time was reduced when the anti-CD8α antibody or the anti-NK1.1 antibody was administered. In particular, when the anti-CD8α antibody was administered, the survival time was remarkably shortened as compared with when the anti-NK1.1 antibody was administered.

These results demonstrate that CD8⁺ T cells play a particularly important role in the antitumor effects of IL12-pMC.

### 8. Therapeutic effect of IL12-pMC against ICI-insensitive cancer

### (8-1) Administration schedule

MO4 cells (1×10⁵) were subcutaneously inoculated into the flank of C57BL/6N mice (day 0). OVA peptide-loaded IL12-pMC (1×10⁵) were intraperitoneally administered on days 4, 11, and 18. In addition, ICIs (mixture of anti-CTLA-4 antibody (100 µg) and an anti-PD-L1 antibody (100 µg)) (hereinafter also referred to as "ICIs") were administered on days 5, 8, 12, and 15. Hereinafter, the resultant mice will be also referred to as "ICIs/OVA peptide-loaded IL12-pMC-administered mice". According to the same schedule, mice administered only ICIs (hereinafter also referred to as "ICIs-administered mice"), mice administered only OVA peptide-loaded IL12-pMC (hereinafter also referred to as "OVA peptide-loaded IL12-pMC-administered mice"), and mice administered nothing (hereinafter also referred to as the "untreated mice") were generated. Tumor growth and survival were evaluated over time for each mouse (n=12). The endpoint was defined as the point when the long diameter of the tumor exceeded 15 mm.

### (8-2) Tumor volume

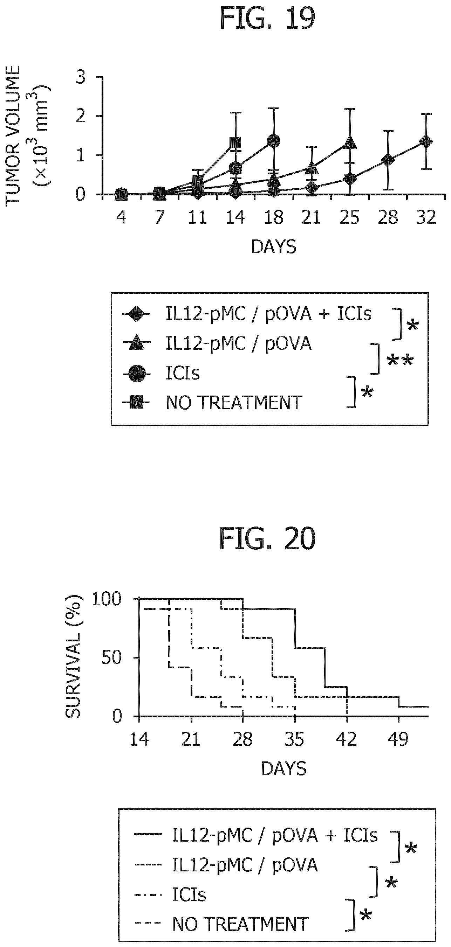

The changes in tumor volume over time are shown in FIG. 19. In the figure, an asterisk (*) indicates a p-value obtained by two-tailed Student's t-test (*p < 0.05, **p < 0.01). The tumor volume was suppressed in the ICIs-administered mice, the OVA peptide-loaded IL12-pMC-administered mice, and the ICIs/OVA peptide-loaded IL12-pMC-administered mice. Because some of the ICIs-administered mice died on day 18, a comparison was made on day 18, and tumor volume was suppressed more in the OVA peptide-loaded IL12-pMC-administered mice than in the ICIs-administered mice. Also, because some of the OVA peptide-loaded IL12-pMC-administered mice died on day 25, a comparison was made on day 25, and tumor volume was suppressed more in the ICIs/OVA peptide-loaded IL12-pMC-administered mice than in the OVA peptide-loaded IL12-pMC-administered mice.

According to WO2021/230304 and a report by Mashima H. et al. (Oncoimmunology, 2020 Sep 6; 9 (1): 1814620 doi: 10.1080/2162402X.2020.1814620), GM-pMC suppressed the tumor growth more than the ICIs, but the combination of GM-pMC and ICIs did not show better antitumor effects than GM-pMC alone. On the contrary, surprisingly, the IL12-pMC exhibited stronger antitumor effects when combined with ICIs.

### (8-3) Survival rate of mice

Survival rates of the mice are shown in FIG. 20. In the figure, an asterisk (*) indicates a p-value obtained by log-rank test (***p < 0.001). The survival time of the ICIs-administered mice, the OVA peptide-loaded IL12-pMC-administered mice, and the ICIs/OVA peptide-loaded IL12-pMC-administered mice was extended. The survival time of the OVA peptide-loaded IL12-pMC-administered mice was longer than that of the ICIs-administered mice. Surprisingly, the survival time of the ICIs/OVA peptide-loaded IL12-pMC-administered mice was even longer than that of the OVA peptide-loaded IL12-pMC-administered mice.

According to WO2021/230304 and a report by Mashima H. et al. (Oncoimmunology, 2020 Sep 6; 9 (1): 1814620 doi: 10.1080/2162402X.2020.1814620), GM-pMC extended survival time more than ICIs, but the combination of GM-pMC and ICIs did not extend survival time compared to administration of GM-pMC alone.

From the above results, it was found that antigen peptide-loaded IL12-pMC are more effective than ICIs against ICIs-insensitive cancer. It was also found that the combination use of antigen peptide-loaded IL12-pMC and ICIs exhibits a synergistic therapeutic effect against ICIs-insensitive cancer.

### 9. Therapeutic effect of IL12-pMC against ICI-sensitive cancer

### (9-1) Administration Schedule

Using BALB/c mouse iPS cells instead of C57BL/6N mouse iPS cells, IL12-pMC was prepared by the same procedures in 1 above and were loaded with AH1 peptide (Eurofins Genomics K.K.) instead of the OVA peptide. Using BALB/c mice instead of C57BL/6N mice, and using CT26 cells (ATCC) instead of the MO4 cells, and using AH1 peptide-loaded IL12-pMC instead of the OVA peptide-loaded IL12-pMC, tumor growth and survival were evaluated over time by the same procedures as in 8 above. The endpoint was defined as the point when the long diameter of the tumor exceeded 15 mm. The CT26 cell is a colorectal cancer cell line derived from a BALB/c mouse. The AH1 peptide is a CTL epitope derived from mouse leukemia virus gp70 (PNAS, 93: 9730-9735, 1996), and is widely used in a tumor immune model using CT26 cells.

### (9-2) Tumor volume

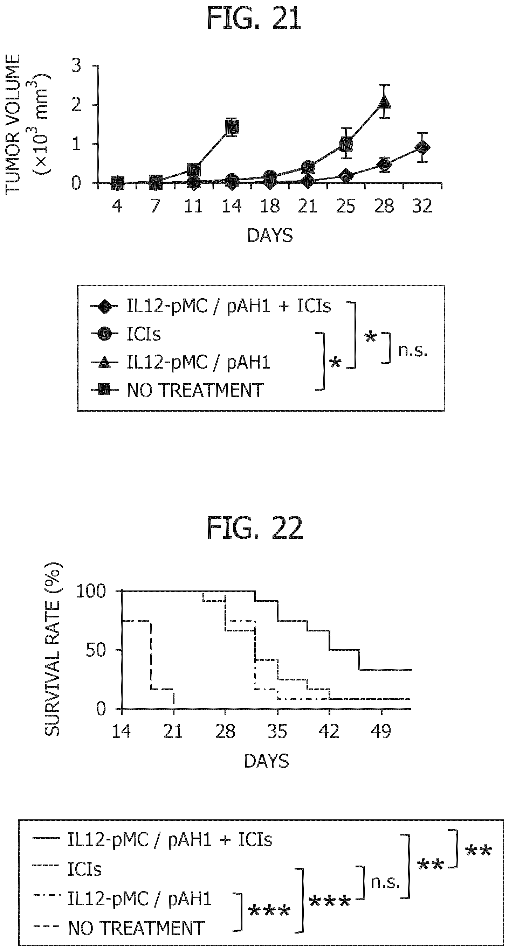

The change in tumor volume over time is shown in FIG. 21. In the figure, an asterisk (*) indicates a p-value obtained by two-tailed Student's t-test (*p < 0.05), and "n. s." means "no significant difference". The tumor volume was suppressed in the ICIs-administered mice, the AH1 peptide-loaded IL12-pMC-administered mice, and the ICIs/AH1 peptide-loaded IL12-pMC-administered mice. The effect of suppressing tumor volume by the administration of ICIs was equivalent to that obtained by the administration of the AH1 peptide-loaded IL12-pMC. The combined administration of ICIs/AH1 peptide-loaded IL12-pMC further suppressed tumor volume than either ICIs or AH1 peptide-loaded IL12-pMC alone.

### (9-3) Survival rate of mice

Survival rates of the mice are shown in FIG. 22. In the figure, an asterisk (*) indicates a p-value obtained by log-rank test (**p < 0.01, ***p < 0.001), and "n. s." means "no significant difference". The survival time of the ICIs-administered mice, the AH1 peptide-loaded IL12-pMC-administered mice, and the ICIs/AH1 peptide-loaded IL12-pMC-administered mice was extended. The survival time of the ICIs-administered mice and the AH1 peptide-loaded IL12-pMC-administered mice was equivalently extended. The survival time of the ICIs/AH1 peptide-loaded IL12-pMC-administered mice was further extended than that of the ICIs-administered mice or the AH1 peptide-loaded IL12-pMC-administered mice.

From the above results, it was found that antigen peptide-loaded IL12-pMC has a therapeutic effect equivalent to that of ICIs against ICIs-sensitive cancer. It was also found that the combination use of antigen peptide-loaded IL12-pMC and ICIs exhibits a synergistic therapeutic effect against ICIs-sensitive cancer.

### (9-4) Evaluation of IFN-γ production ability ex vivo

ELISpot assay was performed for evaluating the ability to produce IFN-γ on mice administered the AH1 peptide-loaded IL12-pMC/ICIs and survived for 56 days after the inoculation of the CT26 cell and naive mice by the same procedures as in (6-4) above, except that the AH1 peptide was used instead of the OVA peptide, that β-gal peptide (Eurofins Genomics) was used instead of the SIY peptide, and that splenic antigen-presenting cells were used instead of the RMA-S cells. The β-gal peptide is a peptide derived from β-galactosidase, and is widely used as a negative control peptide in a tumor immune mode using BALB/c mice.

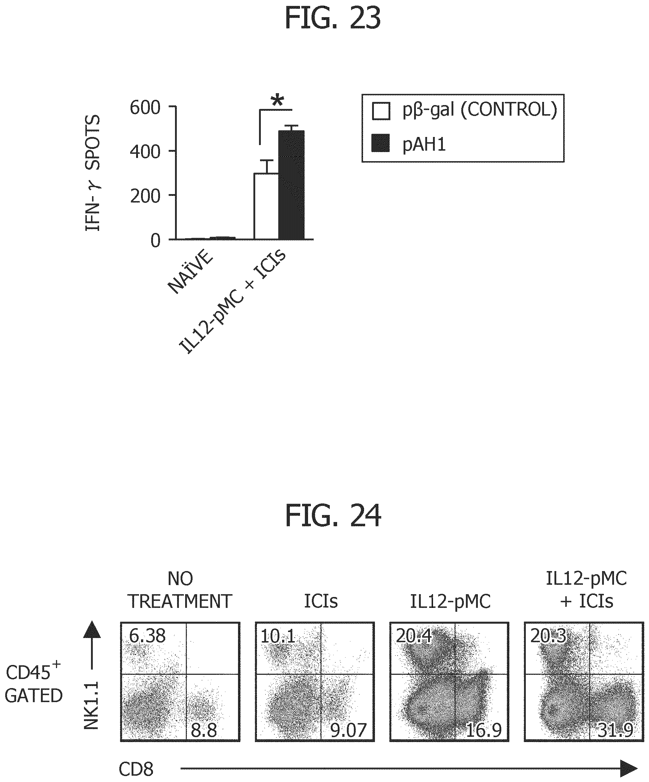

The results are shown in FIG. 23. In the figure, "pAH1" means the case of using splenic antigen-presenting cells loaded with the AH1 peptide (black bar), and "pβ-gal" means the case of using splenic antigen-presenting cells loaded with the β-gal peptide (negative control) (white bar). An asterisk (*) indicates a p-value obtained by two-tailed Student's t-test (*p < 0.05). When CD8⁺ T cells from the AH1 peptide-loaded IL12-pMC/ICIs-administered mice were co-cultured with the AH1 peptide-loaded splenic antigen-presenting cells, the number of IFN-y-producing cells was increased compared to when co-cultured with the β-gal peptide-loaded splenic antigen-presenting cells. These results indicate that AH1-specific T cells present at a higher frequency in the CD8⁺ T cells of the AH1 peptide-loaded IL12-pMC/ICIs-administered mice.

These results demonstrate that IL12-pMC can induce CD8⁺ T cells in an antigen-specific manner.

### 10. Changes in immune cells in cancer tissue

### (10-1) Administration schedule

ICIs/OVA peptide-loaded IL12-pMC-administered mice, ICIs-administered mice, OVA peptide-loaded IL12-pMC-administered mice, and untreated mice (n=3 each) were prepared by the same procedures as in (8-1) above and using MO4 cells (ICIs insensitive). Cancer tissues were harvested on day 17.

### (10-2) Flow cytometry

Mice were injected intraperitoneally with 250 µg of Brefeldin A (AdipoGen) (1 mg/mL) 4 hours before harvesting tumor tissues. Single-cell suspensions were obtained from tumor tissue by mechanical disruption and enzymatic degradation of the extracellular matrix. The isolated tumor tissue was minced and suspended in RPMI-1640 medium containing 1.0 mg/mL collagenase A (Roche Diagnostics), 0.2 mg/mL hyaluronidase (Nacalai Tesque), and 20 mg/mL DNase I (AppliChem). The tissue suspension was digested in a water bath at 37°C for 30 minutes, and then the resultant was filtered through a 70 µm filter to obtain a single-cell suspension. The single-cell suspension was incubated with a Fc receptor blocking reagent (Miltenyi Biotec) and was stained with an immunofluorescence-labeled antibody according to the manufacturer's instructions.

### (10-3) Percentages of CD8⁺ T cells and NK cells

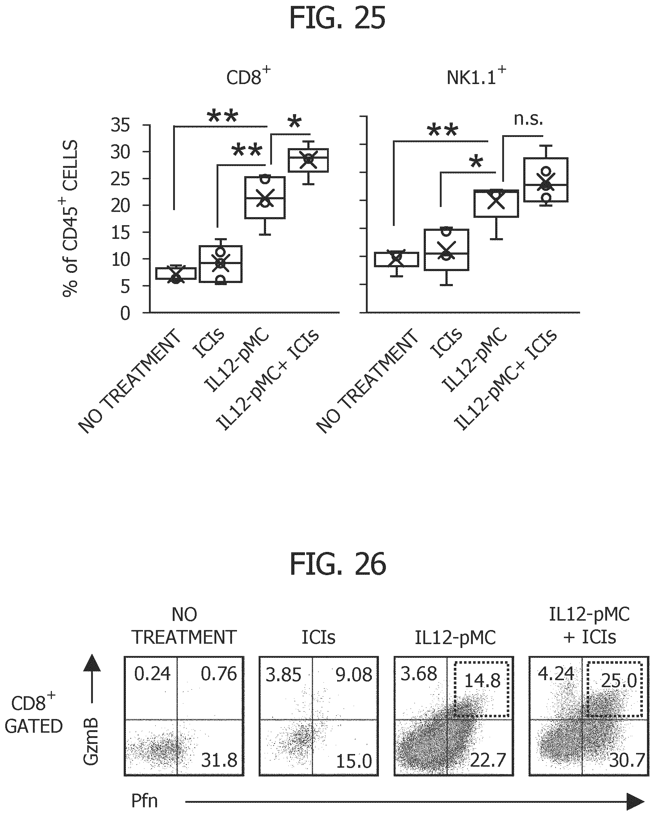

The results of the analysis of the frequencies of CD8⁺ T cells (CD8 positive) and NK cells (NK1.1 positive) in the cancer tissue are shown in FIG. 24. The numbers in each gate indicate the percentage of each measured cell among CD45-positive cells. The percentages of CD8+ T cells and NK cells calculated from flow cytometry results are shown in FIG. 25. In the figure, an asterisk (*) indicates a p-value obtained by two-tailed Student's t-test (*p < 0.05, **p < 0.01). In comparison between the untreated mice and the ICIs-administered mice, the frequencies of CD8⁺ T cells and NK cells in the cancer tissue were not significantly different. In the OVA peptide-loaded IL12-pMC-administered mice, the frequencies of CD8⁺ T cells and NK cells in the cancer tissue were higher than in the ICIs-administered mice. In the ICIs/OVA peptide-loaded IL 12-pMC-administered mice, the frequency of CD8⁺ T cell in the cancer tissue was higher than in the OVA peptide-loaded IL12-pMC-administered mice, but there was no significant difference in the frequency of NK cells.

### (10-4) Percentages of Prf1⁺ GzmB⁺ cells in CD8⁺ T cells in cancer tissue

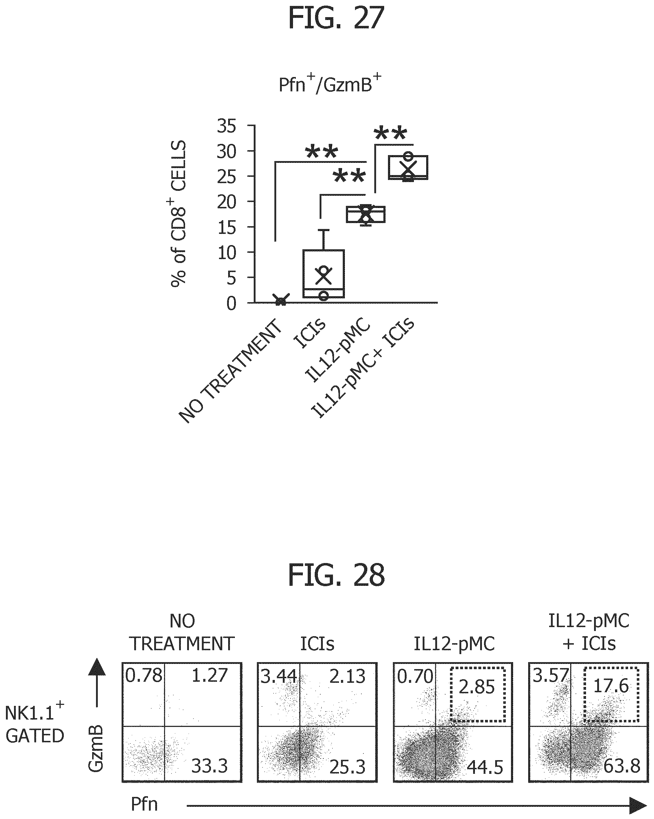

The results of the analysis of expression of intracellular antigens perforin (Pfn) and granzyme B (GzmB) in CD8⁺ T cells in the cancer tissue are shown in FIG. 26. The numbers in each gate indicate the percentage of each measured cell among CD8-positive cells. The percentages of perforin-positive and granzyme B-positive (Prf1⁺GzmB⁺) cells calculated from flow cytometry results are shown in FIG. 27. In the figure, an asterisk (*) indicates a p-value obtained by two-tailed Student's t-test (**p < 0.01). In comparison between the untreated mice and the ICIs-administered mice, there was no significant difference in the frequency of Prf1⁺GzmB⁺ cells in CD8⁺ T cells in the cancer tissue. In the OVA peptide-loaded IL12-pMC-administered mice, the frequency of Prf1⁺GzmB⁺ cells in CD8⁺ T cells in the cancer tissue was higher than in the ICIs-administered mice. In the ICIs/OVA peptide-loaded IL12-pMC-administered mice, the frequency of Prf1⁺GzmB⁺ cells in CD8⁺ T cells in the cancer tissue was higher than in the OVA peptide-loaded IL12-pMC-administered mice.

### (10-4) Percentages of Prf1⁺GzmB⁺ cells in NK cells in cancer tissue

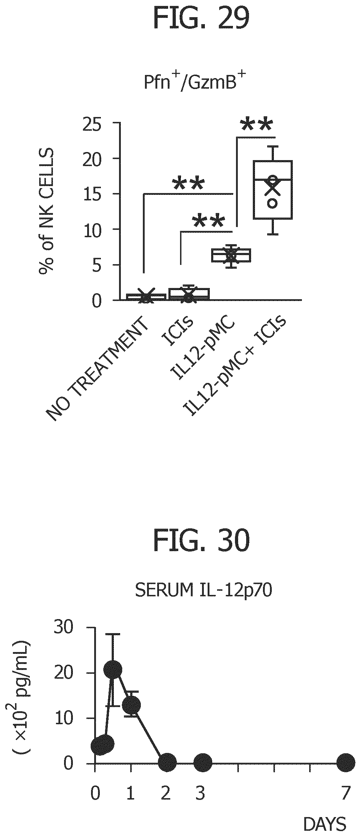

The results of the analysis of expression of intracellular antigens perforin (Pfn) and granzyme B (GzmB) in NK cells (NK1.1⁺ cells) in the cancer tissue are shown in FIG. 28. The numbers in each gate indicate the percentage of each measured cell among NK cells. The percentages of Prf1⁺GzmB⁺ cells calculated from flow cytometry results are shown in FIG. 29. In the figure, an asterisk (*) indicates a p-value obtained by two-tailed Student's t-test (**p < 0.01). In comparison between the untreated mice and the ICIs-administered mice, there was no significant difference in the frequency of Prf1⁺GzmB⁺ cells in NK cells in the cancer tissue. In the OVA peptide-loaded IL12-pMC-administered mice, the frequency of Prf1⁺GzmB⁺ cells in NK cells in the cancer tissue was higher than in the ICIs-administered mouse. In the ICIs/OVA peptide-loaded IL12-pMC-administered mice, the frequency of Prf1⁺GzmB⁺ cells in NK cells in the cancer tissue was higher than in the OVA peptide-loaded IL12-pMC-administered mice.

These results demonstrate that OVA peptide-loaded IL12-pMC induces CD8⁺ T cells and NK cells with high cell-killing ability in cancer tissues. Also, it was found that this effect is promoted by combined use with ICIs.

### 11. Role of IFN-γ in clinical efficacy

### (11-1) Measurement of blood IL-12p70 and IFN-γ concentrations

IL12-pMC (1×10⁵) prepared in 1 above were loaded with the OVA peptide and were intraperitoneally administered to C57BL/6 N mice. Blood samples were collected 3, 6, 12, 24, 48, 72 hours and 7 days after administration, and IL-12p70 and IFN-γ in the blood were quantified by ELISA.

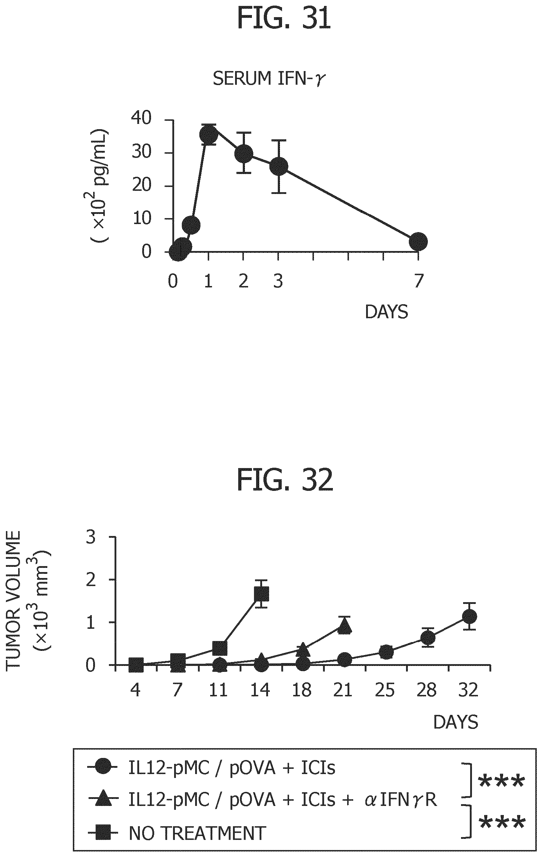

The results are shown in FIGS. 30 and 31. Blood IL-12p70 peaked 12 hours after IL12-pMC administration and was below the detection limit 48 hours later (FIG. 30). On the other hand, blood IFN-γ peaked 24 hours after IL12-pMC administration and was still detected at 2×10³ pg/mL or more at 48 and 72 hours, but was not detected 7 days later (FIG. 31). These results demonstrated that blood IFN-γ increased by IL12-pMC administration, and was maintained for a long period of time.

### (11-2) Administration schedule

ICIs/OVA peptide-loaded IL12-pMC-administered mice and untreated mice were prepared by the same procedures as in (8-1) above and using MO4 cells (ICIs insensitive). In addition, mice were generated by further administering anti-IFN-y receptor antibody (αIFNγR Ab, clone name; GR20, Bio X cell) (200 µg) on 4, 5, 11, 12, 18, and 19 days after the inoculation of MO4 cells to the ICIs/OVA peptide-loaded IL12-pMC-administered mice (hereinafter also referred to as "ICIs/OVA peptide-loaded IL12-pMC/anti-IFNγR antibody mice"). In each mouse (n = 12), tumor growth and survival were evaluated over time. The endpoint was defined as the point when the long diameter of the tumor exceeded 15 mm.

### (11-3) Tumor volume

The changes in tumor volume over time are shown in FIG. 32. In the figure, an asterisk (*) indicates a p-value obtained by two-tailed Student's t-test (***p < 0.001). In the ICIs/OVA peptide-loaded IL12-pMC-administered mice and the ICIs/OVA peptide-loaded IL12-pMC administration/anti-IFNyR antibody mice, the tumor volume was suppressed, but the antitumor effect in the ICIs/OVA peptide-loaded IL12-pMC administration/anti-IFNyR antibody mice was lower than in the ICIs/OVA peptide-loaded II,12-pMC-administered mice.

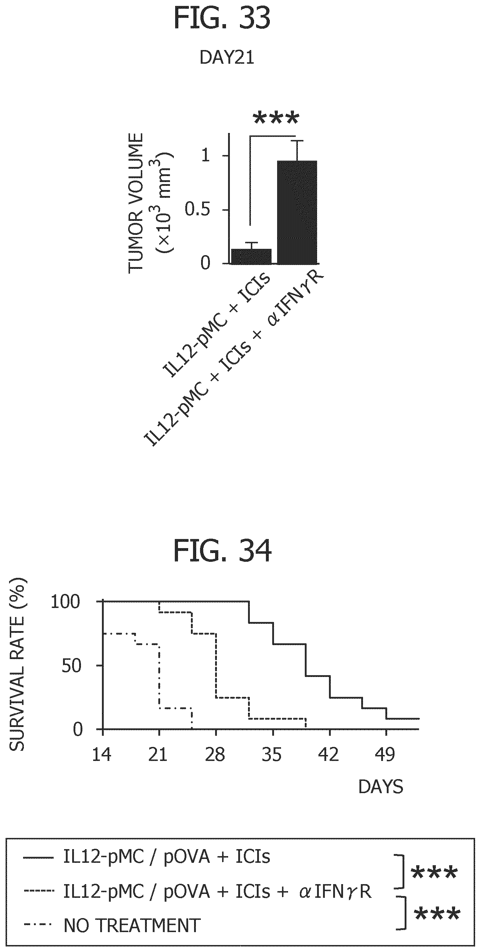

FIG. 33 shows mean tumor volumes in the ICIs/OVA peptide-loaded IL12-pMC-administered mice and the ICIs/OVA peptide-loaded IL12-pMC administration/anti-IFNyR antibody mice on 21 days after the inoculation of MO4 cells. In the figure, an asterisk (*) indicates a p-value obtained by two-tailed Student's t-test (***p < 0.001). The tumor volume was increased by the administration of anti-IFNyR antibody, indicating that IFN-γ plays an important role in the antitumor effect in the ICIs/OVA peptide-loaded IL12-pMC-administered mice.

### (11-4) Survival rate of mice

The survival rates of the mice are illustrated in FIG. 34. In the figure, an asterisk (*) indicates a p-value obtained by log-rank test (***p < 0.001). The survival time of the ICIs/OVA peptide-loaded IL12-pMC-administered mice and the ICIs/OVA peptide-loaded IL12-pMC administration/anti-IFNyR antibody mice was increased, but the survival time of the ICIs/OVA peptide-loaded IL12-pMC administration/anti-IFNyR antibody mice was shorter than that of the ICIs/OVA peptide-loaded IL12-pMC-administered mice.

These results demonstrate that IFN-γ plays an important role in the antitumor effect of combined use of antigen peptide-loaded IL12-pMC and ICIs against ICIs-insensitive cancers.

### 12. Production of human IL-12p70-producing proliferative myeloid cells

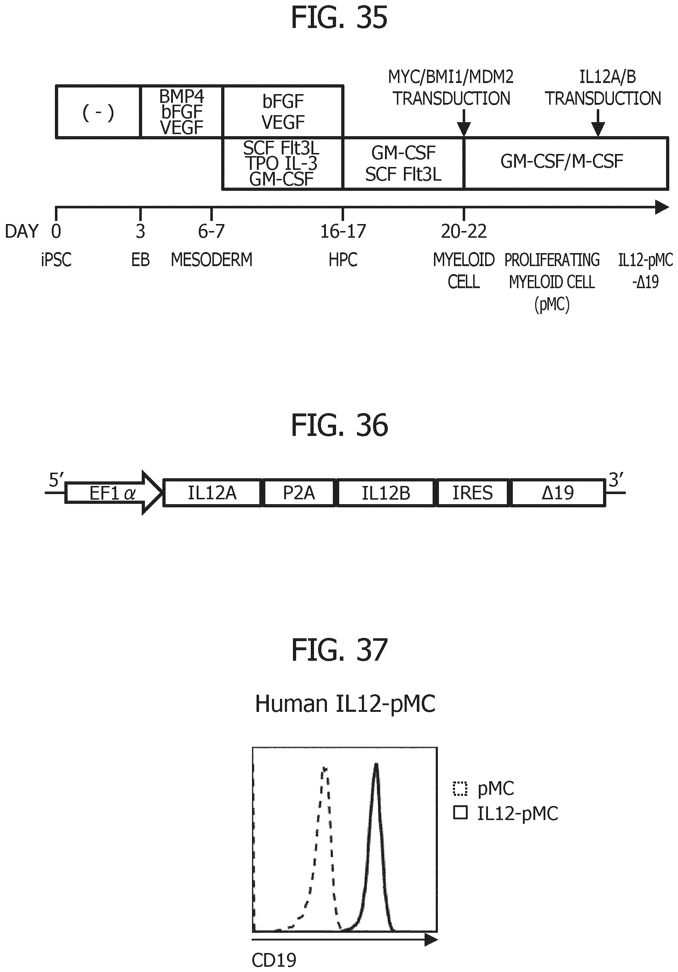

Human IL-12p70-producing proliferative myeloid cells were produced by the following procedures. The production schedule is illustrated in FIG. 35.

### (12-1) Production of human proliferative myeloid cell

Human iPS cells were produced by the method described by Kitayama S. et al (Stem Cell Reports, 2016 Feb 9; 6 (2): 213-27. doi: 10.1016/j.stemcr.2016.01.005). Human proliferative myeloid cells were produced through the following procedures. The human iPS cells were cultured in 6-well plates coated with iMatrix-511 (Laminin-511 E8) (Nippi) in StemFit (Ajinomoto) medium for human iPS cells. The human iPS cells were harvested by treatment with TrypLE Select (Thermo Fisher Scientific) at 37°C for 4 minutes and were seeded onto Costar^{™} ultra-low attachment 6-well plates (Corning) with StemFit containing a final concentration of 10 µM Y-27632 (FUJIFILM Wako Pure Chemical Corporation). On the next day, Y-27632 was removed by changing the medium. After forming embryoid bodies (EBs) by suspension culture for 3 days, subsequent differentiation induction was performed by using serum-free medium X-VIVO15 (Lonza) supplemented with 2 mM L-glutamine (Gibco), 1 mM sodium pyruvate (Gibco), MEM non-essential amino acids solution (1×) (Gibco), 450 µM monothioglycerol (Nacalai Tesque) and 50 µg/mL L-ascorbic acid 2-phosphate (Sigma Aldrich), as the differentiation medium. Differentiation into mesoderm cells was induced by culturing the cells in a differentiation medium containing 50 ng/mL human recombinant BMP-4 (Miltenyi Biotec), 50 ng/mL human recombinant VEGF (FUJIFILM Wako Pure Chemical Corporation), and 50 ng/mL human recombinant bFGF (FUJIFILM Wako Pure Chemical Corporation) for 3 to 4 days. Thereafter, the cells were cultured for 10 days in a differentiation medium supplemented with 50 ng/mL human recombinant vascular endothelial growth factor (VEGF) (FUJIFILM Wako Pure Chemical Corporation), 50 ng/mL human recombinant basic fibroblast growth factor (bFGF) (FUJIFILM Wako Pure Chemical Corporation), 50 ng/mL human recombinant stem cell factor (SCF) (FUJIFILM Wako Pure Chemical Corporation), 50 ng/mL human recombinant Flt3 ligand (Flt3L) (FUJIFILM Wako Pure Chemical Corporation), 10 ng/mL human recombinant thrombopoietin (TPO) (FUJIFILM Wako Pure Chemical Corporation), 20 ng/mL human recombinant IL-3 (FUJIFILM Wako Pure Chemical Corporation), and 50 ng/mL human recombinant GM-CSF (FUJIFILM Wako Pure Chemical Corporation) to induce differentiation into hematopoietic cells. Thereafter, the cells were cultured for 4 to 5 days in a differentiation medium supplemented with 50 ng/mL human recombinant SCF, 50 ng/mL human recombinant Flt3L, and 50 ng/mL human recombinant GM-CSF to induce differentiation into myeloid-type cells (hereinafter, also referred to as "human MC").

A lentiviral vector including human MYC, BMI1 and MDM2 genes was prepared through the following procedures. The cDNA sequences of the human MYC, BMI1 and MDM2 (Ref Seq IDs: NM_001354870.1, NM_005180.9, and NM_002392; SEQ ID NOs: 4 to 6) were inserted into the multicloning site (XhoI/NotI) of pSL (SEQ ID NO: 7) to obtain pSL-MYC, pSL-BMI1, and pSL-MEM2. The pSL was prepared by inserting a partial sequence of CSII-EF-MCS (RIKEN BRC DNA BANK) containing the EF1α promoter into the EcoRI-StuI site of pUC57 (GenScript).

The pSL-MYC, pSL-BMI1, or pSL-MDM2 was introduced into 293T cells together with pCAG-HIVgp and pCMV-VSV-G-RSV-Rev by lipofection. The culture supernatant was collected three days after the lipofection and was filtered through a filter with a pore size of 0.45 µm, and viral particles were concentrated by Lenti-X concentrator. The obtained viral particles were suspended in DMEM, aliquoted into cryovials, and stored at -150°C.

The human MC produced as described above was infected simultaneously with lentiviruses including the human MYC, MBI1, and DMD2 genes. For infection, serum-free medium ALyS705 (Cell Science & Technology Institute, Inc.) supplemented with 50 ng/mL human recombinant GM-CSF, 50 ng/mL human recombinant M-CSF (FUJIFILM Wako Pure Chemical Corporation), and 5% Artificial Serum (Cell Science & Technology Institute, Inc.) was used. Seven to 14 days after the infection, myeloid-type cells proliferating in a GM-CSF/M-CSF-dependent manner appeared, which were named "human proliferative myeloid cells (human pMC)". The human pMC was cultured in αMEM containing 20% FCS, 50 ng/mL human GM-CSF recombinant, and 50 ng/mL human M-CSF recombinant (hereinafter, the medium is referred to as the "hpMC medium").

### (12-2) Production of lentiviral vector expressing human IL-12p70

A cDNA, a fused cDNA including human IL12A and IL12B cDNAs (Ref Seq IDs: NM_000882.4 and NM_002187.3; SEQ ID NOs: 8 and 9) linked via a nucleic acid sequence (SEQ ID NO: 10) encoding the self-cleaving peptide P2A derived from Porcine teschovirus-1 (PTV-1), further comprising an IRES sequence (SEQ ID NO: 11) and a nucleic acid sequence encoding a human CD19 fragment (amino acids 1-333) (SEQ ID NO: 12) attached to the 3' end of the fusion cDNA, was synthesized by entrusting GenScript. This cDNA (IL12A-P2AIL12-B-IRES-Δ19) was inserted into the multicloning site (Xhol/BamHI) of pSL plasmid to obtain pSL-IL12Δ19 (FIG. 36).

The pSL-IL12Δ19 was introduced by lipofection into 293T cells together with pCAG-HIVgp and pCMV-VSV-G-RSV-Rev. Three days after the lipofection, the culture supernatant was collected and filtered through a filter with a pore size of 0.45 µm, and then the viral particles were concentrated using a Lenti-X concentrator (Clontech). The obtained viral particles were suspended in DMEM, dispensed into cryovials, and stored at -150°C.

### (12-3) Production of human IL-12-producing proliferative myeloid cells

The human pMC produced in (12-1) above were seeded on 48-well plates, and were infected with the lentiviral vector produced in (12-2) above. For growth culture after the infection, the hpMC medium was used. Eight days after the infection, the resultant cells were stained with a fluorescently labeled anti-CD19 antibody (BioLegend, clone 4G7), and the cells were isolated based on the expression of Δ19. The isolated Δ19-expressing cells (CD19-positive cells) continued to proliferate in hpMC medium for more than three months. The cells were named "IL-12p70 producing human proliferative myeloid cell (human IL12-pMC)".

The results of evaluating human pMC and human IL12-pMC by flow cytometry are shown in FIG. 37. Human pMC, into which IL12A-P2A-IL12B-IRES-Δ19 had not been introduced, were CD19-negative, whereas human IL12-pMC were CD19-positive. These results confirm that human IL12-pMC expresses the IL12A and IL12B genes.

### 13. Surface marker analysis of human IL-12p70-producing proliferative myeloid cells

Expression of surface markers in the human pMC and human IL12-pMC produced in 12 above was analyzed by immunostaining and flow cytometry. For the immunostaining, antibodies shown in Table 7 below were used.

### Table 7. Antibodies used for marker staining

**[Table 7]**

| Antigen | Species | Clone | Source |
|---|---|---|---|
| HLA Class-I (HLA-A,B,C) | Human | W6/32 | BioLegend |
| HLA Class-II (HLA-DR) | Human | L243 | BioLegend |
| CD40 | Human | HB14 | BioLegend |
| CD80 | Human | 2D10 | BioLegend |
| CD83 | Human | HB15e | BioLegend |
| CD86 | Human | IT2.2 | BioLegend |

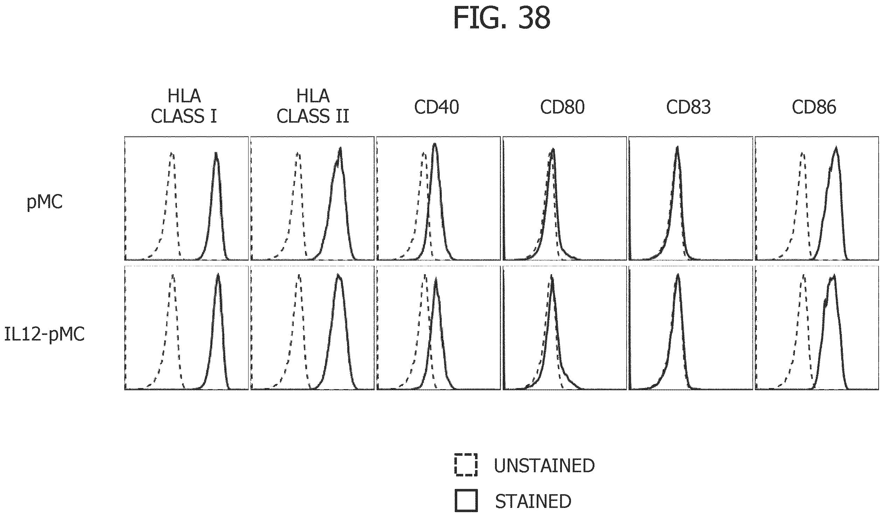

The results are shown in FIG. 38. The histograms of unstained cells are shown in dotted line, and the histograms of stained cells are shown in solid line. Similar to the human pMC, the human IL12-pMC expressed HLA class I molecules, HLA class II molecules, and CD40 and CD86, which are important molecules for T cell stimulation. This suggests that human pMC and human IL12-pMC have T cell-stimulating activity.

### 14. Characteristic analysis of human IL-12p70-producing proliferative myeloid cells (14-1) Proliferation ability

The human pMC and the human IL12-pMC produced in 12 above were seeded on 96-well plates (3.0×10³ cells/well) and cultured in 20% FCS-containing αMEM supplemented with human GM-CSF recombinant (50 ng/mL), human M-CSF recombinant (50 ng/mL), or a combination thereof. Proliferation in each culture condition was evaluated over time by MTT assay by the same procedures as in (3-1) above.

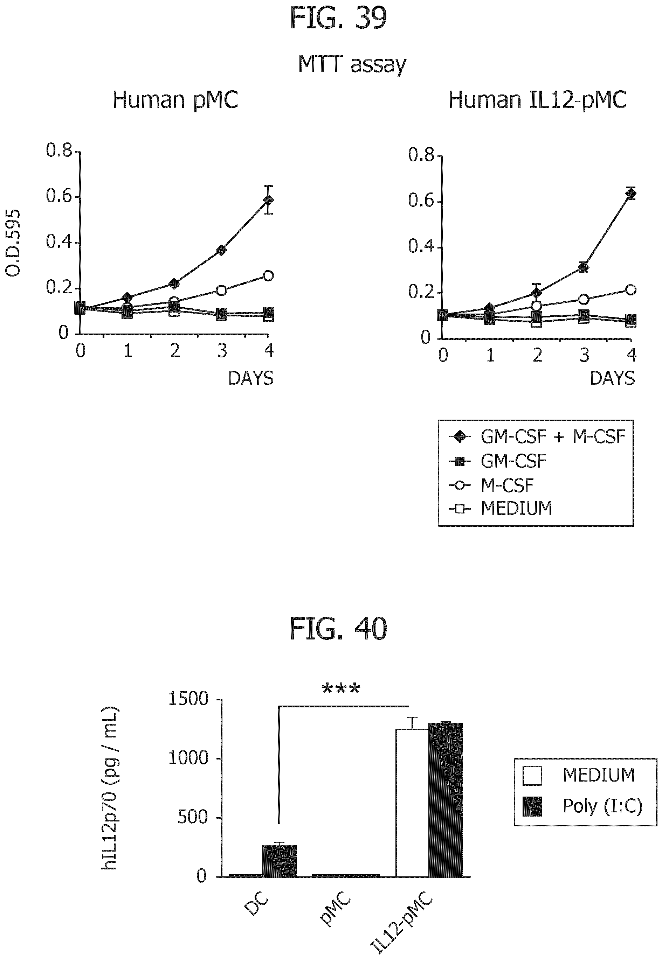

The results are shown in FIG. 39. The proliferation of the human pMC and the human IL12-pMC was M-CSF-dependent, and was further enhanced by adding GM-CSF and M-CSF to the medium in combination. This proliferation continued for 3 months or more. These results indicate that human pMC and human IL12-pMC are unique cells in which proliferation can be regulated by GM-CSF and M-CSF, and can be mass-produced by GM-CSF and M-CSF.

### (14-2) Cytokine production

The human pMC and the human IL12-pMC produced in 12 above, and human DC (see below) were seeded on 96-well plates (1.0×10⁴ cells/well) with 10% FCS-containing RPMI 1640. To the cells, 20 µg/mL poly (I:C) (InvivoGen) was added and cultured for 24 hours. The culture supernatants were collected, and IL-12p70 was quantified using ELISA MAX^{™} Standard Set Human IL-12 (p70) (BioLegend), and IL-10 was quantified using ELISA MAX^{™} Standard Set Human IL-10 (BioLegend). The amounts of IL-12p70 and IL-10 produced without addition of poly (I:C) (medium only) were also measured in the same manner (control).

The human DC was prepared through the following procedures. CD14-positive monocytes were isolated from human peripheral blood mononuclear cells using CD14 MicroBeads (Miltenyi Biotec). The CD14-positive monocytes were induced into dendritic cells by culturing them for 6 days in RPMI 1640 containing 20% FCS supplemented with human IL-4 recombinant (BioLegend) (50 ng/mL) and human GM-CSF recombinant (50 ng/mL). The thus obtained cells were used as "human DC".

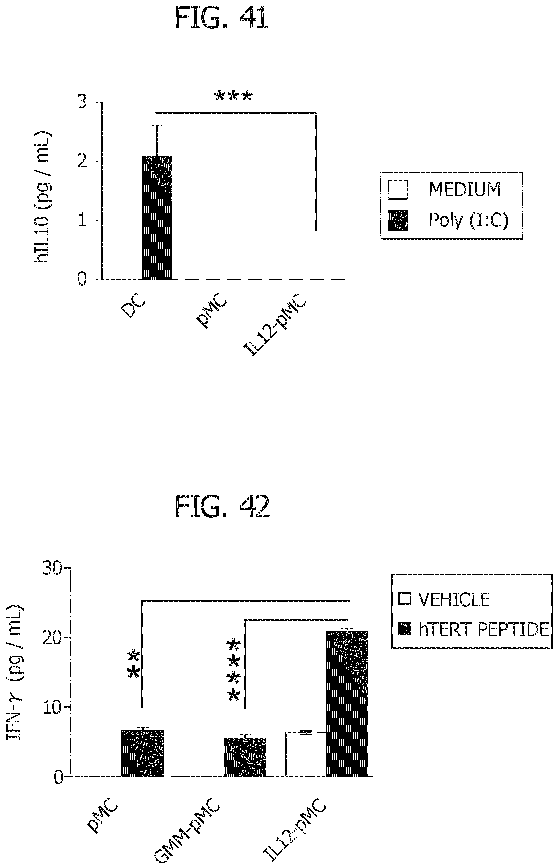

The results for the IL-12p70s are shown in FIG. 40 and Table 8, and the results for the IL-10s are shown in FIG. 41 and Table 9. In the figures, human DC, human pMC, and human IL12-pMC are simply referred to as "DC", "pMC", and "IL12-pMC" respectively. An asterisk (*) indicates a p-value obtained by one-way ANOVA with Tukey post-hoc test (***p < 0.001).

The human IL12-pMC produced a large amount of IL-12p70 even without poly (I:C) stimulation (white bar), and the production amount was not increased with the poly (I:C) stimulation (black bar). Surprisingly, the amount of IL-12p70 produced by human IL12-pMC was 4.8-fold higher than that produced by poly(I:C)-stimulated human DC and 86-fold higher than that produced by human pMC.

### Table 8. IL-12p70 production

**[Table 8]**

| | Human DC | Human pMC | Human IL12-pMC |
|---|---|---|---|
| Medium | 15 pg/ml | 15 pg/ml | 1247.67 pg/ml |
| poly(I:C) | 267.35 pg/ml | 15 pg/ml | 1293.49 pg/ml |

The human DC produced IL-10 with the poly (I:C) stimulation. On the other hand, the human IL12-pMC did not produce IL-10, regardless of the presence or absence of poly(I:C) stimulation. The human IL12-pMC did not produce the immunosuppressive cytokine IL-10, suggesting that human IL12-pMC have less immunosuppressive activity than human DC. Accordingly, it was suggested that human IL12-pMC are superior to poly (I:C)-stimulated human DC in inducing cellular immune responses.

### Table 9. Amount of IL-10 produced

**[Table 9]**

| | Human DC | Human pMC | Human IL12-pMC |
|---|---|---|---|
| Medium | 0 pg/ml | 0 pg/ml | 0 pg/ml |
| poly(I:C) | 2.09 pg/ml | 0 pg/ml | 0 pg/ml |

### 15. Stimulating activity of human IL-12p70-producing proliferative myeloid cells on T cells and NK cells

Stimulating activity of the human IL-12p70-producing proliferative myeloid cells on antigen-specific CD8⁺ T cells, alloreactive T cells, and NK cells was evaluated.

### (15-1) Antigen-specific CD8⁺ T cell-stimulating activity

The human pMC and the human IL12-pMC produced in 12 above, and human GM-CSF/M-CSF-producing proliferative myeloid cells disclosed in WO2021/230304 (ipAPC-GMM, referred to as "human GMM-pMC" in the Examples) were loaded with 10 µM human telomerase reverse transcriptase (hTERT) 461-469 peptide (hereinafter, also referred to as the "hTERT peptide"). After 16 hours, the resultant cells were seeded in 96-well round bottom plates (5×10⁴ cells/well), and were irradiated (20 Gy). By the method described in paper by Arai J. et al. (Blood, 2001 May 1; 97 (9): 2903-7), hTERT peptide-specific CD8⁺ T cells prepared from the peripheral blood mononuclear cells of a HLA-A*24:02-positive donor (3×10⁴ cells/well) were added to each of the wells of the human pMC, the human IL12-pMC, and the GMM-pMC, and were co-cultured for 48 hours in 10% FCS-containing RPMI 1640. Culture supernatants were collected, and IFN-γ was quantified using ELISA MAX^{™} Standard Set Human IFN-γ (BioLegend). The IFN-γ production in the absence of hTERT peptide loading (vehicle) in each cells was also determined in the same way (control).

The results are shown in FIG. 42 and Table 10. In the figure, human pMC, human GMM-pMC, and human IL12-pMC are simply referred to as "pMC", "GMM-pMC", and "IL12-pMC" respectively. An asterisk (*) indicates a p-value obtained by one-way ANOVA with Tukey post-hoc test (**p < 0.01, ***p < 0.001). The human IL12-pMC had a higher ability to induce IFN-γ production in antigen-specific CD8⁺ T cells than the human pMC or human GMM-pMC. Surprisingly, the amount of IFN-γ produced by the hTERT peptide-specific CD8⁺ T cells co-cultured with the human IL12-pMC not loaded with hTERT peptide was more than 900-fold higher than that produced by the hTERT peptide-specific CD8⁺ T cell co-cultured with human pMC or human GMM-pMC not loaded with the hTERT peptide (Table 10). Moreover, surprisingly, the amount of IFN-γ produced by the hTERT peptide-specific CD8⁺ T cells co-cultured with human IL12-pMC loaded with the hTERT peptide was 3.8-fold than that produced by the hTERT peptide-specific CD8⁺ T cells co-cultured with human pMC or human GMM-pMC loaded with the hTERT peptide (Table 10).

### Table 10. Amount of IFN-γ produced by antigen-specific CD8⁺ T cells

**[Table 10]**

| | Human pMC | Human GMM-pMC | Human IL12-pMC |
|---|---|---|---|
| Vehicle | 0.007 ng/ml | 0.007 ng/ml | 6.33049 ng/ml |
| hTERT peptide | 6.55399 ng/ml | 5.43532 ng/ml | 20.71627 ng/ml |

### (15-2) Alloreactive T cell-stimulating activity

T cells were isolated from the peripheral blood mononuclear cells of a donor whose HLA types did not match those of the human pMC and the human IL12-pMC produced in 12 above, using Pan T Cell isolation kit, human (Miltenyi Biotec). The mixture of the T cells (5×10⁴ cells/well), and irradiated (20 Gy) human pMC, human IL12-pMC, human DC or poly (I:C)-stimulated human DC produced as in (14-2) above (mixing ratio of 15:1, 45:1, 135:1, 405:1, 1215:1, or 3645:1), or only the T cells were seeded in 96-well round bottom plates, and were co-cultured for 5 days in 10% FCS-containing RPMI 1640. Culture supernatants were collected, and IFN-γ was quantified using ELISA MAX^{™} Standard Set Human IFN-γ.

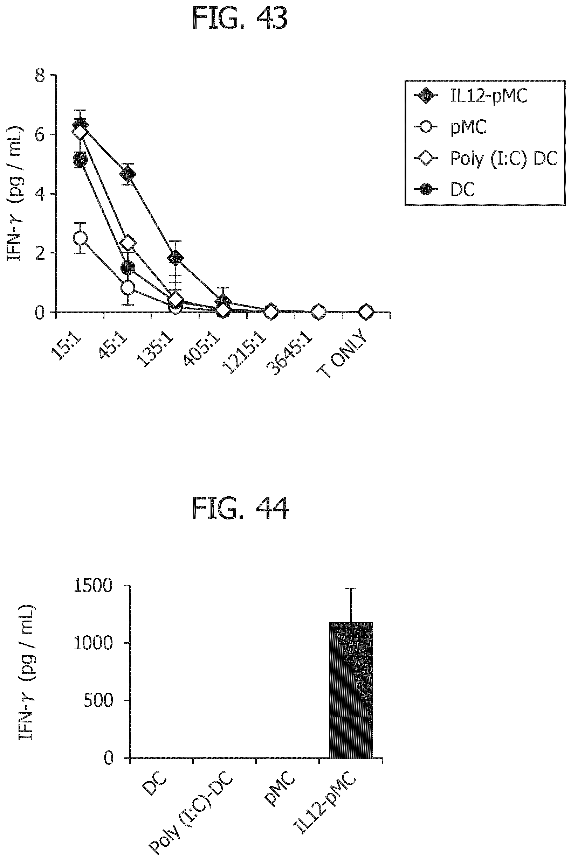

The results are shown in FIG. 43. The human IL12-pMC had a higher ability to induce IFN-γ production in the alloreactive T cells than the human pMC, the human DC, or the poly (I:C)-stimulated human DC.

### (15-3) NK cell-stimulating activity

NK cells were isolated from peripheral blood mononuclear cells of a donor whose HLA types matched those of the human pMC and the human IL12-pMC produced in 12 above, using NK cell isolation kit, human (Miltenyi Biotec). The NK cells (3×10⁴ cells/well) were mixed with the human pMC or the human IL12-pMC produced in 12 above, or the human DC or poly (I:C)-stimulated human DC produced in (14-2) above, were irradiated (20 Gy), and the resultants were seeded in 96-well round bottom plates, and co-cultured for 48 hours in 10% FCS-containing PRMI 1640. Culture supernatants were collected, and IFN-γ was quantified using ELISA MAX^{™} Standard Set Human IFN-γ.

The results are shown in FIG. 44 and Table 11. In the figure, "DC" means the human DC, "poly (I:C)-DC" means the poly (I:C)-stimulated human DC, "pMC" means the human pMC, and "IL12-pMC" means the human IL12-pMC. The human IL12-pMC had a higher ability to induce IFN-γ production in NK cells than the human pMC, the human DC, or the poly (I:C)-stimulated human DC. Surprisingly, the amount of IFN-γ produced by the NK cells co-cultured with the human IL12-pMC was at least 200-fold higher than that produced by the NK cells co-cultured with the human pMC, the human DC, or the poly (I:C)-stimulated human DC.

### Table 11. Amount of IFN-γ produced by NK cell

**[Table 11]**

| Human DC | Human poly(I:C)-DC | Human pMC | Human IL12-pMC |
|---|---|---|---|
| 5 pg/ml | 5 pg/ml | 5 pg/ml | 1175.69 pg/ml |

These results revealed that the human IL12-pMC have a strong ability to induce IFN-γ production in T cells and NK cells.

### 16. Irradiation to stop proliferation of human IL12-pMC

The human IL12-pMC produced in 12 above proliferates in GM-CSF- and M-CSF-dependent manners, and hence might be cancerous. Therefore, irradiation is expected before administration to humans. The radiation dose at which proliferation of human IL12-pMC is arrested in the presence or absence of GM-CSF and M-CSF was examined.

The human pMC and the human IL12-pMC produced in 12 above were seeded in 96-well round bottom plates (3×10³ cells/well) by using 20% FCS-containing αMEM supplemented with or without GM-CSF and M-CSF (50 ng/mL each),and were irradiated with 0, 2.5, 5, 10, 20, 40, or 80 Gy. After 6 days, 1 µCi 3H-thymidine was added thereto, and after 16 hours, the amount of radiation incorporated into the human pMC or human IL12-pMC was measured using a liquid scintillation counter.

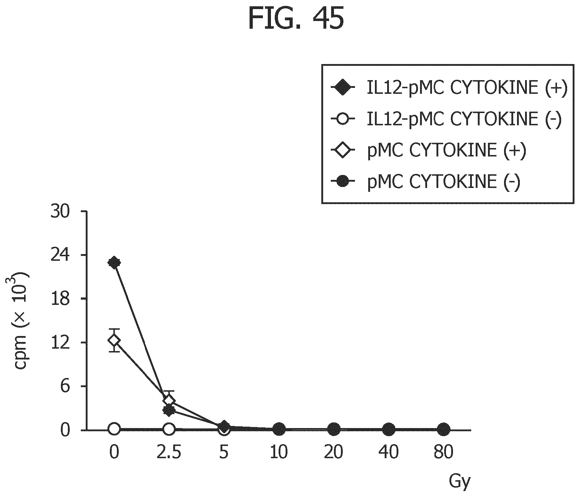

The results are shown in FIG. 45. In the figure, "IL12-pMC" means the human IL12-pMC, "pMC" means the human pMC, and "cytokine" means the GM-CSF and M-CSF. They did not proliferate even without irradiation (0 Gy), whereas in the presence of GM-CSF and M-CSF, they proliferated after irradiation with 0, 2.5, or 5 Gy, and proliferation was arrested by irradiation with 10 Gy.

### 17. Tumorigenicity of human IL12-pMC

As risk assessment for the application of the human IL12-pMC to human cancer therapy, the tumorigenicity of the human IL12-pMC was tested with severely immunodeficient mice (NSG mice, The Jackson Laboratory Japan). Human IL12-pMC into which a luciferase gene (CSII-EF-Luciferase-IRES-hygromycine phosphotransferase: distributed from Dr. Satoru Senju, Kumamoto University) had been introduced, with or without irradiation at 10 Gy, were intraperitoneally administered to NSG mice (n = 6) (5×10⁶ cells: 50 times the estimated therapeutic dose). The proliferation or loss of the human IL12-pMC in mice was evaluated over time by *in vivo* imaging. During evaluation, VivoGlo^{™} Luciferin, In Vivo Grade (Promega) was administered (3 mg/mouse) under 2% isoflurane inhalation anesthesia, and bioluminescence was measured with IVIS Lumina S5 (Perkin Elmer).

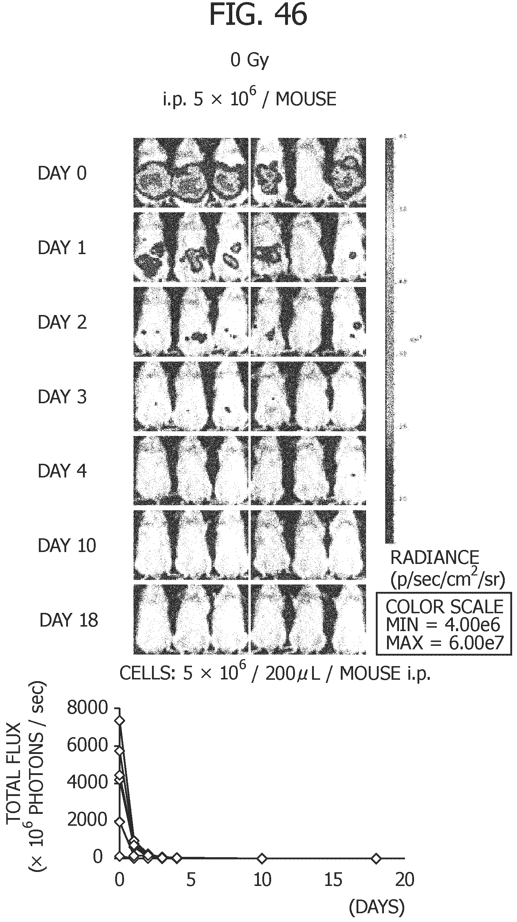

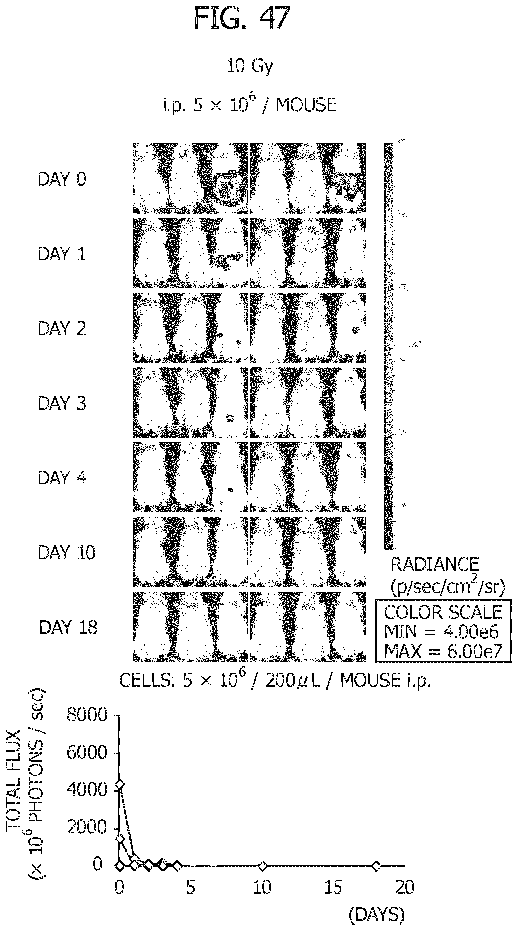

The results are shown in FIGS. 46 and 47. In mice intraperitoneally administered with non-irradiated human IL12-pMC, bioluminescence was no longer detectable after 4 days, and no tumorigenic growth was observed (FIG. 46). Similarly, in mice intraperitoneally administered with irradiated human IL12-pMC, bioluminescence was no longer detectable after 4 days, and no tumorigenic growth was observed (FIG. 47). Human IL12-pMC did not grow tumorigenically in immunodeficient mice regardless of irradiation, suggesting that human IL12-pMC is highly safe with respect to tumorigenicity and is suitable for pharmaceutical applications.

### 18. Antitumor effect of mouse IL12-pMC (without peptide loading)

### (18-1) Administration schedule

MO4 cells (1×10⁵) were subcutaneously inoculated in the both legs of C57BL/6N mice (day 0). On days 5, 12, 19, and 26, the IL12-pMC were administered around the inoculation site of the right leg (administered 1.25×10⁵ cells (n = 14), 2.5×10⁵ cells (n = 12), 5×10⁵ cells (n = 12) or 1×10⁶ cells (n = 12)). For a negative control, the therapeutic cells were not administered (n = 11). Each mouse was assessed over time for tumor growth and survival in the right and left legs. The endpoint was defined as the point when the long diameter of the tumor exceeded 15 mm.

### (18-2) Tumor volume

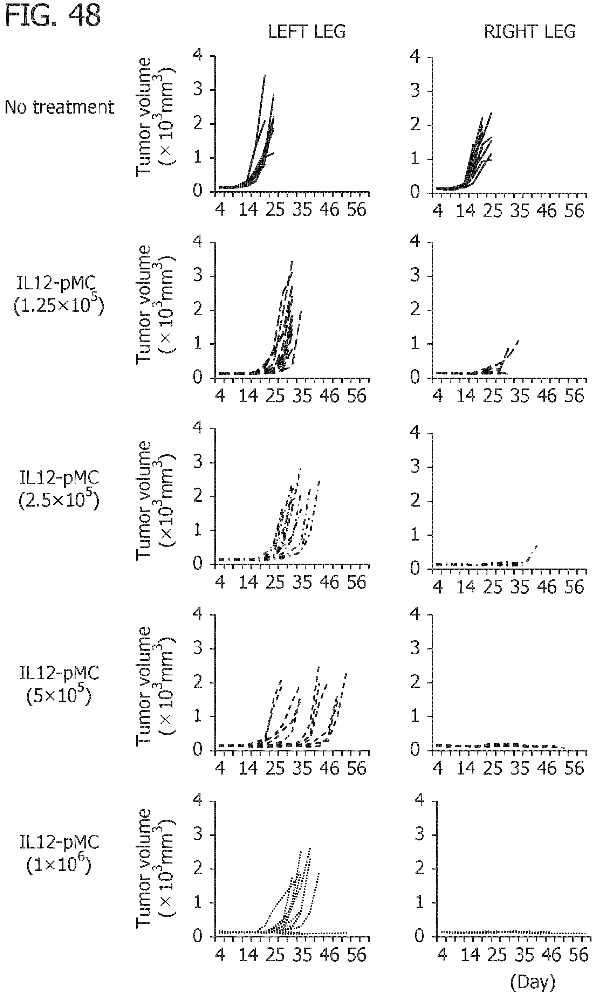

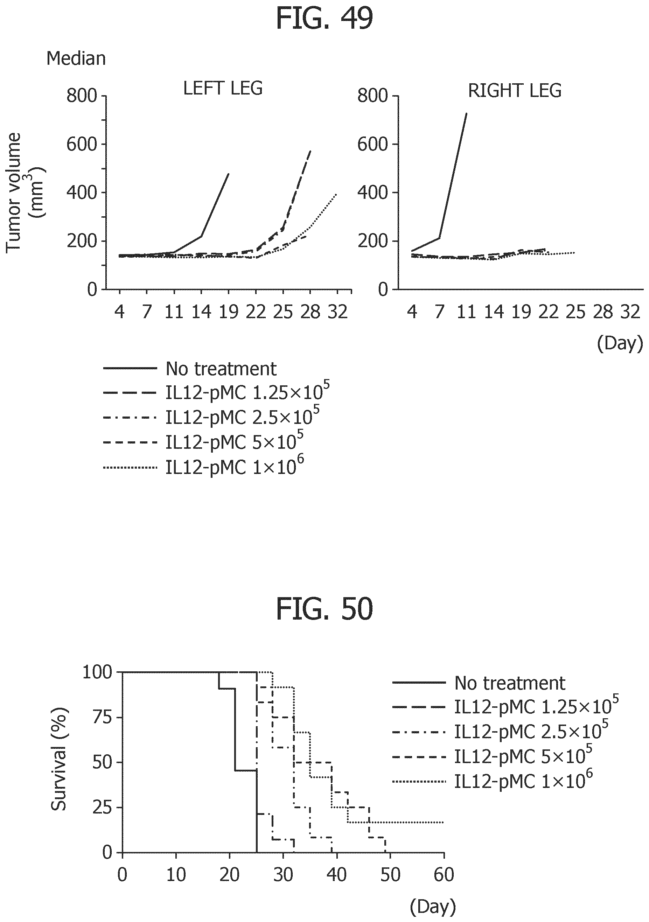

The changes in tumor volume over time in each mouse are shown in FIG. 48, and changes over time in the median tumor diameter for each mouse group are shown in FIG. 49. The results of the statistical significance test for the tumor volume in each mouse on day 19 are shown in Table 12. In the table, an asterisk (*) indicates a p-value obtained by one-way ANOVA with Tukey post-hoc test (****p < 0.0001), and "ns" indicates "no significant difference". The administration of the IL12-pMC strongly suppressed the growth of the tumors in the right leg (administration site). Moreover, surprisingly, the growth of the tumors in the left leg (remote site) was also significantly suppressed. These results indicate that IL12-pMC can suppress the growth of caner, not only at the administration site, but also at distant sites.

### Table 12. Statistically significance difference in tumor volume

**[Table 12]**

| | Left leg | | Right leg | |
|---|---|---|---|---|
| | P value | P value summary | P value | P value summary |
| No treatment vs IL12-pMC 1.25×10⁵ | <0.0001 | **** | <0.0001 | **** |
| No treatment vs IL12-pMC 2.5×10⁵ | <0.0001 | **** | <0.0001 | **** |
| No treatment vs IL12-pMC 5×10⁵ | <0.0001 | **** | <0.0001 | **** |
| No treatment vs IL12-pMC 1×10⁶ | <0.0001 | **** | <0.0001 | **** |
| IL12-pMC 1.25×10⁵ vs IL12-pMC 2.5×10⁵ | >0.9999 | ns | >0.9999 | ns |
| IL12-pMC 1.25×10⁵ vs IL12-pMC 5×10⁵ | >0.9999 | ns | >0.9999 | ns |
| IL12-pMC 1.25×10⁵ vs IL12-pMC 1×10⁶ | 0.9997 | ns | 0.9998 | ns |
| IL12-pMC 2.5×10⁵ vs IL12-pMC 5×10⁵ | >0.9999 | ns | >0.9999 | ns |
| IL12-pMC 2.5×10⁵ vs IL12-pMC 1×10⁶ | >0.9999 | ns | >0.9999 | ns |
| IL12-pMC 5×10⁵ vs IL12-pMC 1×10⁶ | 0.9998 | ns | 0.9998 | ns |

### (18-2) Survival rate of mice

Survival rates of the mice are shown in FIG. 50, and the results of the statistical significance test are shown in Table 13. In the table, an asterisk (*) indicates a p-value obtained by log-rank test (*p < 0.05, **p < 0.01, ***p < 0.001, ****p < 0.0001), and "ns" indicates "no significant difference". IL12-pMC extended the survival of mice bearing tumors in both legs in a dose-dependent manner.

### Table 13. Statistically significant difference in survival rate

**[Table 13]**

| | P value | P value summary |
|---|---|---|
| No treatment vs IL12-pMC 1.25×10⁵ | <0.0001 | **** |
| No treatment vs IL12-pMC 2.5×10⁵ | <0.0001 | **** |
| No treatment vs IL12-pMC 5×10⁵ | <0.0001 | **** |
| No treatment vs IL12-pMC 1×10⁵ | <0.0001 | **** |
| IL12-pMC 1.25×10⁵ vs IL12-pMC 2.5×10⁵ | 0.0004 | *** |
| IL12-pMC 1.25×10⁵ vs IL12-pMC 5×10⁵ | 0.0001 | *** |
| IL12-pMC 1.25×10⁵ vs IL12-pMC 1×10⁶ | <0.0001 | **** |
| IL12-pMC 2.5×10⁵ vs IL12-pMC 5×10⁵ | 0.0419 | * |
| IL12-pMC 2.5×10⁵ vs IL12-pMC 1×10⁶ | 0.0535 | ns |
| IL12-pMC 5×10⁵ vs IL12-pMC 1×10⁶ | 0.3147 | ns |

### 19. Antitumor effect of mouse IL12-pMC (2) (without peptide loading)

### (19-1) Administration Schedule

MO4 cells (1×10⁵) were subcutaneously inoculated in both legs of C57BL/6N mice (day 0). The following four treatment groups were created (n = 12 per group). A negative control group received neither therapeutic cells nor antibodies (n=12). Each mouse was assessed over time for tumor growth and survival in the right and left legs. The endpoint was defined as the point when the long diameter of the tumor exceeded 15 mm.

Treatment group 1: pMC (1×10⁶) were administered to the right leg on days, 5, 7, 12, 14, 19, and 21.

Treatment group 2: anti-PD-L1 antibody (200 µg) was intraperitoneally administered on days 5, 12, and 19.

Treatment group 3: IL12-pMC (1×10⁶) were administered to the right leg on days 5, 7, 12, 14, 19, and 21.

Treatment group 4: IL12-pMC (1×10⁶) were administered to the right leg on days 5, 7, 12, 14, 19, and 21, and anti-PD-L1 antibody (200 µg) was intraperitoneally administered on days 5, 12, and 19.

### (19-2) Tumor volume

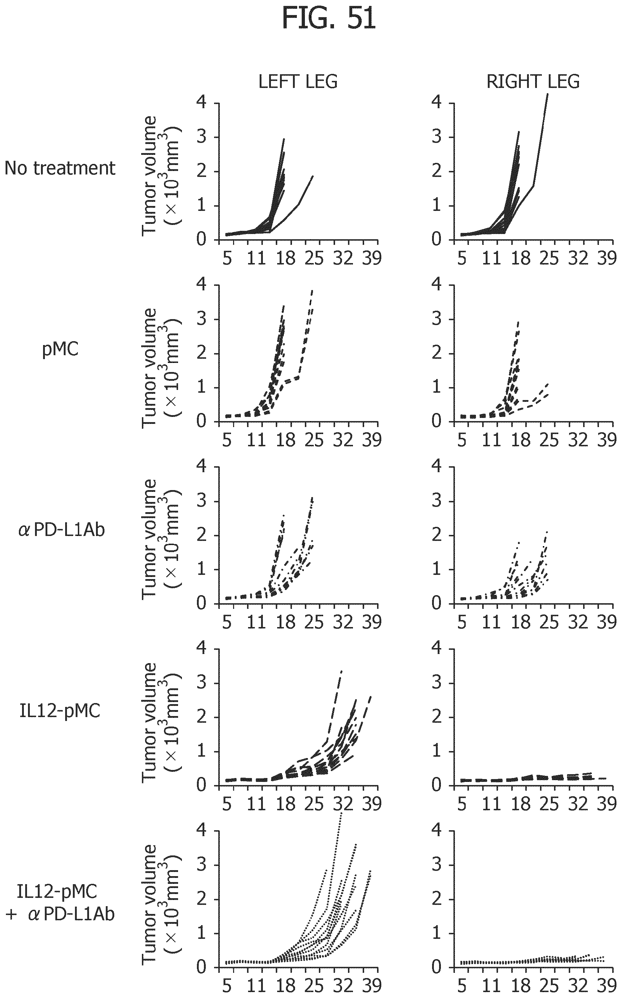

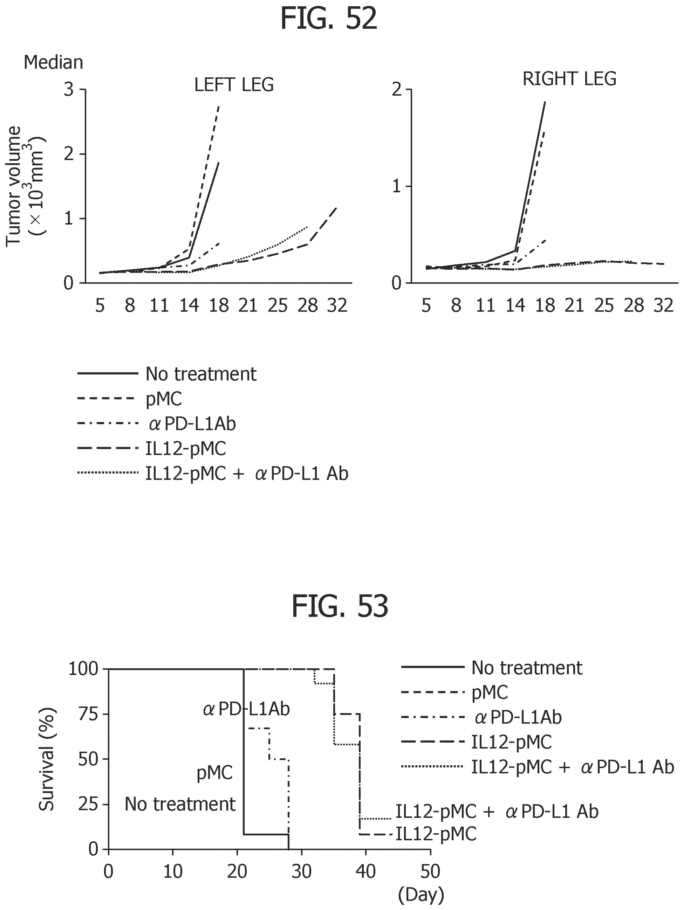

The changes in tumor volume over time in each mouse are shown in FIG. 51, and changes over time in the median tumor diameter for each mouse group are shown in FIG. 52. In the figures, "pMC" indicates the treatment group 1, "αPD-L1Ab" indicates the treatment group 2, "IL12-pMC" indicates the treatment group 3, and "IL12-pMC+αPD-L1Ab" indicates the treatment group 4. The results of a statistical significance test for the tumor volume in each mouse on day 18 are shown in Table 14. In the table, an asterisk (*) indicates a p-value obtained by one-way ANOVA with Tukey post-hoc test (*p < 0.05, **p < 0.01, ***p < 0.001, ****p < 0.0001), and "ns" indicates "no significant difference". Treatment groups 3 and 4, which received IL12-pMC, had a superior cancer suppression effect in the right leg (administration site) compared to the groups that did not receive IL12-pMC (untreated group, treatment groups 1 and 2). No significant difference was observed between treatment group 3, which received only IL12-pMC, and treatment group 4, which received both IL12-pMC and an anti-PD-L1 antibody, but the cancer suppression effect in treatment group 4 was superior.

### Table 14. Statistically significance difference in tumor volume

**[Table 14]**

| | Left leg | | Right leg | |
|---|---|---|---|---|
| | P value | P value summary | P value | P value summary |
| No treatment vs pMC | 0.1995 | ns | 0.0891 | ns |
| No treatment vs αPD-L1 Ab | 0.0259 | * | <0.0001 | **** |
| No treatment vs IL12-pMC | <0.0001 | **** | <0.0001 | **** |
| No treatment vs IL12-pMC+αPD-L1 Ab | <0.0001 | **** | <0.0001 | **** |
| pMC vs αPD-L1 Ab | <0.0001 | **** | 0.0025 | ** |
| pMC vs IL12-pMC | <0.0001 | **** | <0.0001 | **** |
| pMC vs IL12-pMC+aPD-L1 Ab | <0.0001 | **** | <0.0001 | **** |
| αPD-L1 Ab vs IL12-pMC | 0.009 | ** | 0.0014 | ** |
| αPD-L1 Ab vs IL12-pMC+aPD-L1 Ab | 0.0075 | ** | 0.0009 | *** |
| IL12-pMC vs IL12-pMC+aPD-L1 Ab | >0.9999 | ns | >0.9841 | ns |

### (18-3) Survival rate of mice

The survival rates of mice are shown in FIG. 53, and the results of a statistical significance test are shown in Table 15. In the table, an asterisk (*) indicates a p-value obtained by log-rank test (*p < 0.05, **p < 0.01, ***p < 0.001, ****p < 0.0001), and "ns" indicates "no significant difference". In the treatment group 3 (administered IL12-pMC), the survival of mice was extended more than in the negative control group, the treatment group 1 (administered pMC), and the treatment group 2 (administered anti-PD-L1 antibody). No significance difference in survival time was observed between the treatment group 3 (administered IL12-pMC) and the treatment group 4 (administered IL12-pMC + anti-PD-L1 antibody). On the other hand, the survival time of mice in treatment group 4 (administered IL12-pMC + anti-PD-L1 antibody) was extended more than in treatment group 2 (administered anti-PD-L1 antibody).

### Table 15. Statistically significance difference in survival rate

**[Table 15]**

| | P value | P value summary |
|---|---|---|
| No treatment vs pMC | 0.6532 | ns |
| No treatment vs αPD-L1 Ab | 0.0117 | * |
| No treatment vs IL12-pMC | <0.0001 | **** |
| No treatment vs IL12-pMC+αPD-L1 Ab | <0.0001 | **** |
| pMC vs αPD-L1 Ab | 0.1693 | ns |
| pMC vs IL12-pMC | 0.0003 | *** |
| pMC vs IL12-pMC+aPD-L1 Ab | 0.0002 | *** |
| αPD-L1 Ab vs IL12-pMC | 0.0234 | * |
| αPD-L1 Ab vs IL12-pMC+aPD-L1 Ab | 0.0188 | * |
| IL12-pMC vs IL12-pMC+aPD-L1 Ab | 0.4349 | ns |

These results revealed that IL12-pMC can exhibit the antitumor effect without load with a cancer antigen.

### 20. Production of MHC-deficient mouse proliferative myeloid cells (universal-type pMC)

The H-2Kb/H-2Db and I-Ab genes in the pMC produced in 1 above were knocked out by genome editing to produce a universal-type pMC (pMC-Univ). A complex of an sgRNA targeting a consensus sequence in the mouse H-2Kb/H-2Db gene (Cancer Sci., 2019, 110 (10): 3027-3037; SEQ ID NO: 13) and Cas9 protein (Guide-it^{™} Recombinant Cas9, Takara Bio), and a complex of an sgRNA targeting a sequence in the mouse I-Ab gene (Cancer Sci., 2019, 110 (10): 3027-3037; SEQ ID NO: 14) and Cas9 protein were introduced into the pMC using Neon Transfection System (Thermo Fisher Scientific). TrueGuide Synthetic gRNAs were used as the sgRNAs. After the introduction, the cells were cultured in pMC medium for 7 days. The cells were stained with the following antibodies, and H-2Kb/Db-negative I-A/E-negative cells were isolated by flow cytometry. The cells were named "pMC-Univ".

### Table 16. Antibodies used for staining

**[Table 16]**

| Antigen | Species | Clone | Source |
|---|---|---|---|
| H-2K^{b}/D^{b} | Mouse | 28-8-6 | BioLegend |
| I-A/E | Mouse | M5/114.15.2 | BioLegend |

The pMC-Univ were cultured for 24 hours in pMC medium with or without 50 ng/mL mouse IFN-γ (BioLegend), and were stained with the antibody shown in Table 16 to be analyzed by flow cytometry. The pMC was used as a control.

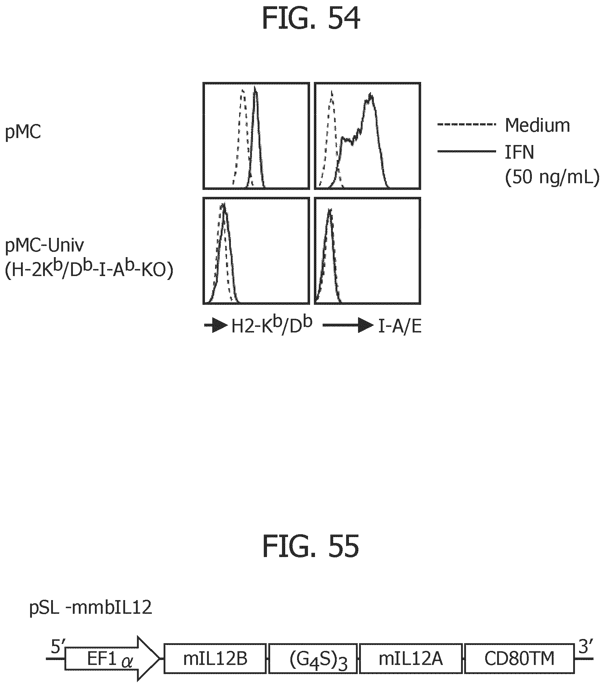

The results are shown in FIG. 54. It is known that IFN-γ has the ability to upregulate the expression of MHC, and the expression of H-2Kb/H-2Db and I-Ab increased in the pMC cultured in the presence of IFN-γ. On the contrary, in pMC-Univ, even when cultured in the presence of IFN-γ, no expression of H-2Kb/H-2Db or I-Ab was observed. These results confirmed that the pMC-Univ is deficient in H-2Kb/H-2Db and I-Ab.

### 21. Production of membrane-bound-IL-12-expressing MHC-deficient mouse proliferative myeloid cells

### (21-1) Production of lentiviral vector expressing membrane-bound mouse IL-12

The cDNA (J. Immunother. Cancer, 2020, 8: e000210) was synthesized by entrusting GenScript, connecting in the 5' to 3' direction, the cDNA of mouse I112a (SEQ ID NO: 1), a nucleic acid sequence encoding the (G₄S)₃ linker (SEQ ID NO: 15), the cDNA of I112b (SEQ ID NO: 2), and a nucleic acid sequence encoding the transmembrane domain of mouse CD80 (SEQ ID NO: 16). The fused cDNA was inserted into the multicloning site of pSL (SEQ ID NO: 7) to obtain pSL-mbIL12 (FIG. 55).

The pSL-mbIL12, pCAG-HIVgp, and pCMV-VSV-G-RSV-Rev were introduced into 293T cells by lipofection. Three days after the lipofection, the culture supernatant was collected and filtered through a filter with a pore size of 0.45 µm, and then the viral particles were concentrated using a Lenti-X concentrator (Clontech). The obtained viral particles were suspended in DMEM, dispensed into cryovials, and stored at -150°C.

### (21-2) Production of membrane-bound-IL-12-expressing MHC-deficient pMC

The pMC-Univ produced in 20 above were seeded in 48-well plates, and were infected with the lentiviral vector produced in (21-1) above. For growth culture after the infection, the pMC medium was used. Eight days after the infection, the resultant cells were stained with an anti-IL12/23 antibody (Table 17), and IL12/23-positive cells were isolated by flow cytometry. The isolated IL12/23-positive cells continued to proliferate in pMC medium for more than three months. The cells were named "mbIL12-pMC-Univ".

### Table 17. Antibody used for staining

**[Table 17]**

| Antigen | Species | Clone | Source |
|---|---|---|---|
| IL12/23 | Mouse | C15.6 | BioLegend |

Membrane-bound-IL-12-expressing pMC was produced as a control through the same procedures except that pMC was used instead of pMC-Univ, and the resultant was named "mbIL12-pMC".

### (21-3) Cell surface marker analysis

The pMC, the pMC-Univ, the mbIL12-pMC, and the mbIL12-pMC-Univ were stained with an anti-IL12/23 antibody (Table 17) to be analyzed by flow cytometry.

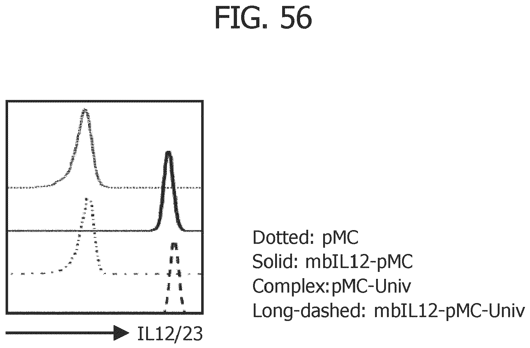

The results are shown in FIG. 56. The pMC and the pMC-Univ were not stained by the anti-IL12/23 antibody, confirming that they do not express IL-12 on the cell surface. In contrast, the mbIL12-pMC and the mbIL12-pMC-Univ were stained by the anti-IL12/23 antibody, confirming that they expressed IL-12 on the cell surface.

### 22. Production of chimeric antigen receptor-macrophage (CAR-M)-like membrane-bound-IL-12-expressing MHC-deficient mouse proliferative myeloid cells

Therapeutic efficacy of chimeric antigen receptor (CAR)-expressing macrophages (CAR-M) against solid tumors has been reported (Nat. Biotechnol., 2020, 38 (8) 947-953). It is also known that the phagocytosis of macrophages is activated by inhibiting the CD47-SIRP signaling in macrophages, and SIRPα-deficient macrophages have a strong ability to kill cancer cells (Nat. Commun., 2021, 12(1): 3229). In the Examples, membrane-bound-IL-12-expressing MHC-deficient mouse proliferative myeloid cells and membrane-bound-IL-12-expressing MHC/SIRPα-deficient mouse proliferative myeloid cells, expressing CAR which consists of an scFv derived from anti-human glypican 3 (GPC3) antibody clone GC33 (US 7919086 B) (hereinafter, simply referred to as "GC33"), a CD8α-derived transmembrane domain, and a CD3ζ signaling domain, were produced.

### (22-1) Production of mouse proliferative myeloid cell lacking MHC and SIRPα

A complex of an sgRNA (TrueGuide Synthetic gRNA) targeting a sequence (SEQ ID NO: 17) in mouse SIRPα gene and Cas9 protein was introduced into the pMC-Univ and the mbIL12-pMC-Univ using Neon Transfection System (Thermo Fisher Scientific). After the introduction, the cells were cultured in pMC medium for 7 days. The cells were stained with the following antibody, and SIRPα-negative cells were isolated by flow cytometry. The thus obtained cells were named respectively as "pMC-Univ-SIRPα-KO" and "mbIL12-pMC-Univ-SIRPα-KO".

### Table 18. Antibody used for staining

**[Table 18]**

| Antigen | Species | Clone | Source |
|---|---|---|---|
| SIRPα | Mouse | P84 | BioLegend |

### (22-2) Production of lentiviral vector expressing GC33-based CAR

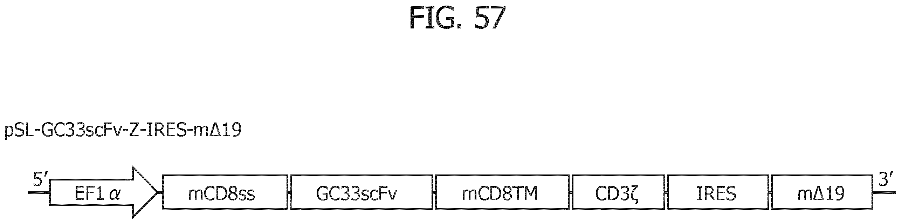

A cDNA was synthesized by entrusting GenScript, connecting in the 5' to 3' direction, a cDNA encoding a signaling sequence of mouse CD8α (CDS1-81) (SEQ ID NO: 18), a nucleic acid sequence encoding VH of GC33 (SEQ ID NO: 19), a nucleic acid sequence encoding 205c linker (Biochemistry, 1991, 30 (42): 10117-10125) (SEQ ID NO: 20), a nucleic acid sequence encoding VL of GC33 (SEQ ID NO: 21), a cDNA encoding a transmembrane region of mouse CD8α (CDS424-699) (SEQ ID NO: 22), a cDNA encoding an intracellular region of mouse CD3ζ (CDS154-495) (SEQ ID NO: 23), an IRES sequence (SEQ ID NO: 11), and a cDNA encoding an extracellular region of mouse CD19 (CDS1-993) (SEQ ID NO: 24). The fused cDNA was inserted into the multicloning site of the pSL (SEQ ID NO: 7) to obtain SL-GC33scFv-Z-IRES-mΔ19 (FIG. 57).

The pSL-GC33scFv-Z-IRES-mΔ19, pCAG-HIVgp, and pCMV-VSV-G-RSV-Rev were introduced into 293T cells by lipofection. Three days after the lipofection, the culture supernatant was collected and filtered through a filter with a pore size of 0.45 µm, and then the viral particles were concentrated using Lenti-X concentrator (Clontech). The obtained viral particles were suspended in DMEM, dispensed into cryovials, and stored at -150°C.

### (22-3) Production of CAR-M-like MHC-deficient mouse proliferative myeloid cells

The pMC-Univ, the mbIL12-pMC-Univ, the pMC-Univ-SIRPα-KO, and the mbIL12-pMC-Univ-SIRPα-KO were respectively seeded in 48-well plates, and were infected with the lentiviral vector produced in (22-2) above. For growth culture after the infection, the pMC medium was used. Eight days after the infection, the resultant cells were stained with following the antibodies, and CD19-positive cells were isolated by flow cytometry. The thus obtained cells were respectively named "pMC-Univ-GC33-Z", "mbIL12-pMC-Univ-GC33-Z", "pMC-Univ-SIRPα-KO-GC33-Z", and "mbIL12-pMC-Univ-SIRPα-KO-GC33-Z".

### Table 19. Antibody used for staining

**[Table 19]**

| Antigen | Species | Clone | Source |
|---|---|---|---|
| SIRPα | Mouse | P84 | BioLegend |

### (22-4) Cell surface marker analysis

The pMC-Univ, the mbIL12-pMC-Univ, and the cells produced in (22-1) and (22-3) above were stained with antibodies shown in Tables 17 to 19, and FITC-labeled human GPC3 (ACRO Biosystems) to be analyzed by flow cytometry.

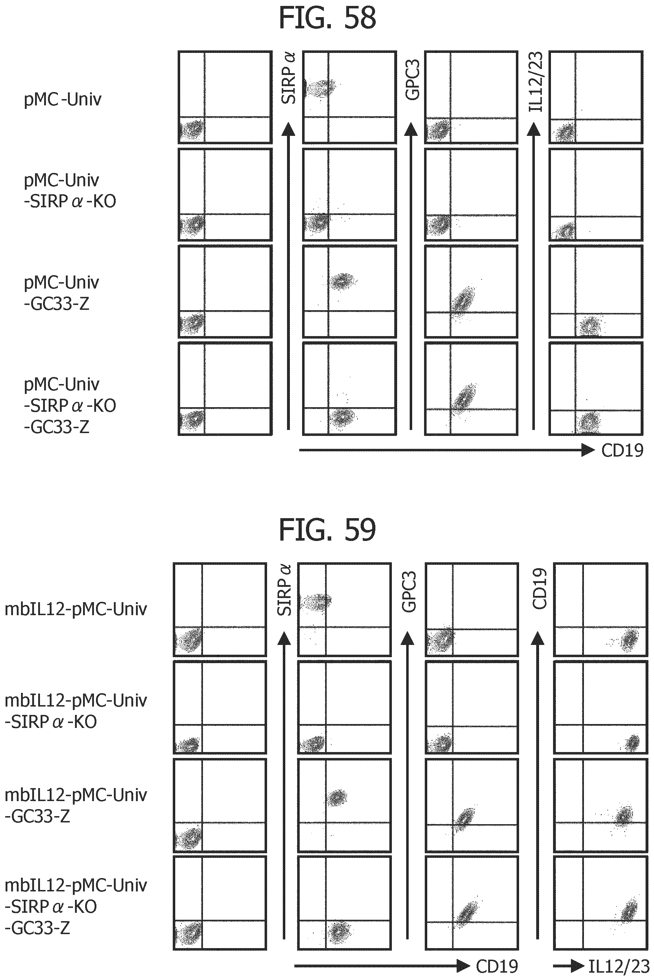

The results are shown in FIGS. 58 and 59. The pMC-Univ was SIRPα-positive, CD19-negative, GPC3-binding-negative, and IL12/23-negative. The pMC-Univ-SIRPα-KO was SIRPα-negative, CD19-negative, GPC3-binding-negative, and IL12/23-negative. The pMC-Univ-GC33-Z was SIRPα-positive, CD19-positive, GPC3-binding-positive, and IL12/23-negative. The pMC-Univ-SIRPα-KO-GC33-Z was SIRPα-negative, CD19-positive, GPC3-binding-positive, and IL12/23-negative. The mbIL12-pMC-Univ was SIRPα-positive, CD19-negative, GPC3-binding-negative, and IL12/23-positive. The mbIL12-pMC-Univ-SIRPα-KO was SIRPα-negative, CD19-negative, GPC3-binding-negative, and IL12/23-positive. The mbIL12-pMC-Univ-GC33-Z was SIRPα-positive, CD19-positive, GPC3-binding-positive, and IL12/23-positive. The mbIL12-pMC-Univ-SIRPα-KO-GC33-Z was SIRPα-negative, CD19-positive, GPC3-binding-positive, and IL12/23-positive.

### 23. Cancer-killing activity of CAR-M-like membrane-bound-IL-12-expressing MHC-deficient mouse proliferative myeloid cells

### (23-1) Production of a cancer cell model expressing the cancer antigen GPC3

### (i) Production of luciferase-expressing MC38

Luciferase-expressing MC38 (MC38-Luc) was produced by introducing a luciferase gene (CSII-EF-Luciferase-IRES-hygromycine phosphotransferase; distributed from Dr. Satoru Senju, Kumamoto University) into mouse colorectal cancer cell line MC38 (distributed from Dr. Jeffery Schlom, National Cancer Institute USA).

### (ii) Production of lentiviral vector expressing GPC3

A cDNA of human GPC3 (RefSeq ID: NM_004484, synthesis entrusted to GenScrip) was inserted into the multicloning site of SCII-EF plasmid (RIKEN BRC DNA BANK) to produce CEII-EF-GPC3. The CSII-EF-GPC3, the pCAG-HIVgp, and the pCMV-VSV-G-RSV-Rev were introduced into 293T cells by lipofection. Three days after the lipofection, the culture supernatant was collected and filtered through a filter with a pore size of 0.45 µm, and the viral particle was concentrated with Lenti-X concentrator. The obtained viral particles were suspended in DMEM, dispensed into cryovials, and stored at -150°C.

### (iii) Production of MC38 that expresses GPC3 and luciferase

The MC38-Luc cells produced in (i) above were seeded in 48-well plates, and were infected with the lentiviral vector produced in (ii) above. For growth culture after the infection, 10% FCS-containing DMEM was used. Eight days after the infection, the cells were stained with the following antibody, and GPC3-positive cells were isolated by flow cytometry. The thus obtained cells were named "MC38-Luc-GPC3".

### Table 20. Antibody used for staining

**[Table 20]**

| Antigen | Species | Clone | Source |
|---|---|---|---|
| Glypican-3 | Human | 1G12 | BioMosaics |

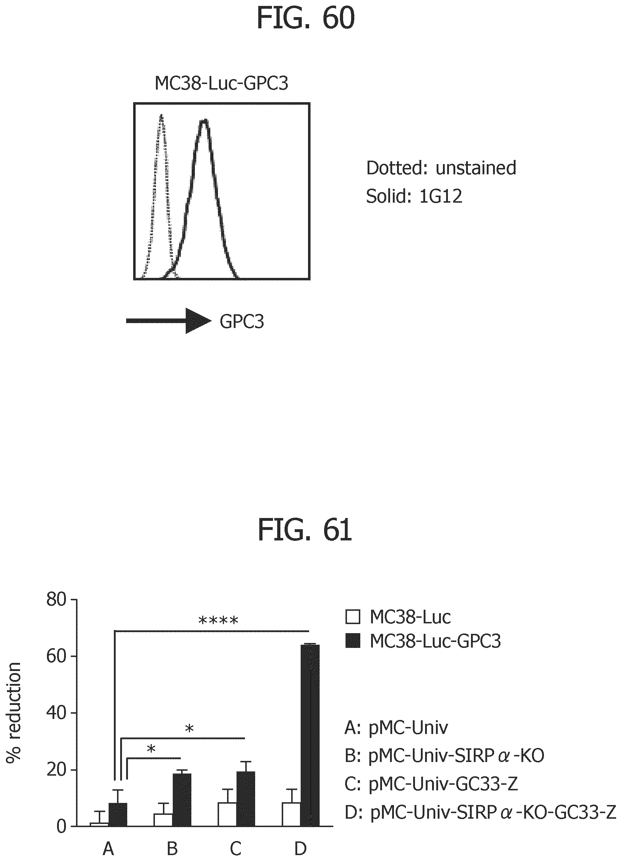

The MC38-Luc-GPC3 was stained with an anti-GPC3 antibody (Table 20) to be analyzed by flow cytometry. The results are shown in FIG. 60. It was thus confirmed that the MC38-Luc-GPC3 expressed GPC3 on the cell surface.

### (23-2) Evaluation of cancer-killing activity

The MC38-Luc-GPC3 or the MC38-Luc was used as target cells, and the pMC-Univ, the mbIL12-pMC-Univ, and the cells produced in (22-1) and (22-3) above were used as effector cells. The target cells (3×10³ cells/well) and the effector cells (3×10⁴ cells/well) were seeded on 96-well plates, and they were co-cultured in 10% FCS-containing RPMI. After adding a substrate of luciferase (Bright-Glo^{™} Luciferase assay, Promega Corporation) thereto and incubating for 5 minutes, the bioluminescence was measured with SpectraMax^{™} i3 (Molecular Devices). The percentage reduction of bioluminescence (% reduction) was calculated using the following formula: [(maximum luminescence - background luminescence) - (sample luminescence - background luminescence)]/(maximum luminescence - background luminescence) ×100

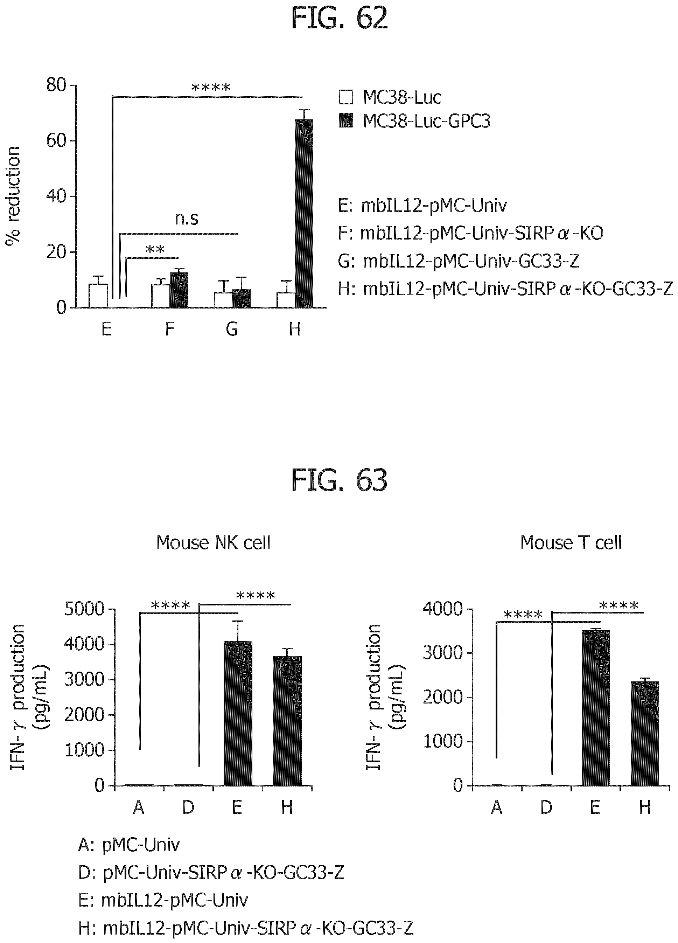

The results are shown in FIGS. 61 and 62. In the figures, an asterisk (*) indicates a p-value obtained by one-way ANOVA with Tukey post-hoc test (*p < 0.05, **p < 0.01, ***p < 0.001, ****p < 0.0001), and "ns" indicates "no significant difference". Co-culture of the MC38-Luc-GPC3 and the pMC-Univ-SIRPα-KO-GC33-Z and co-culture of the MC38-Luc-GPC3 and the mbIL12-pMC-Univ-SIRPα-KO-GC33-Z reduced bioluminescence by more than 60% (FIG. 61D, FIG. 62H). These results confirmed that the pMC-Univ-SIRPα-KO-GC33-Z and the mbIL12-pMC-Univ-SIRPα-KO-GC33-Z had strong cancer-killing activity.

### 24. Ability of membrane-bound-IL-12-expressing MHC-deficient mouse proliferative myeloid cells to induce IFN-γ production

NK cells were isolated from splenocytes of C57BL/6N mice by using NK Cell Isolation Kit, mouse (Miltenyi Biotec). The pMC-Univ, the pMC-Univ-SIRPα-KO-GC33-Z, the mbIL12-pMC-Univ, and the mbIL12-pMC-Univ-SIRPα-KO-GC33-Z were seeded on 96-well plates (1.0×10⁴ cells/well each) and were irradiated (20 Gy). Thereafter, NK cells (5×10⁴ cells/well) were added thereto, and were co-cultured for 48 hours in 10% FCS-containing RPMI 1640. Culture supernatants were collected, and IFN-γ was quantified using ELISA MAX^{™} Standard Set Human IFN-y.

In addition, co-cultures with the pMC-Univ, the pMC-Univ-SIRPα-KO-GC33-Z, the mbIL12-pMC-Univ, and the mbIL12-pMC-Univ-SIRPα-KO-GC33-Z were performed through the same procedures as described above, except T cells were used instead of NK cells, and IFN-γ was quantified. T cells isolated from splenocytes of C57BL/6N mice using Pan T cell isolation kit, mouse (Miltenyi Biotec).

The results are shown in FIG. 63. In the figure, an asterisk (*) indicates a p-value obtained by one-way ANOVA with Tukey post-hoc test (*p < 0.05, **p < 0.01, ***p < 0.001, ****p < 0.0001), and "ns" indicates "no significant difference". The mbIL12-pMC-Univ, and the mbIL12-pMC-Univ-SIRPα-KO-GC33-Z strongly induced the IFN-γ production in NK cells and in T cells. These results revealed that membrane-bound IL-12-expressing MHC-deficient mouse proliferative myeloid cells have a strong ability to induce IFN-γ production by T cells and NK cells regardless of the expression of CAR.

### 25. Antitumor effect of membrane-bound-IL-12-expressing MHC-deficient mouse proliferative myeloid cells in vivo

### (25-1) Production of MC38-Luc-GPC3 lacking H-2Kb/H-2Db

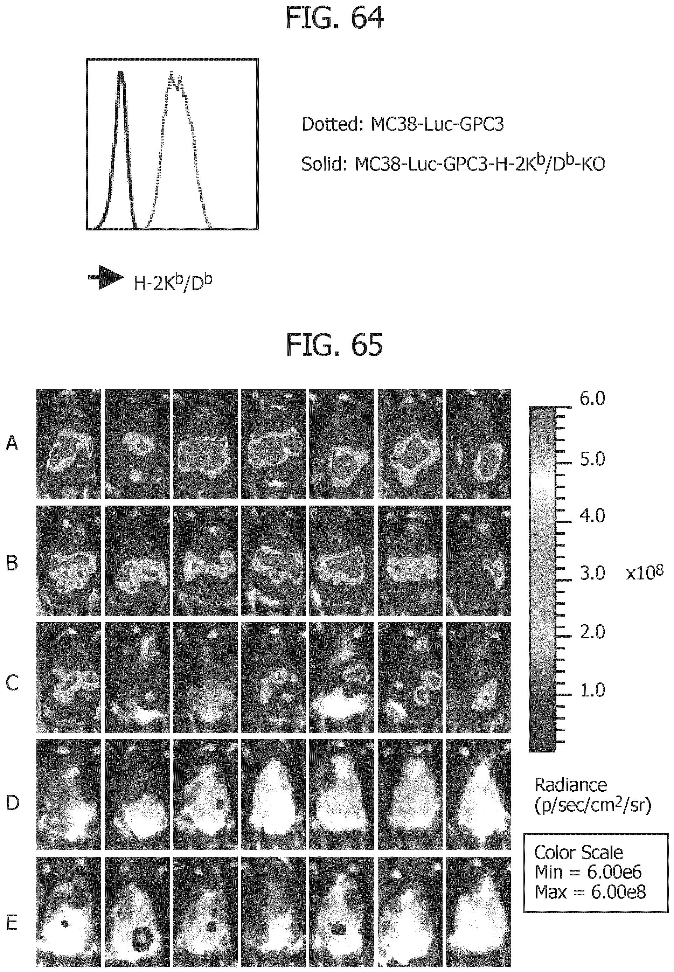

When MC38-Luc-GPC3 is administered to mice, T-cell responses can be induced by GPC3 presented on MHC of the MC38-Luc-GPC3 *per se.* Therefore, in order to accurately evaluate the *in vivo* antitumor effect of only the membrane-bound-IL-12-expressing MHC-deficient mouse proliferative myeloid cells, H-2Kb/H-2Db-lacking MC38-Luc-GPC3 was produced as a cancer cell model that cannot cause T-cell response by itself. The H-2Kb/H-2Db gene in the MC38-Luc-GPC3 produced in (23-1) above was knocked out by the same procedures as in 20 above. DMEM containing 10% FCS was used for growth culture after introduction of the sgRNA-Cas9 protein complex. After culturing for 7 days, H-2Kb/Db-negative I-A/E-negative cells were isolated by flow cytometry, and were named "MC38-Luc-GPC3-H-2Kb/H-2Db-KO" (FIG. 64).

### (25-2) Antitumor effect

The MC38-Luc-GPC3-H-2Kb/H-2Db-KO cells (5×10⁵/300 µL) were intraperitoneally inoculated in C57BL/6N mice (day 0). On days 1, 3, 7, 10, 14, 17, 21, 25, 28, and 31, 3×10⁶/300 µL of therapeutic cells (the pMC-Univ, the pMC-Univ-SIRPα-KO-GC33-Z, the mbIL12-pMC-Univ, or the mbIL12-pMC-Univ-SIRPα-KO-GC33-Z; irradiated with 10 Gy) were intraperitoneally administered (n = 7 each). In a negative control group, the therapeutic cells were not administered (n = 7). In a healthy control group, neither inoculation of cancer cells nor administration of the therapeutic cells was performed (n = 7). On days 1, 3, 7, 10, 14, 17, 22, 25, 29, and 32, VivoGlo^{™} Luciferin was intraperitoneally administered, and the bioluminescence was measured with IVIS Lumina S5. The endpoint was defined as 20% or more weight loss, hypothermia (≤ 32°C), or ascites accumulation observed compared to healthy controls.

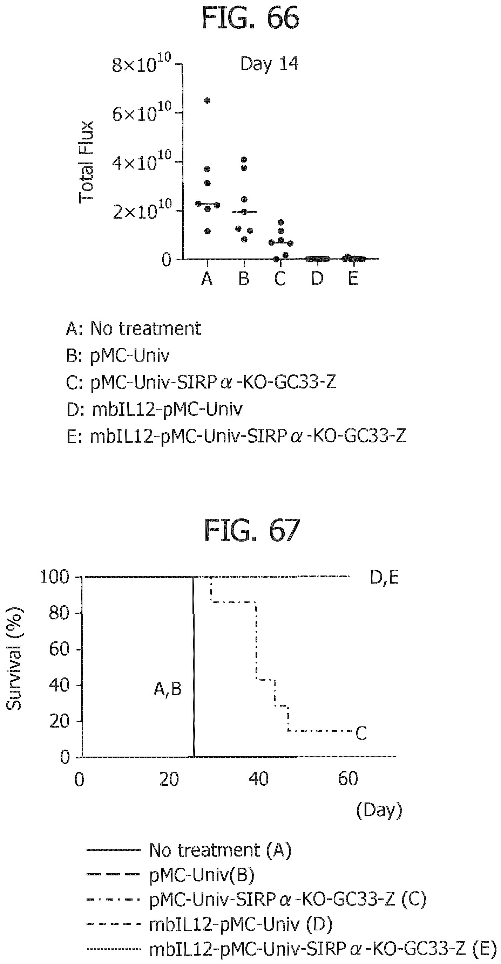

The results of *in vivo* imaging of each mouse on day 14 are shown in FIG. 65, and the total flux of luciferase luminescence (photons/sec) is shown in FIG. 66. In the figures, "A" indicates the negative control group, "B" indicates the pMC-Univ-administered group, "C" indicates the pMC-Univ-SIRPα-KO-GC33-Z-administered group, "D" indicates the mbIL12-pMC-Univ-administered group, and "E" indicates the mbIL12-pMC-Univ-SIRPα-KO-GC33-Z-administered group. Also, the results of statistical significance tests for the total flux of luciferase luminescence shown in FIG. 66 are presented in Table 21. In the table, an asterisk (*) indicates a p-value obtained by two-tailed Student's t-test (*p < 0.05, **p < 0.01, ***p < 0.001, ****p < 0.0001), and "ns" indicates "no significant difference". The pMC-Univ-SIRPα-KO-GC33-Z exhibited stronger inhibition of cancer growth than the negative control group and the pMC-Univ. Also, the mbIL12-pMC-Univ and the mbIL12-pMC-Univ-SIRPα-KO-GC33-Z exhibited the strongest inhibition of cancer growth, and the effect was significantly stronger than that of the pMC-Univ-SIRPα-KO-GC33-Z. The pMC-Univ did not show any inhibitory effect on cancer growth.

### Table 21. Statistically significant difference in luciferase luminescence intensity

**[Table 21]**

| | P value | P value summary |
|---|---|---|
| A vs B | 0.5772 | ns |
| A vs C | 0.0014 | ** |
| A vs D | <0.0001 | **** |
| A vs E | <0.0001 | **** |
| B vs C | 0.0143 | * |
| B vs D | 0.0007 | *** |
| C vs D | 0.0037 | ** |
| C vs E | 0.0045 | ** |
| D vs E | 0.2161 | ns |

### (25-3) Survival rate of mice

Survival rates of the mice are shown in FIG. 67, and the results of a statistical significance test on the survival rates of FIG. 67 are shown in Table 22. In the table, an asterisk (*) indicates a p-value obtained by log-rank test (*p < 0.05, **p < 0.01, ***p < 0.001, ****p < 0.0001), and "ns" indicates "no significant difference". The mbIL12-pMC-Univ and the mbIL12-pMC-Univ-SIRPα-KO-GC33-Z extended the survival time the most, and the effect was significantly stronger than that of the pMC-Univ-SIRPα-KO-GC33-Z. The pMC-Univ did not extend the survival time.

### Table 22. Statistically significant difference in survival rate

**[Table 22]**

| | P value | P value summary |
|---|---|---|
| A vs B | >0.9999 | ns |
| A vs C | 0.0003 | *** |
| A vs D | 0.0003 | *** |
| A vs E | 0.0003 | *** |
| B vs C | 0.0003 | *** |
| B vs D | 0.0003 | *** |
| B vs E | 0.0003 | *** |
| C vs D | 0.0013 | ** |
| C vs E | 0.0013 | ** |
| D vs E | >0.9999 | ns |

These results revealed the antitumor effects of the pMC-Univ-SIRPα-KO-GC33-Z, the mbIL12-pMC-Univ, and the mbIL12-pMC-Univ-SIRPα-KO-GC33-Z. The pMC-Univ-SIRPα-KO-GC33-Z, the mbIL12-pMC-Univ, and the mbIL12-pMC-Univ-SIRPα-KO-GC33-Z had stronger antitumor effects than the pMC-Univ. The mbIL12-pMC-Univ, and the mbIL12-pMC-Univ-SIRPα-KO-GC33-Z exhibited strongest antitumor effect.

### 26. Antitumor effect of membrane-bound-IL-12-expressing MHC-deficient mouse proliferative myeloid cells in vivo (2)

The mbIL12-pMC-Univ and the mbIL12-pMC-Univ-SIRPα-KO-GC33-Z, which were confirmed to have strong antitumor effects in 25 above, were compared with each other in more detail. MC38-Luc-GPC3-H-2Kb/H-2Db-KO cells (5×10⁵/500 µL) were intraperitoneally inoculated in C57BL/6N mice (day 0). On days 2, 4, 8, 11, 14, and 17, 3×10⁶/300 µL, 1×10⁶/300 µL, 3×10⁵/300 µL, or 1×10⁵/300 µL of the mbII,12-pMC-Univ, or the mbIL12-pMC-Univ-SIRPα-KO-GC33-Z (irradiated with 10 Gy) was intraperitoneally administered (n = 10 each). In a control group, the pMC-Univ-SIRPα-KO-GC33-Z (3×10⁶/300 µL) were administered (n = 10). In a negative control group, the therapeutic cells were not administered (n = 10). On days 4, 8, 11, 15, and 18, VivoGlo^{™} Luciferin was intraperitoneally administered, and the bioluminescence was measured with IVIS Lumina S5.

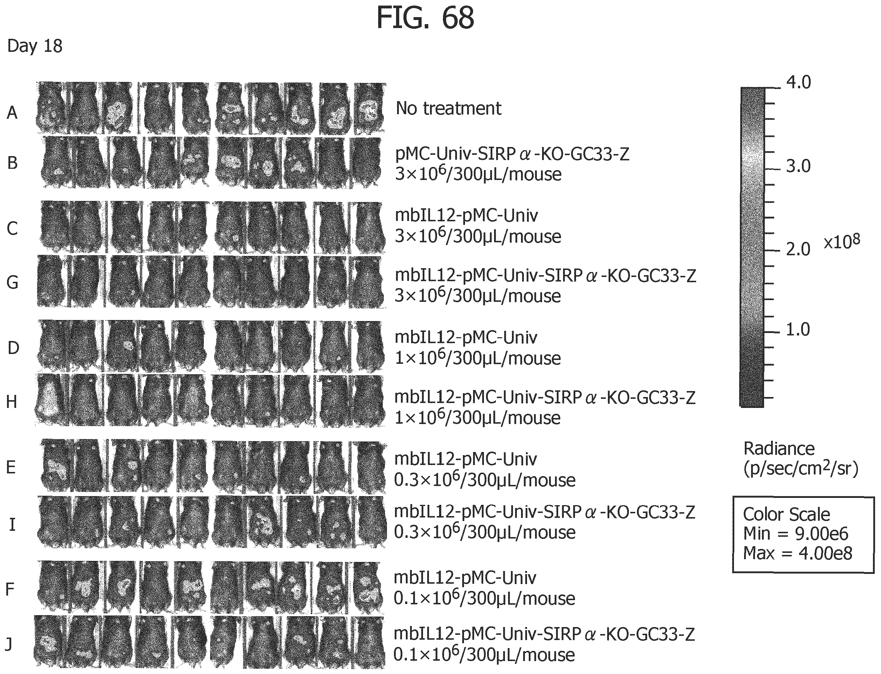

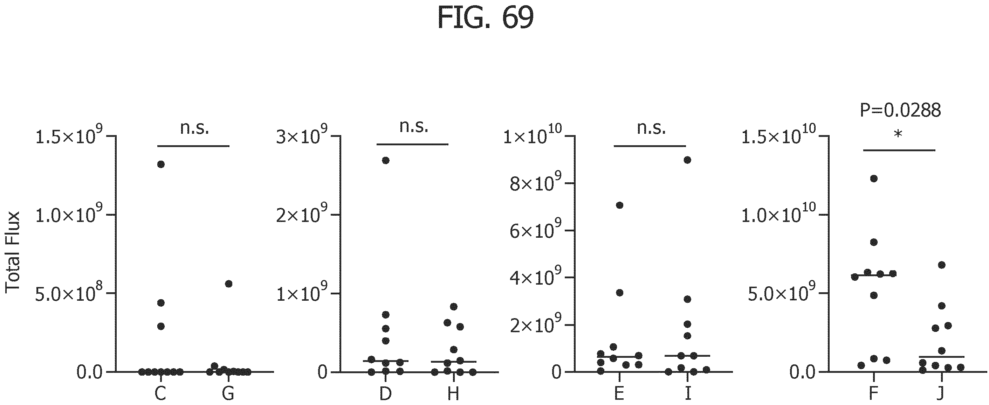

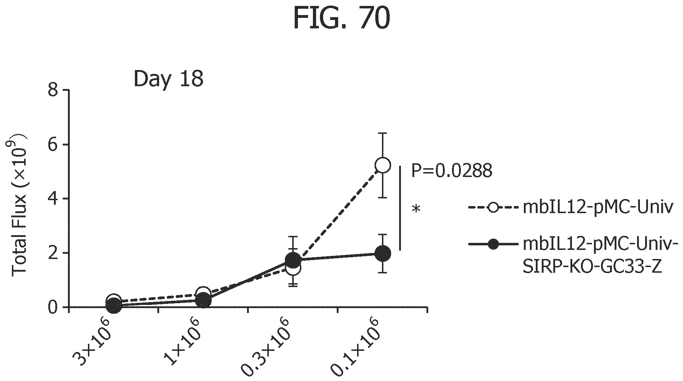

The results of *in vivo* imaging of each mouse on day 18 are shown in FIG. 68, the total flux of luciferase luminescence (photons/sec) is shown in FIG. 69, and a graph summarizing them for each group is shown in FIG. 70. In the figures, an asterisk (*) indicates a p-value obtained by Mann-Whitney U test (*p < 0.05). In comparison, at doses of 3×10⁶/300 µL, 1×10⁶/300 µL, or 3×10⁵/300 µL, no significant differences were observed between the mbIL12-pMC-Univ and the mbIL12-pMC-Univ-SIRPα-KO-GC33-Z. However, when compared at a dose of 1×10⁵/300 µL, the mbIL12-pMC-Univ-SIRPα-KO-GC33-Z suppressed cancer growth more significantly than the mbIL12-pMC-Univ. These results revealed that mbIL12-pMC-Univ-SIRPα-KO-GC33-Z exhibited a particularly excellent antitumor effects, and would be extremely useful for the development of universal cellular formulations for cancer treatment.

### 27. Production of HLA-matched IL-12p70-producing human proliferative myeloid cells

### (27-1) Production of endogenous-HLA-deficient human proliferative myeloid cells (universal-type human pMC)

A complex of Cas9 protein and sgRNA targeting the consensus sequence in the HLA-A*24:02, HLA-B*40:06, HLA-B*55:02, HLA-C*01:02 and HLA-C*15:02 genes (Cell Stem Cell, 2019, 24 (4): 566-578, e7; SEQ ID NO: 25), and a complex of Cas9 protein and sgRNA targeting human MHC class II transactivator (CIITA) gene (TrueGuide Synthetic gRNA; SEQ ID NO: 26), was introduced into the human pMC produced in (12-1) above using Neon Transfection System. After the introduction, the cells were cultured in pMC medium for 7 days. The resultant cells were stained with the following antibodies, and an HLA-ABC-negative HLA-DR-negative cells were isolated by flow cytometry. The thus obtained cells were named "pMC-HLA-KO".

Through similar procedures, endogenous HLA-deficient cells (universal-type human IL12-pMC) were produced from the human IL12-pMC produced in (12-3) above, and were named "IL12-pMC-HLA-KO".

### Table 23. Antibodies used for staining

**[Table 23]**

| Antigen | Species | Clone | Source |
|---|---|---|---|
| HLA-ABC | Human | W6/32 | BioLegend |
| HLA-DR | Human | L243 | BioLegend |

### (27-2) Production of lentiviral vector expressing HLA-A2 and HLA-A24

The cDNA of HLA-A2 (HLA*02:01) (IPD accession No. HLA00005; SEQ ID NO: 27) and the cDNA of HLA-A24 (HLA-A*24:02) (IPD accession No. HLA00050; SEQ ID NO: 28) were synthesized by entrusting GenScript. The cDNAs were inserted into the multicloning site of CSII-EF-MCS plasmid (RIKEN BRC DNA BANK) to produce CSII-EF-HLA-A2 anDCII-EF-HLA-A24. The CSII-EF-HLA-A2 or CSII-EF-HLA-A24, and pCAG-HIVgp and pCMV-VSV-G-RSV-Rev were introduced into 293T cells by lipofection. Three days after the lipofection, the culture supernatants were collected and filtered through a filter with a pore size of 0.45 µm, and then the viral particles were concentrated using a Lenti-X concentrator (Clontech). The obtained viral particles were suspended in DMEM, dispensed into cryovials, and stored at -150°C.

### (27-3) Production of endogenous HLA-deficient human proliferative myeloid cells expressing exogenous HLA-A2 and HLA-A24

The pMC-HLA-KO and the IL12-pMC-HLA-KO produced in (27-1) above were infected with the lentivirus produced in (27-2) above. For growth culture after the infection, the hpMC medium was used. Five days after the infection, the resultant cells were stained with the following antibodies, and HLA-A2-positive cells or HLA-A24-positive cells were isolated by flow cytometry. The isolated cells continued to grow in hpMC medium for more than three months. The thus obtained exogenous-HLA-A2-expressing universal-type human pMC, exogenous-HLA-A24 expressing universal-type human pMC, exogenous-HLA-A2 expressing universal-type human IL12-pMC, and exogenous HLA-A24-expressing universal-type human IL12-pMC were respectively named "pMC-HLA-KO-A2", "pMC-HLA-KO-A24", "IL12-pMC-HLA-KO-A2", and "IL12-pMC-HLA-KO-A24".

### Table 24. Antibody used for staining

**[Table 24]**

| Antigen | Species | Clone | Source |
|---|---|---|---|
| HLA-A24 | Human | 17A10 | MBL |
| HLA-A2 | Human | BB7.2 | MBL |

### (27-4) Cell surface marker analysis

The human pMC, the pMC-HLA-KO, the pMC-HLA-KO-A2, the pMC-HLA-KO-A24, the IL12-pMC-HLA-KO-A2, and the IL12-pMC-HLA-KO-A24 were stained with a fluorescently labeled anti-CD19 antibody (BioLegend, Clone 4G7) and antibodies shown in Tables 23 and 24 to be analyzed by flow cytometry.

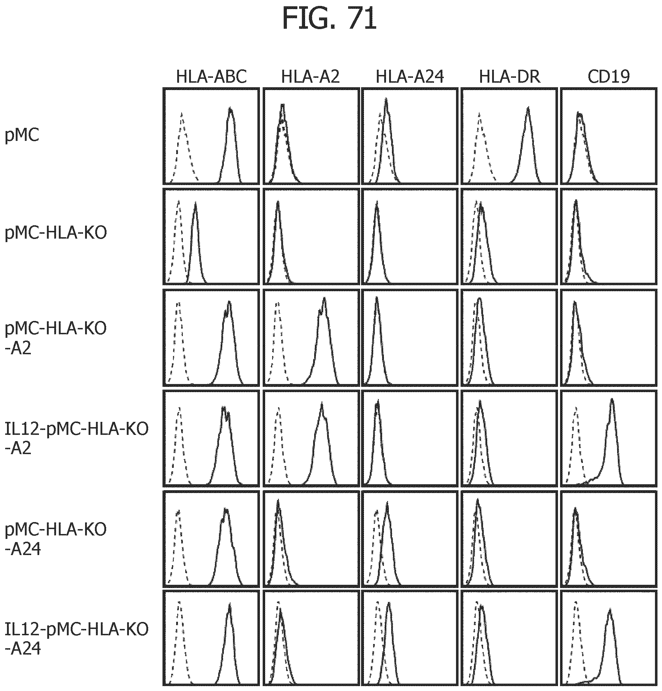

The results are shown in FIG. 71. The histograms of unstained cells are shown in dotted line, and the histograms of stained cells are shown in solid line. The HLA type of a donor of the iPS cell from which the human pMC was derived (hereinafter, referred to as the "pMC donor") was HLA-A2-negative HLA-A24-positive, with which the staining result of the human pMC matched. On the other hand, the pMC-HLA-KO was not stained with any of the anti-HLA-A2 antibody, the anti-HLA-A24 antibody, and the anti-HLA-DR antibody. The pMC-HLA-KO-A2 and the IL12-pMC-HLA-KO-A2 were stained with the anti-HLA-A2 antibody. The pMC-HLA-KO-A24 and the IL12-pMC-HLA-KO-A24 were stained with the anti-HLA-A24 antibody. Also, the IL12-pMC-HLA-KO-A2 and the IL12-pMC-HLA-KO-A24 were stained with the anti-CD19 antibody, confirming that they express CD19 (a marker for IL12-expression).

### 28. Immune evasion ability of HLA-matched IL-12p70-producing human proliferative myeloid cells

T cells were isolated from peripheral blood mononuclear cells of the pMC donor, as well as donors A and B, whose HLA types do not match those of the pMC donor, using a Pan T cell isolation kit, human. The thus isolated T cells were respectively named "pMC-donor T cell", "donor-A T cell", and "donor-B T cell".

The HLA types of the pMC donor, the donor A, and the donor B are shown in Table 25. The donor A matches with the pMC donor in having HLA-A24, but is different from the pMC donor in a part of HLA-B and HLA-C. The donor B is completely different from the pMC donor in HLA-A and HLA-C, and is different from the pMC donor in a part of HLA-B.

### Table 25. HLA type of donor

**[Table 25]**

| | | | | | | |
|---|---|---|---|---|---|---|
| pMC donor | HLA-A | | HLA-B | | HLA-C | |
| | 24:02:01 | - | 40:06:01 | 55:02:01 | 01:02:01 | 15:02:01 |
| | HLA-DRB1 | | HLA-DRB3/4/5 | | HLA-DQA1 | |
| | 04:05:01 | 09:01:02 | 4*01:03:01 | 4*01:03:02 | 03:02:01 | 03:03:01 |
| | HLA-DQB1 | | HLA-DPA1 | | HLA-DPB1 | |
| | 03:03:02 | 04:01:01 | 01:03:01 | 02:02:02 | 02:01:02 | 05:01:01 |
| donor A | HLA-A | | HLA-B | | HLA-C | |
| | 24:02:01 | 31:01:02 | 40:01:02 | 51:01:01 | 14:02:01 | 15:02:01 |
| | HLA-DRB1 | | HLA-DRB3/4/5 | | HLA-DQA1 | |
| | 12:02:01 | 14:05:01 | 3*02:02:01 | 3*03:01:03 | 01:04:01 | 06:01:01 |
| | HLA-DQB1 | | HLA-DPA1 | | HLA-DPB1 | |
| | 03:01:01 | 05:03:01 | 02:02:02 | - | 05:01:01 | - |
| donor B | HLA-A | | HLA-B | | HLA-C | |
| | 26:02:01 | 31:01:02 | 40:06:01 | 51:01:01 | 08:01:01 | 14:02:01 |
| | HLA-DRB1 | | HLA-DRB3/4/5 | | HLA-DQA1 | |
| | 09:01:02 | 14:03:01 | 3*01:01:02 | 4*01:03:02 | 03:02:01 | 05:07 |
| | HLA-DQB1 | | HLA-DPA1 | | HLA-DPB1 | |
| | 03:01:01 | 03:03:02 | 01:03:01 | 02:02:02 | 02:01:02 | 05:01:01 |

The human pMC, the pMC-HLA-KO, the pMC-HLA-KO-A2, or the pMC-HLA-KO-A24 (irradiated with 20 Gy) (1×10⁴ cells/well) were mixed with the pMC-donor T cells, the donor-A T cells, or the donor-B T cells (5×10⁵ cells/well) and were co-cultured in 10% FCS-containing RPMI 1640. After 6 days, 1 µCi 3H-thymidine was added thereto, and after 16 hours, the amount of radiation incorporated into the T cells were measured using a liquid scintillation counter.

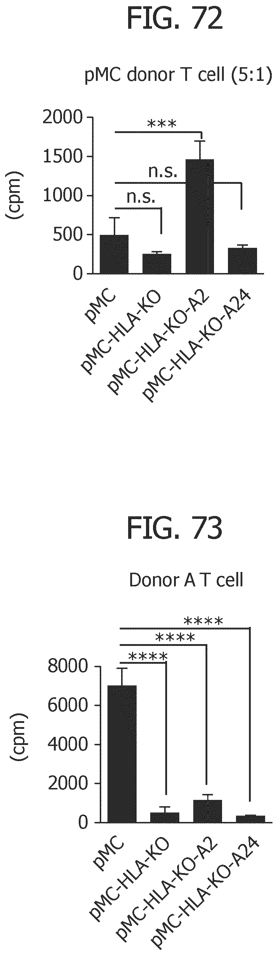

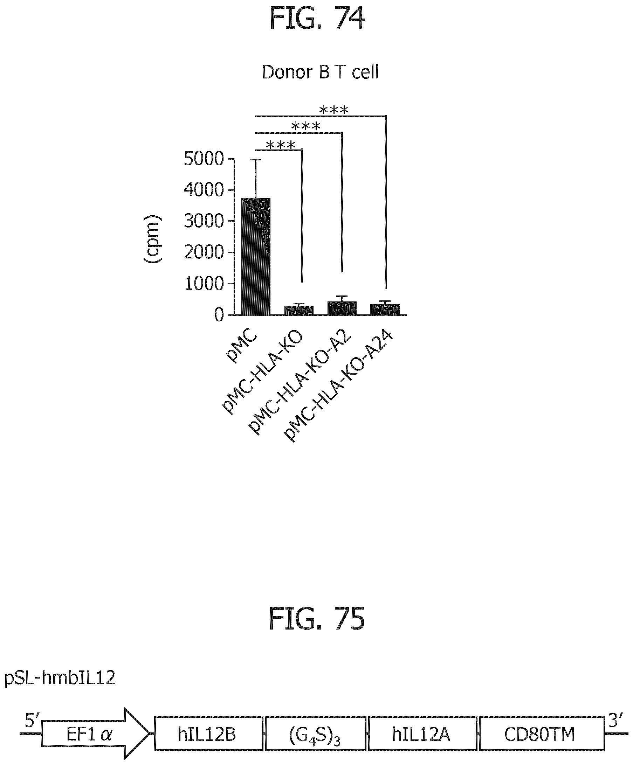

The results are shown in FIGS. 72 to 74. An asterisk (*) indicates a p-value obtained by one-way ANOVA with Tukey post-hoc test (*p < 0.05, **p < 0.01, ***p < 0.001, ****p < 0.0001), and "n.s." indicates "no significant difference". The pMC-donor T cells showed a strong proliferative response to the pMC-HLA-KO-A2, and thus, occurrence of immune rejection was confirmed (FIG. 72), whereas they did not show a proliferative response to the pMC-HLA-KO or pMC-HLA-KO-A24, and thus, avoidance of immune rejection was confirmed (FIG. 72). The donor-A T cell and the donor-B T cell showed a strong proliferative response only to the pMC (FIGS. 73 and 74). Since the donor B had neither HLA-A2 nor HLA-A24, it was presumed that the proliferative response of the donor-B T cell to the pMC-HLA-KO-A2 and the pMC-HLA-KO-A24 would increase; however, the increase was not observed (FIG. 74). The results of statistical significance test are shown in Tables 26 to 28.

### Table 26. Statistically significance difference in proliferative response of pMC-donor T cells

**[Table 26]**

| | P value | P value summary |
|---|---|---|
| pMC vs pMC-HLA-KO | 0.3405 | ns |
| pMC vs pMC-HLA-KO-A2 | 0.0004 | *** |
| pMC vs pMC-HLA-KO-A24 | 0.6294 | ns |

### Table 27. Statistically significance difference in proliferative response of donor-A T cells

**[Table 27]**

| | P value | P value summary |
|---|---|---|
| pMC vs pMC-HLA-KO | <0.0001 | **** |
| pMC vs pMC-HLA-KO-A2 | <0.0001 | **** |
| pMC vs pMC-HLA-KO-A24 | <0.0001 | **** |

### Table 28. Statistically significance difference in proliferative response of donor-B T cells

**[Table 28]**

| | P value | P value summary |
|---|---|---|
| pMC vs pMC-HLA-KO | 0.0006 | *** |
| pMC vs pMC-HLA-KO-A2 | 0.0008 | *** |
| pMC vs pMC-HLA-KO-A24 | 0.0007 | *** |

These results demonstrate that antigen-presenting cells that induce only the desired T-cell response without eliciting an alloreactive T-cell response can be generated by deleting endogenous HLA and then exogenously expressing the necessary HLA.

### 29. Production of membrane-bound-IL-12-expressing MHC-deficient human proliferative myeloid cells

### (29-1) Production of lentiviral vector expressing membrane-bound human IL-12

The cDNA (J. Immunother. Cancer, 2020, 8: e000210) was synthesized by entrusting GenScript, connecting in the 5' to 3' direction, the cDNA of human IL-12B (SEQ ID NO: 9), a nucleic acid sequence encoding the (G₄S)₃ linker (SEQ ID NO: 15), CDS169-762 of the cDNA of human IL-12A (SEQ ID NO: 8), and a nucleic acid sequence encoding the transmembrane domain (CDS721-867) of human CD80. The fused cDNA was inserted into the multicloning site of the pSL (SEQ ID NO: 7) to obtain pSL-hmbIL12 (FIG. 75).

The pSL-hmbIL12, the pCAG-HIVgp, and the pCMV-VSV-G-RSV-Rev were introduced into 293T cells by lipofection. Three days after the lipofection, the culture supernatant was collected and filtered through a filter with a pore size of 0.45 µm, and then the viral particles were concentrated using a Lenti-X concentrator (Clontech). The obtained viral particles were suspended in DMEM, dispensed into cryovials, and stored at -150°C.

### (29-2) Production of membrane-bound-IL-12-expressing MHC-deficient human pMC

The human pMC were seeded on 48-well plates, and were infected with the lentiviral vector produced in (29-1) above. For growth culture after the infection, the hpMC medium was used. Eight days after the infection, the resultant cells were stained with the following antibody, and IL12-70-positive cells were isolated by flow cytometry. The isolated IL12p70-positive cells continued to proliferate in pMC medium for more than three months. The cells were named "human mbIL12-pMC".

### Table 29. Antibody used for staining

**[Table 29]**

| Antigen | Species | Clone | Source |
|---|---|---|---|
| IL12p70 | Human | 130-103-741 | Miltenyi Biotec |

### (29-3) Cell surface marker analysis

The human pMC and the human mbIL12-pMC were stained with an anti-IL12p70 antibody (Table 29) to be analyzed by flow cytometry.

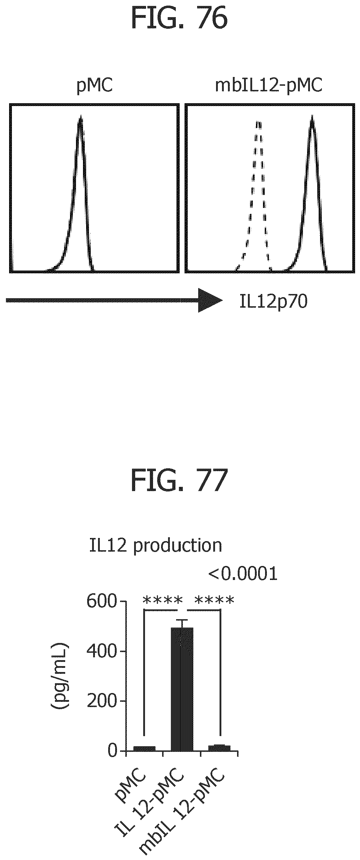

The results are shown in FIG. 76. In the figure, the word "human" is omitted from each cell name. The histograms of unstained cells are shown in dotted line, and the histograms of stained cells are shown in solid line. It was confirmed that the human mbIL12-pMC expressed IL12p70 on the cell surface.

### 30. IL12-secretion ability of membrane-bound-IL-12-expressing MHC-deficient human proliferative myeloid cells

The human pMC, the human IL12-pMC, or the human mbIL12-pMC (2×10³ cells/well each) were seeded on 96-well plates, and cultured for 24 hours in 10% FCS-containing RPMI 1640. Culture supernatants were collected, and IL-12p70 was quantified using ELISA MAX^{™} Standard Set Human IL-12 (p70).

The results are shown in FIG. 77. In the figure, the word "human" is omitted from each cell name. In the figure, an asterisk (*) indicates a p-value obtained by one-way ANOVA with Tukey post-hoc test (****p < 0.0001). IL-12 was detected from the culture supernatant of the human IL12-pMC, whereas IL-12 was not detected from the culture supernatant of the human mbIL12-pMC. These results revealed that human mbIL12-pMC expressed IL12 on the cell surface, but the cell does not secrete it extracellularly.

### 31. Surface marker analysis of membrane-bound-IL-12-expressing MHC-deficient human proliferative myeloid cells

The expression of a surface marker on the human pMC, the human IL12-pMC, the human mbIL12-pMC, and the human DC was analyzed by flow cytometry. For immunostaining, antibodies shown in Table 30 below were used. The human DC was prepared through the procedures described in (14-2) above.

### Table 30. Antibodies Used for Marker Staining

**[Table 30]**

| Antigen | Species | Clone | Source |
|---|---|---|---|
| CD116 | Human | 4H1 | BioLegend |
| IL12Rβ2 | Human | S16020B | BioLegend |
| CD115 | Human | 9-402-1 E4 | BioLegend |
| CD3 | Human | HIT3a | BioLegend |
| CD16 | Human | 3G8 | BioLegend |
| CD32 | Human | FUN-2 | BioLegend |
| CD64 | Human | 10.1 | BioLegend |
| FcεRIα | Human | AER-37 | BioLegend |
| SIRPα | Human | 15-414 | BioLegend |

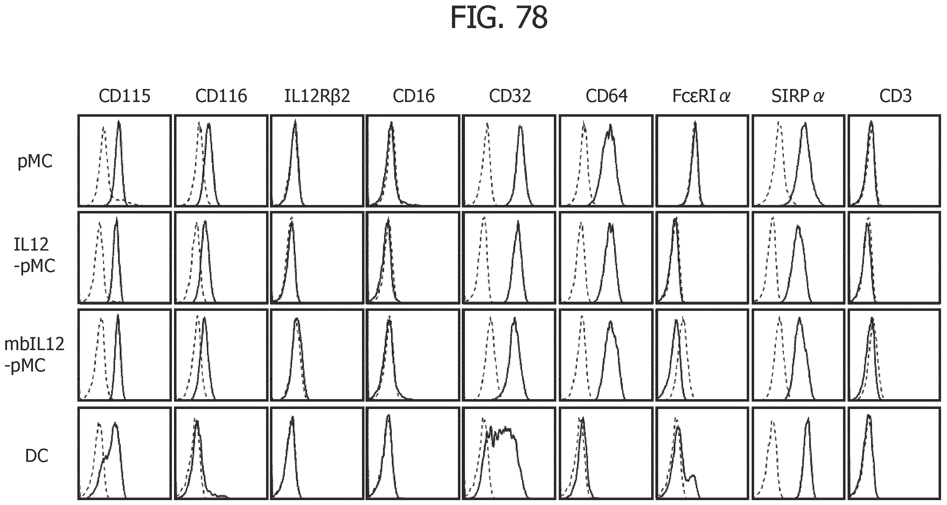

The results are shown in FIG. 78. In the figure, the word "human" is omitted from each cell name. The human mbIL12-pMC, as well as the human pMC, expressed CD115, CD116, CD32, CD64, and SIRPα, but did not express IL12Rβ2, CD16, FcεRIα, and CD3. These results confirmed that the expression of the membrane-bound IL-12 did not affect the expression of other cell surface proteins. The human DC expressed CD115, CD116, CD32, CD64, FcεRIα, and SIRPα, but did not express IL12Rβ2, CD16, and CD3.

### 32. Production of chimeric antigen receptor (CAR)-expressing human proliferative myeloid cells

### (32-1) Production of lentiviral vector expressing GC33-based CAR

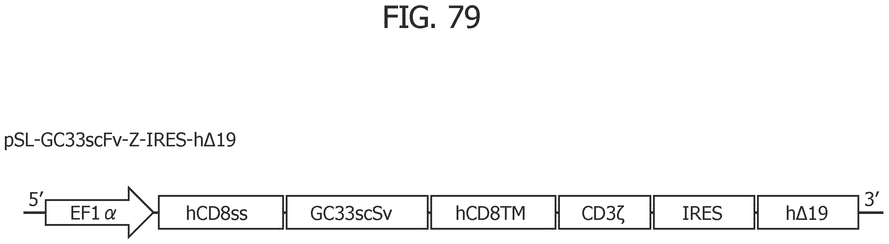

A cDNA was synthesized by entrusting GenScript, connecting in the 5' to 3' direction, a signaling sequence (amino acids 1-21) of human CD8α (SEQ ID NO: 30), a nucleic acid sequence encoding VH of GC33 (SEQ ID NO: 19), a nucleic acid sequence encoding a 205c linker (Biochemistry, 1991, 30 (42): 10117-10125) (SEQ ID NO: 20), a nucleic acid sequence encoding VL of GC33 (SEQ ID NO: 21), a cDNA encoding a transmembrane region (amino acids 128-212) of human CD8α (SEQ ID NO: 31), a cDNA encoding an intracellular region (amino acids 52-164) of human CD3ζ (SEQ ID NO: 32), an IRE sequence (SEQ ID NO: 11), and a cDNA encoding an extracellular region (amino acids 1-333) of human CD19 (SEQ ID NO: 12). The fused cDNA was inserted into the multicloning site of the pSL (SEQ ID NO: 7) to obtain pSL-GC33scFv-Z-IRES-hΔ19 (FIG. 79).

The pSL-GC33scFv-Z-IRES-hΔ19, the pCAG-HIVgp, and the pCMV-VSV-G-RSV-Rev were introduced into 293T cells by lipofection. Three days after the lipofection, the culture supernatant was collected and filtered through a filter with a pore size of 0.45 µm, and the viral particle was concentrated using Lenti-X concentrator. The obtained viral particles were suspended in DMEM, dispensed into cryovials, and stored at -150°C.

### (32-2) Production of CAR-expressing human proliferative myeloid cells

The human pMC and the human mbIL12-pMC were respectively seeded on 48-well plates, and were infected with the lentiviral vector produced in (32-1) above. For growth culture after the infection, the hpMC medium was used. Eight days after the infection, the resultant cells were stained with a fluorescently labeled anti-CD19 antibody (BioLegend, Clone 4G7), and cells were isolated by using the expression of Δ19 as an indicator. The isolated Δ19-expressing cells (CD19-positive cells) continued to proliferate in hpMC medium for more than three months. The thus obtained cells were respectively named "human pMC-GC-33-Z" and "human mbIL12-pMC-GC33-Z".

### (32-3) Cell surface marker analysis

The human pMC, the human mbIL12-pMC, and the human pMC-GC33-Z and the human mbIL12-pMC-GC33-Z produced in (32-2) above were stained with a fluorescently labeled anti-CD 19 antibody, the anti-IL12 antibody shown in Table 29, and FITC-labeled human GPC3 (ACRO Biosystems) to be analyzed by flow cytometry.

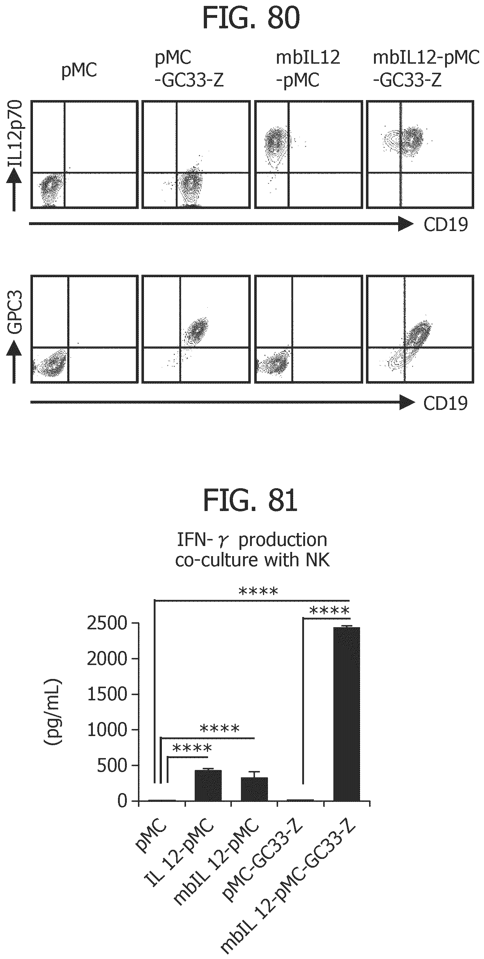

The results are shown in FIG. 80. In the figure, the word "human" is omitted from each cell name. The human pMC-GC33-Z and the human mbILI2-pMC-GC33-Z were CD19-positive and GPC3 binding-positive, confirming that they express GC33-based CAR. The human mbIL12-pMC and the human mbIL12-pMC-GC33-Z were IL12p70-positive, confirming that they express IL12 on the cell surface.

### 33. Ability of CAR- and/or membrane-bound IL-12-expressing human proliferative myeloid cells to induce IFN-γ production

NK cells were isolated from the peripheral blood mononuclear cells of the pMC donor (Table 25), using NK Cell Isolation Kit, human (Miltenyi Biotec). T cells were also isolated from the peripheral blood mononuclear cells of the pMC donor (Table 25), using Pan T cell isolation kit, human (Miltenyi Biotec). The NK cells or the T cells (5×10⁴ cells/well) were mixed with the human pMC, the human IL12-pMC, the human mbIL12-pMC, the human pMC-GC33-Z, and the human mbIL12-pMC-GC33-Z, each irradiated with 20 Gy (1×10⁴ cells/well), and the resultants were seeded in 96-well round bottom plates, and co-cultured for 48 hours in 10% FCS-containing PRMI 1640. Culture supernatants were collected, and IFN-γ was quantified using ELISA MAX^{™} Standard Set Human IFN-γ.

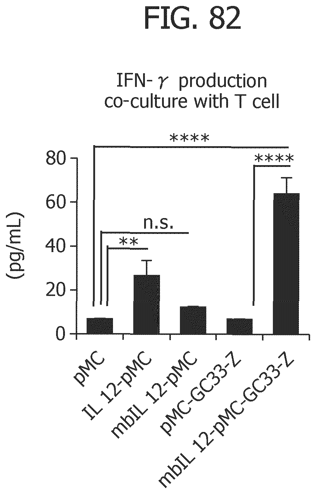

The results are shown in FIGS. 81 and 82. In the figures, the word "human" is omitted from each cell name. In the figure, an asterisk (*) indicates a p-value obtained by one-way ANOVA with Tukey post-hoc test (**p < 0.01, ***p < 0.001, ****p < 0.0001), and "n.s." indicates "no significant difference". All the human proliferative myeloid cells induced IFN-γ production in the NK cells and/or the T cells. In particular, the human mbIL12-pMC-GC33-Z, expressing both chimeric antigen receptors and the membrane-bound IL-12, induced the IFN-γ production in the NK cells and/or the T cells much more strongly than the other cells. These results indicated that proliferative myeloid cells that express both chimeric antigen receptors and membrane-bound IL-12 have a high antitumor effect *in vivo.*

## Claims

1. A method for producing a proliferative myeloid cell that constitutively produces IL-12p70, comprising the steps of:
(1) introducing a nucleic acid encoding c-MYC into a myeloid cell;
(2) culturing the cell obtained by the step (1) in the presence of GM-CSF and M-SCF; and
(3) introducing a nucleic acid encoding an IL-12p35 subunit and a nucleic acid encoding an IL-12p40 subunit into the cell obtained by the step (2).

2. The method according to claim 1, wherein a nucleic acid encoding BMI1, and a nucleic acid encoding MDM2, LYL1 or BCL2 isoform alpha, are further introduced in the step (1).

3. The method according to claim 1, wherein the myeloid cell is differentiated from a pluripotent stem cell.

4. A proliferative myeloid cell that constitutively produces IL-12p70, produced by the method according to any one of claims 1 to 3.

5. A proliferative myeloid cell that constitutively produces IL-12p70, comprising an exogenous nucleic acid encoding c-MYC, an exogenous nucleic acid encoding an IL-12p35 subunit, and an exogenous nucleic acid encoding an IL-12p40 subunit.

6. The cell according to claim 5, wherein the cell proliferates in GM-CSF- and M-CSF-dependent manners.

7. The cell according to claim 5, wherein the cell produces IL-12p70 in an amount of at least 300 pg/1×10⁴ cells/24 hours in the absence of CD40-CD40 ligand interaction or toll-like receptor stimulation.

8. The cell according to claim 5, wherein the cell produces at least 5-fold more IL-12p70 than a monocyte-derived dendritic cell in the presence of toll-like receptor stimulation.

9. The cell according to claim 5, wherein the cell expresses at least one costimulatory molecule selected from the group consisting of CD86, CD80, and CD83.

10. The cell according to claim 5, wherein the cell does not express a major histocompatibility complex class I and/or class II molecule.

11. The cell according to claim 10, wherein the cell is deficient in genes encoding major histocompatibility complex class I and/or class II and/or a gene encoding a factor involved in the expression thereof.

12. The cell according to claim 11, wherein the cell further comprises an exogenous nucleic acid encoding a chimeric antigen receptor.

13. The cell according to claim 11, wherein the cell is further deficient in SIRPα gene.

14. The cell according to claim 5, wherein the cell is deficient in endogenous genes encoding major histocompatibility complex class I and/or class II and/or a gene encoding a factor involved in the expression thereof, and further comprises an exogenous nucleic acid encoding a desired major histocompatibility complex class I and/or class II.

15. A composition for activating a T cell and/or a NK cell, comprising the cell according to any one of claims 5 to 14.

16. The composition according to claim 15, wherein the composition induces interferon-γ production by the T cell and/or the NK cell.

17. A pharmaceutical composition for preventing or treating cancer in a subject, comprising the cell according to any one of claims 5 to 14.

18. A method for preventing or treating cancer in a subject, comprising the step of administering the cell according to any one of claims 5 to 14 to the subject.

19. The method according to claim 18, further comprising the step of administering an immune checkpoint inhibitor to the subject.
